# EUROPEAN PATENT SPECIFICATION

(11) **EP 2 248 895 B1**
(45) Date of publication and mention of the grant of the patent: **06.07.2016**
(21) Application number: 10009016.6
(22) Date of filing: 20.12.2004
(51) Int. Cl.: C12N 15/113, A61K 31/7125, A61K 31/04, A61K 31/166, A61K 31/52, A61K 31/7048, A61K 38/18, A61P 35/00, C07K 14/495

(54) **Combination therapy associating a TGF-beta antagonist with a chemotherapeutic agent**
Kombinations-therapie die ein TGF-beta Antagonisten mit einem Chemotherapeutikum assoziiert
Thérapie combinatoire associant un antagoniste du TGF-beta avec un agent chimiothérapeutique

(30) Priority: 19.12.2003 EP 03029367; 05.02.2004 US 541771 P
(43) Date of publication of application: 10.11.2010
(62) Divisional of application: 04804940.7
(73) Proprietor: ISARNA Therapeutics GmbH, 80807 München (DE)
(72) Inventor: Schlingensiepen, Karl-Hermann, 93093 Donaustauf (DE); Schlingensiepen, Reimar, 93051 Regensburg (DE)
(74) Representative: V.O.

(56) References cited:
- WO-A-94/25588
- WO-A-99/63975
- WO-A-2004/005552
- ARTEAGA C L ET AL: "REVERSAL OF TAMOXIFEN RESISTANCE OF HUMAN BREAST CARCINOMAS IN VIVO BY NEUTRALIZING ANTIBODIES TO TRANSFORMING GROWTH FACTOR-BETA", JOURNAL OF THE NATIONAL CANCER INSTITUTE, vol. 91, no. 1, 6 January 1999 (1999-01-06), pages 46-53, XP000940473, ISSN: 0027-8874
- WOJTOWICZ-PRAGA SLAWOMIR: "Reversal of tumor-induced immunosuppression by TGF-beta inhibitors.", INVESTIGATIONAL NEW DRUGS, vol. 21, no. 1, February 2003 (2003-02), pages 21-32, XP008035079, ISSN: 0167-6997
- JACHIMCZAK P ET AL: "THE EFFECT OF TRANSFORMING GROWTH FACTOR-BETA 2-SPECIFIC PHOSPHOROTIHIOATE-ANTI-SENSE OLIGODEOXYNUCLEOTIDES IN REVERSING CELLULAR IMMUNOSUPPRESSION IN MALIGNANT GLIOMA", JOURNAL OF NEUROSURGERY, vol. 78, no. 6, 1993, pages 944-951, XP000886109, ISSN: 0022-3085
- YU D ET AL: "HYBRID OLIGONUCLEOTIDES: SYNTHESIS, BIOPHYSICAL PROPERTIES STABILITY STUDIES, AND BIOLOGICAL ACTIVITY", BIOORGANIC & MEDICINAL CHEMISTRY, vol. 4, no. 10, 1996, pages 1685-1692, XP000644792, ISSN: 0968-0896

### SEQUENCE LISTING

<110> Antisense Pharma GmbH
<120> PHARMACEUTICAL COMPOSITION
<130> 042613wo CS/FM
<140> PCT/EP2004/053604
   <141> 2004-12-20
<160> 221
<170> PatentIn Ver. 2.1
<210> 1
   <211> 14
   <212> DNA
   <213> Artificial Sequence
<220>
   <223> Description of Artificial Sequence:human TGF-beta
   1 antisense oligonucleotide
<400> 1
   cgatagtctt gcag 14
<210> 2
   <211> 14
   <212> DNA
   <213> Artificial Sequence
<220>
   <223> Description of Artificial Sequence:human TGF-beta
   1 antisense oligonucleotide
<400> 2
   gtcgatagtc ttgc 14
<210> 3
   <211> 14
   <212> DNA
   <213> Artificial Sequence
<220>
   <223> Description of Artificial Sequence:human TGF-beta
   1 antisense oligonucleotide
<400> 3
   cttggacagg atct 14
<210> 4
   <211> 14
   <212> DNA
   <213> Artificial Sequence
<220>
   <223> Description of Artificial Sequence:human TGF-beta
   1 antisense oligonucleotide
<400> 4
   ccaggaattg ttgc 14
<210> 5
   <211> 14
   <212> DNA
   <213> Artificial Sequence
<220>
   <223> Description of Artificial Sequence:human TGF-beta
   1 antisense oligonucleotide
<400> 5
   cctcaatttc ccct 14
<210> 6
   <211> 14
   <212> DNA
   <213> Artificial Sequence
<220>
   <223> Description of Artificial Sequence:human TGF-beta
   1 antisense oligonucleotide
<400> 6
   gatgtccact tgca 14
<210> 7
   <211> 14
   <212> DNA
   <213> Artificial Sequence
<220>
   <223> Description of Artificial Sequence:human TGF-beta
   1 antisense oligonucleotide
<400> 7
   ctccaaatgt aggg 14
<210> 8
   <211> 14
   <212> DNA
   <213> Artificial Sequence
<220>
   <223> Description of Artificial Sequence:human TGF-beta
   1 antisense oligonucleotide
<400> 8
   accttgctgt actg 14
<210> 9
   <211> 14
   <212> DNA
   <213> Artificial Sequence
<220>
   <223> Description of Artificial Sequence:human TGF-beta
   1 antisense oligonucleotide
<400> 9
   gtagtacacg atgg 14
<210> 10
   <211> 14
   <212> DNA
   <213> Artificial Sequence
<220>
   <223> Description of Artificial Sequence:human TGF-beta
   1 antisense oligonucleotide
<400> 10
   cacgtagtac acga 14
<210> 11
   <211> 14
   <212> DNA
   <213> Artificial Sequence
<220>
   <223> Description of Artificial Sequence:human TGF-beta

   1 antisense oligonucleotide
<400> 11
   catgttggac agct 14
<210> 12
   <211> 14
   <212> DNA
   <213> Artificial Sequence
<220>
   <223> Description of Artificial Sequence:human TGF-beta
   1 antisense oligonucleotide
<400> 12
   gcacgatcat gttg 14
<210> 13
   <211> 16
   <212> DNA
   <213> Artificial Sequence
<220>
   <223> Description of Artificial Sequence:human TGF-beta
   1 antisense oligonucleotide
<400> 13
   tgtactctgc ttgaac 16
<210> 14
   <211> 18
   <212> DNA
   <213> Artificial Sequence
<220>
   <223> Description of Artificial Sequence:human TGF-beta
   1 antisense oligonucleotide
<400> 14
   ctgatgtgtt gaagaaca 18
<210> 15
   <211> 16
   <212> DNA
   <213> Artificial Sequence
<220>
   <223> Description of Artificial Sequence:human TGF-beta
   1 antisense oligonucleotide
<400> 15
   ctctgatgtg ttgaag 16
<210> 16
   <211> 16
   <212> DNA
   <213> Artificial Sequence
<220>
   <223> Description of Artificial Sequence:human TGF-beta
   1 antisense oligonucleotide
<400> 16
   ggaagtcaat gtacag 16
<210> 17
   <211> 18
   <212> DNA
   <213> Artificial Sequence
<220>
   <223> Description of Artificial Sequence:human TGF-beta
   1 antisense oligonucleotide
<400> 17
   catgtcgata gtcttgca 18
<210> 18
   <211> 18
   <212> DNA
   <213> Artificial Sequence
<220>
   <223> Description of Artificial Sequence:human TGF-beta
   1 antisense oligonucleotide
<400> 18
   agctgaagca atagttgg 18
<210> 19
   <211> 18
   <212> DNA
   <213> Artificial Sequence
<220>
   <223> Description of Artificial Sequence:human TGF-beta
   1 antisense oligonucleotide
<400> 19
   gtcatagatt tcgttgtg 18
<210> 20
   <211> 18
   <212> DNA
   <213> Artificial Sequence
<220>
   <223> Description of Artificial Sequence:human TGF-beta
   1 antisense oligonucleotide
<400> 20
   ctccactttt aacttgag 18
<210> 21
   <211> 18
   <212> DNA
   <213> Artificial Sequence
<220>
   <223> Description of Artificial Sequence:human TGF-beta
   1 antisense oligonucleotide
<400> 21
   tgctgtattt ctggtaca 18
<210> 22
   <211> 14
   <212> DNA
   <213> Artificial Sequence
<220>
   <223> Description of Artificial Sequence:human TGF-beta
   2 antisense oligonucleotide
<400> 22
   cacacagtag tgca 14
<210> 23
   <211> 14
   <212> DNA
   <213> Artificial Sequence
<220>
   <223> Description of Artificial Sequence:human TGF-beta
   2 antisense oligonucleotide
<400> 23
   gcacacagta gtgc 14
<210> 24
   <211> 17
   <212> DNA
   <213> Artificial Sequence
<220>
   <223> Description of Artificial Sequence:human TGF-beta
   2 antisense oligonucleotide
<400> 24
   gcttgctcag gatctgc 17
<210> 25
   <211> 14
   <212> DNA
   <213> Artificial Sequence
<220>
   <223> Description of Artificial Sequence:human TGF-beta
   2 antisense oligonucleotide
<400> 25
   tactcttcgt cgct 14
<210> 26
   <211> 14
   <212> DNA
   <213> Artificial Sequence
<220>
   <223> Description of Artificial Sequence:human TGF-beta
   2 antisense oligonucleotide
<400> 26
   cttggcgtag tact 14
<210> 27
   <211> 14
   <212> DNA
   <213> Artificial Sequence
<220>
   <223> Description of Artificial Sequence:human TGF-beta
   2 antisense oligonucleotide
<400> 27
   gtaaacctcc ttgg 14
<210> 28
   <211> 19
   <212> DNA
   <213> Artificial Sequence
<220>
   <223> Description of Artificial Sequence:human TGF-beta
   2 antisense oligonucleotide
<400> 28
   gtctattttg taaacctcc 19
<210> 29
   <211> 20
   <212> DNA
   <213> Artificial Sequence
<220>
   <223> Description of Artificial Sequence:human TGF-beta
   2 antisense oligonucleotide
<400> 29
   gcatgtctat tttgtaaacc 20
<210> 30
   <211> 18
   <212> DNA
   <213> Artificial Sequence
<220>
   <223> Description of Artificial Sequence:human TGF-beta
   2 antisense oligonucleotide
<400> 30
   cggcatgtct attttgta 18
<210> 31
   <211> 14
   <212> DNA
   <213> Artificial Sequence
<220>
   <223> Description of Artificial Sequence:human TGF-beta
   2 antisense oligonucleotide
<400> 31
   ggcatcaagg tacc 14
<210> 32
   <211> 14
   <212> DNA
   <213> Artificial Sequence
<220>
   <223> Description of Artificial Sequence:human TGF-beta
   2 antisense oligonucleotide
<400> 32
   ctgtagaaag tggg 14
<210> 33
   <211> 18
   <212> DNA
   <213> Artificial Sequence
<220>
   <223> Description of Artificial Sequence:human TGF-beta
   2 antisense oligonucleotide
<400> 33
   acaattctga agtagggt 18
<210> 34
   <211> 18
   <212> DNA
   <213> Artificial Sequence
<220>
   <223> Description of Artificial Sequence:human TGF-beta
   2 antisense oligonucleotide
<400> 34
   tcaccaaatt ggaagcat 18
<210> 35
   <211> 20
   <212> DNA
   <213> Artificial Sequence
<220>
   <223> Description of Artificial Sequence:human TGF-beta
   2 antisense oligonucleotide
<400> 35
   gctttcacca aattggaagc 20
<210> 36
   <211> 14
   <212> DNA
   <213> Artificial Sequence
<220>
   <223> Description of Artificial Sequence:human TGF-beta
   2 antisense oligonucleotide
<400> 36
   ctggcttttg ggtt 14
<210> 37
   <211> 18
   <212> DNA
   <213> Artificial Sequence
<220>
   <223> Description of Artificial Sequence:human TGF-beta
   2 antisense oligonucleotide
<400> 37
   tctgatatag ctcaatcc 18
<210> 38
   <211> 18
   <212> DNA
   <213> Artificial Sequence
<220>
   <223> Description of Artificial Sequence:human TGF-beta
   2 antisense oligonucleotide
<400> 38
   tcctagtgga ctttatag 18
<210> 39
   <211> 16
   <212> DNA
   <213> Artificial Sequence
<220>
   <223> Description of Artificial Sequence:human TGF-beta
   2 antisense oligonucleotide
<400> 39
   tttttcctag tggact 16
<210> 40
   <211> 19
   <212> DNA
   <213> Artificial Sequence
<220>
   <223> Description of Artificial Sequence:human TGF-beta
   2 antisense oligonucleotide
<400> 40
   caattatcct gcacatttc 19
<210> 41
   <211> 16
   <212> DNA
   <213> Artificial Sequence
<220>
   <223> Description of Artificial Sequence:human TGF-beta
   2 antisense oligonucleotide
<400> 41
   gcaattatcc tgcaca 16
<210> 42
   <211> 16
   <212> DNA
   <213> Artificial Sequence
<220>
   <223> Description of Artificial Sequence:human TGF-beta
   2 antisense oligonucleotide
<400> 42
   gcagcaatta tcctgc 16
<210> 43
   <211> 14
   <212> DNA
   <213> Artificial Sequence
<220>
   <223> Description of Artificial Sequence:human TGF-beta
   2 antisense oligonucleotide
<400> 43
   tggcattgta ccct 14
<210> 44
   <211> 14
   <212> DNA
   <213> Artificial Sequence
<220>
   <223> Description of Artificial Sequence:human TGF-beta
   2 antisense oligonucleotide
<400> 44
   tgtgctgagt gtct 14
<210> 45
   <211> 16
   <212> DNA
   <213> Artificial Sequence
<220>
   <223> Description of Artificial Sequence:human TGF-beta
   2 antisense oligonucleotide
<400> 45
   cctgctgtgc tgagtg 16
<210> 46
   <211> 14
   <212> DNA
   <213> Artificial Sequence
<220>
   <223> Description of Artificial Sequence:human TGF-beta
   2 antisense oligonucleotide
<400> 46
   cttgggtgtt ttgc 14
<210> 47
   <211> 18
   <212> DNA
   <213> Artificial Sequence
<220>
   <223> Description of Artificial Sequence:human TGF-beta
   2 antisense oligonucleotide
<400> 47
   tttagctgca tttgcaag 18
<210> 48
   <211> 14
   <212> DNA
   <213> Artificial Sequence
<220>
   <223> Description of Artificial Sequence:human TGF-beta
   2 antisense oligonucleotide
<400> 48
   gccacttttc caag 14
<210> 49
   <211> 14
   <212> DNA
   <213> Artificial Sequence
<220>
   <223> Description of Artificial Sequence:human TGF-beta
   3 antisense oligonucleotide
<400> 49
   tcgagcttcc ccca 14
<210> 50
   <211> 14
   <212> DNA
   <213> Artificial Sequence
<220>
   <223> Description of Artificial Sequence:human TGF-beta
   3 antisense oligonucleotide
<400> 50
   ccccgagccc aagg 14
<210> 51
   <211> 13
   <212> DNA
   <213> Artificial Sequence
<220>
   <223> Description of Artificial Sequence:human TGF-beta
   3 antisense oligonucleotide
<400> 51
   cccgacgagc cgg 13
<210> 52
   <211> 14
   <212> DNA
   <213> Artificial Sequence
<220>
   <223> Description of Artificial Sequence:human TGF-beta
   3 antisense oligonucleotide
<400> 52
   acgcaccaag gcga 14
<210> 53
   <211> 15
   <212> DNA
   <213> Artificial Sequence
<220>
   <223> Description of Artificial Sequence:human TGF-beta
   3 antisense oligonucleotide
<400> 53
   cgggttgtcg agccc 15
<210> 54
   <211> 13
   <212> DNA
   <213> Artificial Sequence
<220>
   <223> Description of Artificial Sequence:human TGF-beta
   3 antisense oligonucleotide
<400> 54
   cggcagtgcc ccg 13
<210> 55
   <211> 14
   <212> DNA
   <213> Artificial Sequence
<220>
   <223> Description of Artificial Sequence:human TGF-beta
   3 antisense oligonucleotide
<400> 55
   cgcaattctg ctcg 14
<210> 56
   <211> 14
   <212> DNA
   <213> Artificial Sequence
<220>
   <223> Description of Artificial Sequence:human TGF-beta
   3 antisense oligonucleotide
<400> 56
   ttcgttgtgc tccc 14
<210> 57
   <211> 14
   <212> DNA
   <213> Artificial Sequence
<220>
   <223> Description of Artificial Sequence:human TGF-beta
   3 antisense oligonucleotide
<400> 57
   attccgactc ggtg 14
<210> 58
   <211> 17
   <212> DNA
   <213> Artificial Sequence
<220>
   <223> Description of Artificial Sequence:human TGF-beta
   3 antisense oligonucleotide
<400> 58
   acgtgcgtca tcaccgt 17
<210> 59
   <211> 10
   <212> DNA
   <213> Artificial Sequence
<220>
   <223> Description of Artificial Sequence:human TGF-beta
   3 antisense oligonucleotide
<400> 59
   ccaagaagcc 10
<210> 60
   <211> 14
   <212> DNA
   <213> Artificial Sequence
<220>
   <223> Description of Artificial Sequence:human TGF-beta
   3 antisense oligonucleotide
<400> 60
   cctaatgcct tcca 14
<210> 61
   <211> 14
   <212> DNA
   <213> Artificial Sequence
<220>
   <223> Description of Artificial Sequence:human TGF-beta
   3 antisense oligonucleotide
<400> 61
   tcagcagggc cagg 14
<210> 62
   <211> 17
   <212> DNA
   <213> Artificial Sequence
<220>
   <223> Description of Artificial Sequence:human TGF-beta
   3 antisense oligonucleotide
<400> 62
   gcaaagttca gcagggc 17
<210> 63
   <211> 16
   <212> DNA
   <213> Artificial Sequence
<220>
   <223> Description of Artificial Sequence:human TGF-beta
   3 antisense oligonucleotide
<400> 63
   ggcaaagttc agcagg 16
<210> 64
   <211> 18
   <212> DNA
   <213> Artificial Sequence
<220>
   <223> Description of Artificial Sequence:human TGF-beta
   3 antisense oligonucleotide
<400> 64
   gtggcaaagt tcagcagg 18
<210> 65
   <211> 14
   <212> DNA
   <213> Artificial Sequence
<220>
   <223> Description of Artificial Sequence:human TGF-beta
   3 antisense oligonucleotide
<400> 65
   gtggcaaagt tcag 14
<210> 66
   <211> 18
   <212> DNA
   <213> Artificial Sequence
<220>
   <223> Description of Artificial Sequence:human TGF-beta
   3 antisense oligonucleotide
<400> 66
   gaccgtggca aagttcag 18
<210> 67
   <211> 15
   <212> DNA
   <213> Artificial Sequence
<220>
   <223> Description of Artificial Sequence:human TGF-beta
   3 antisense oligonucleotide
<400> 67
   agagaggctg accgt 15
<210> 68
   <211> 17
   <212> DNA
   <213> Artificial Sequence
<220>
   <223> Description of Artificial Sequence:human TGF-beta
   3 antisense oligonucleotide
<400> 68
   gagagagaga ggctgac 17
<210> 69
   <211> 15
   <212> DNA
   <213> Artificial Sequence
<220>
   <223> Description of Artificial Sequence:human TGF-beta
   3 antisense oligonucleotide
<400> 69
   acagagagag gctga 15
<210> 70
   <211> 15
   <212> DNA
   <213> Artificial Sequence
<220>
   <223> Description of Artificial Sequence:human TGF-beta
   3 antisense oligonucleotide
<400> 70
   gtggacagag agagg 15
<210> 71
   <211> 18
   <212> DNA
   <213> Artificial Sequence
<220>
   <223> Description of Artificial Sequence:human TGF-beta
   3 antisense oligonucleotide
<400> 71
   caactggaca gagagagg 18
<210> 72
   <211> 16
   <212> DNA
   <213> Artificial Sequence
<220>
   <223> Description of Artificial Sequence:human TGF-beta 3 antisense oligonucleotide
<400> 72
   tcttcttgat gtggcc 16
<210> 73
   <211> 17
   <212> DNA
   <213> Artificial Sequence
<220>
   <223> Description of Artificial Sequence:human TGF-beta
   3 antisense oligonucleotide
<400> 73
   ccctcttctt cttgatg 17
<210> 74
   <211> 14
   <212> DNA
   <213> Artificial Sequence
<220>
   <223> Description of Artificial Sequence:human TGF-beta
   3 antisense oligonucleotide
<400> 74
   caccctcttc ttct 14
<210> 75
   <211> 17
   <212> DNA
   <213> Artificial Sequence
<220>
   <223> Description of Artificial Sequence:human TGF-beta
   3 antisense oligonucleotide
<400> 75
   atggatttct ttggcat 17
<210> 76
   <211> 13
   <212> DNA
   <213> Artificial Sequence
<220>
   <223> Description of Artificial Sequence:human TGF-beta
   3 antisense oligonucleotide
<400> 76
   ggatttcttt ggc 13
<210> 77
   <211> 18
   <212> DNA
   <213> Artificial Sequence
<220>
   <223> Description of Artificial Sequence:human TGF-beta
   3 antisense oligonucleotide
<400> 77
   aagttggact ctcttctc 18
<210> 78
   <211> 18
   <212> DNA
   <213> Artificial Sequence
<220>
   <223> Description of Artificial Sequence:human TGF-beta
   3 antisense oligonucleotide
<400> 78
   taagttggac tctcttct 18
<210> 79
   <211> 16
   <212> DNA
   <213> Artificial Sequence
<220>
   <223> Description of Artificial Sequence:human PGE
   antisense oligonucleotide
<400> 79
   taggagtggt tgaggc 16
<210> 80
   <211> 17
   <212> DNA
   <213> Artificial Sequence
<220>
   <223> Description of Artificial Sequence:human PGE
   antisense oligonucleotide
<400> 80
   gtgtaggagt ggttgag 17
<210> 81
   <211> 14
   <212> DNA
   <213> Artificial Sequence
<220>
   <223> Description of Artificial Sequence:human PGE
   antisense oligonucleotide
<400> 81
   ctgtgtagga gtgg 14
<210> 82
   <211> 14
   <212> DNA
   <213> Artificial Sequence
<220>
   <223> Description of Artificial Sequence:human PGE
   antisense oligonucleotide
<400> 82
   cccacatgcc tgtg 14
<210> 83
   <211> 14
   <212> DNA
   <213> Artificial Sequence
<220>
   <223> Description of Artificial Sequence:human PGE
   antisense oligonucleotide
<400> 83
   cgatgaacaa cgag 14
<210> 84
   <211> 16
   <212> DNA
   <213> Artificial Sequence
<220>
   <223> Description of Artificial Sequence:human PGE
   antisense oligonucleotide
<400> 84
   ctggcgatga acaacg 16
<210> 85
   <211> 14
   <212> DNA
   <213> Artificial Sequence
<220>
   <223> Description of Artificial Sequence:human PGE
   antisense oligonucleotide
<400> 85
   cgctggcgat gaac 14
<210> 86
   <211> 15
   <212> DNA
   <213> Artificial Sequence
<220>
   <223> Description of Artificial Sequence:human PGE
   antisense oligonucleotide
<400> 86
   gagctagtcc cgttg 15
<210> 87
   <211> 15
   <212> DNA
   <213> Artificial Sequence
<220>
   <223> Description of Artificial Sequence:human PGE
   antisense oligonucleotide
<400> 87
   gcgaagagct agtcc 15
<210> 88
   <211> 17
   <212> DNA
   <213> Artificial Sequence
<220>
   <223> Description of Artificial Sequence:human PGE
   antisense oligonucleotide
<400> 88
   ccagttatgc gaagagc 17
<210> 89
   <211> 16
   <212> DNA
   <213> Artificial Sequence
<220>
   <223> Description of Artificial Sequence:human PGE
   antisense oligonucleotide
<400> 89
   ccccagttat gcgaag 16
<210> 90
   <211> 14
   <212> DNA
   <213> Artificial Sequence
<220>
   <223> Description of Artificial Sequence:human VEGF
   antisense oligonucleotide
<400> 90
   cggccgcggt gtgt 14
<210> 91
   <211> 17
   <212> DNA
   <213> Artificial Sequence
<220>
   <223> Description of Artificial Sequence:human VEGF
   antisense oligonucleotide
<400> 91
   cgggaatgct tccgccg 17
<210> 92
   <211> 17
   <212> DNA
   <213> Artificial Sequence
<220>
   <223> Description of Artificial Sequence:human VEGF
   antisense oligonucleotide
<400> 92
   cggctcaccg cctcggc 17
<210> 93
   <211> 14
   <212> DNA
   <213> Artificial Sequence
<220>
   <223> Description of Artificial Sequence:human VEGF
   antisense oligonucleotide
<400> 93
   cacgtctgcg gatc 14
<210> 94
   <211> 18
   <212> DNA
   <213> Artificial Sequence
<220>
   <223> Description of Artificial Sequence:human VEGF
   antisense oligonucleotide
<400> 94
   ccccgcatcg catcaggg 18
<210> 95
   <211> 14
   <212> DNA
   <213> Artificial Sequence
<220>
   <223> Description of Artificial Sequence:human VEGF
   antisense oligonucleotide
<400> 95
   cgccttgcaa cgcg 14
<210> 96
   <211> 14
   <212> DNA
   <213> Artificial Sequence
<220>
   <223> Description of Artificial Sequence:human VEGF
   antisense oligonucleotide
<400> 96
   ccgaccgggg ccgg 14
<210> 97
   <211> 14
   <212> DNA
   <213> Artificial Sequence
<220>
   <223> Description of Artificial Sequence:human VEGF
   antisense oligonucleotide
<400> 97
   gttcatggtt tcgg 14
<210> 98
   <211> 15
   <212> DNA
   <213> Artificial Sequence
<220>
   <223> Description of Artificial Sequence:human VEGF
   antisense oligonucleotide
<400> 98
   gcagaaagtt catgg 15
<210> 99
   <211> 14
   <212> DNA
   <213> Artificial Sequence
<220>
   <223> Description of Artificial Sequence:human VEGF
   antisense oligonucleotide
<400> 99
   gctgatagac atcc 14
<210> 100
   <211> 14
   <212> DNA
   <213> Artificial Sequence
<220>
   <223> Description of Artificial Sequence:human VEGF
   antisense oligonucleotide
<400> 100
   gcgctgatag acat 14
<210> 101
   <211> 16
   <212> DNA
   <213> Artificial Sequence
<220>
   <223> Description of Artificial Sequence:human VEGF
   antisense oligonucleotide
<400> 101
   gtagctgcgc tgatag 16
<210> 102
   <211> 14
   <212> DNA
   <213> Artificial Sequence
<220>
   <223> Description of Artificial Sequence:human VEGF
   antisense oligonucleotide
<400> 102
   ctcgatctca tcag 14
<210> 103
   <211> 17
   <212> DNA
   <213> Artificial Sequence
<220>
   <223> Description of Artificial Sequence:human VEGF
   antisense oligonucleotide
<400> 103
   atgtactcga tctcatc 17
<210> 104
   <211> 16
   <212> DNA
   <213> Artificial Sequence
<220>
   <223> Description of Artificial Sequence:human VEGF
   antisense oligonucleotide
<400> 104
   gaagatgtac tcgatc 16
<210> 105
   <211> 16
   <212> DNA
   <213> Artificial Sequence
<220>
   <223> Description of Artificial Sequence:human VEGF
   antisense oligonucleotide
<400> 105
   cttgaagatg tactcg 16
<210> 106
   <211> 14
   <212> DNA
   <213> Artificial Sequence
<220>
   <223> Description of Artificial Sequence:human VEGF
   antisense oligonucleotide
<400> 106
   gcatcgcatc aggg 14
<210> 107
   <211> 14
   <212> DNA
   <213> Artificial Sequence
<220>
   <223> Description of Artificial Sequence:human VEGF
   antisense oligonucleotide
<400> 107
   ccgcatcgca tcag 14
<210> 108
   <211> 18
   <212> DNA
   <213> Artificial Sequence
<220>
   <223> Description of Artificial Sequence:human VEGF
   antisense oligonucleotide
<400> 108
   catttgttgt gctgtagg 18
<210> 109
   <211> 18
   <212> DNA
   <213> Artificial Sequence
<220>
   <223> Description of Artificial Sequence:human VEGF
   antisense oligonucleotide
<400> 109
   ggtctgcatt cacatttg 18
<210> 110
   <211> 15
   <212> DNA
   <213> Artificial Sequence
<220>
   <223> Description of Artificial Sequence:human VEGF
   antisense oligonucleotide
<400> 110
   ctttggtctg cattc 15
<210> 111
   <211> 15
   <212> DNA
   <213> Artificial Sequence
<220>
   <223> Description of Artificial Sequence:human VEGF
   antisense oligonucleotide
<400> 111
   ctttctttgg tctgc 15
<210> 112
   <211> 17
   <212> DNA
   <213> Artificial Sequence
<220>
   <223> Description of Artificial Sequence:human VEGF
   antisense oligonucleotide
<400> 112
   gctctatctt tctttgg 17
<210> 113
   <211> 17
   <212> DNA
   <213> Artificial Sequence
<220>
   <223> Description of Artificial Sequence:human VEGF
   antisense oligonucleotide
<400> 113
   gtcttgctct atctttc 17
<210> 114
   <211> 16
   <212> DNA
   <213> Artificial Sequence
<220>
   <223> Description of Artificial Sequence:human VEGF
   antisense oligonucleotide
<400> 114
   cttgtcttgc tctatc 16
<210> 115
   <211> 18
   <212> DNA
   <213> Artificial Sequence
<220>
   <223> Description of Artificial Sequence:human VEGF
   antisense oligonucleotide
<400> 115
   catctgcaag tacgttcg 18
<210> 116
   <211> 18
   <212> DNA
   <213> Artificial Sequence
<220>
   <223> Description of Artificial Sequence:human VEGF
   antisense oligonucleotide
<400> 116
   cacatctgca agtacgtt 18
<210> 117
   <211> 18
   <212> DNA
   <213> Artificial Sequence
<220>
   <223> Description of Artificial Sequence:human VEGF
   antisense oligonucleotide
<400> 117
   gtcacatctg caagtacg 18
<210> 118
   <211> 14
   <212> DNA
   <213> Artificial Sequence
<220>
   <223> Description of Artificial Sequence:human VEGF
   antisense oligonucleotide
<400> 118
   catctgcaag tacg 14
<210> 119
   <211> 15
   <212> DNA
   <213> Artificial Sequence
<220>
   <223> Description of Artificial Sequence:human VEGF
   antisense oligonucleotide
<400> 119
   cacatctgca agtac 15
<210> 120
   <211> 14
   <212> DNA
   <213> Artificial Sequence
<220>
   <223> Description of Artificial Sequence:human VEGF
   antisense oligonucleotide
<400> 120
   gtcacatctg caag 14
<210> 121
   <211> 14
   <212> DNA
   <213> Artificial Sequence
<220>
   <223> Description of Artificial Sequence:human VEGF
   antisense oligonucleotide
<400> 121
   cttgtcacat ctgc 14
<210> 122
   <211> 16
   <212> DNA
   <213> Artificial Sequence
<220>
   <223> Description of Artificial Sequence:human VEGF
   antisense oligonucleotide
<400> 122
   ggcttgtcac atctgc 16
<210> 123
   <211> 16
   <212> DNA
   <213> Artificial Sequence
<220>
   <223> Description of Artificial Sequence:human VEGF
   antisense oligonucleotide
<400> 123
   ctcggcttgt cacatc 16
<210> 124
   <211> 14
   <212> DNA
   <213> Artificial Sequence
<220>
   <223> Description of Artificial Sequence:human VEGF
   antisense oligonucleotide
<400> 124
   ctccttcctc ctgc 14
<210> 125
   <211> 18
   <212> DNA
   <213> Artificial Sequence
<220>
   <223> Description of Artificial Sequence:human VEGF
   antisense oligonucleotide
<400> 125
   gcttgaagat gtacctcg 18
<210> 126
   <211> 15
   <212> DNA
   <213> Artificial Sequence
<220>
   <223> Description of Artificial Sequence:human VEGF
   antisense oligonucleotide
<400> 126
   cgttgctctc cgacg 15
<210> 127
   <211> 18
   <212> DNA
   <213> Artificial Sequence
<220>
   <223> Description of Artificial Sequence:human IL-10
   antisense oligonucleotide
<400> 127
   gtaaaactgg atcatctc 18
<210> 128
   <211> 18
   <212> DNA
   <213> Artificial Sequence
<220>
   <223> Description of Artificial Sequence:human IL-10
   antisense oligonucleotide
<400> 128
   cttcttttgc aagtctgt 18
<210> 129
   <211> 18
   <212> DNA
   <213> Artificial Sequence
<220>
   <223> Description of Artificial Sequence:human IL-10
   antisense oligonucleotide
<400> 129
   tgagctgtgc atgccttc 18
<210> 130
   <211> 15
   <212> DNA
   <213> Artificial Sequence
<220>
   <223> Description of Artificial Sequence:human IL-10
   antisense oligonucleotide
<400> 130
   agtcaggagg accag 15
<210> 131
   <211> 15
   <212> DNA
   <213> Artificial Sequence
<220>
   <223> Description of Artificial Sequence:human IL-10
   antisense oligonucleotide
<400> 131
   tgggtgccct ggcct 15
<210> 132
   <211> 15
   <212> DNA
   <213> Artificial Sequence
<220>
   <223> Description of Artificial Sequence:human IL-10
   antisense oligonucleotide
<400> 132
   catgttaggc aggtt 15
<210> 133
   <211> 17
   <212> DNA
   <213> Artificial Sequence
<220>
   <223> Description of Artificial Sequence:human IL-10
   antisense oligonucleotide
<400> 133
   aggcatctcg gagatct 17
<210> 134
   <211> 16
   <212> DNA
   <213> Artificial Sequence
<220>
   <223> Description of Artificial Sequence:human IL-10
   antisense oligonucleotide
<400> 134
   aaagtcttca ctctgc 16
<210> 135
   <211> 17
   <212> DNA
   <213> Artificial Sequence
<220>
   <223> Description of Artificial Sequence:human IL-10
   antisense oligonucleotide
<400> 135
   aacaagttgt ccagctg 17
<210> 136
   <211> 15
   <212> DNA
   <213> Artificial Sequence
<220>
   <223> Description of Artificial Sequence:human IL-10
   antisense oligonucleotide
<400> 136
   catcacctcc tccag 15
<210> 137
   <211> 18
   <212> DNA
   <213> Artificial Sequence
<220>
   <223> Description of Artificial Sequence:human IL-10
   antisense oligonucleotide
<400> 137
   gggtcttcag gttctccc 18
<210> 138
   <211> 18
   <212> DNA
   <213> Artificial Sequence
<220>
   <223> Description of Artificial Sequence:human IL-10
   antisense oligonucleotide
<400> 138
   cacggccttg ctcttgtt 18
<210> 139
   <211> 18
   <212> DNA
   <213> Artificial Sequence
<220>
   <223> Description of Artificial Sequence:human IL-10
   antisense oligonucleotide
<400> 139
   ttattaaagg cattcttc 18
<210> 140
   <211> 18
   <212> DNA
   <213> Artificial Sequence
<220>
   <223> Description of Artificial Sequence:human IL-10
   antisense oligonucleotide
<400> 140
   aagatgtcaa actcactc 18
<210> 141
   <211> 18
   <212> DNA
   <213> Artificial Sequence
<220>
   <223> Description of Artificial Sequence:human IL-10
   antisense oligonucleotide
<400> 141
   gtagttgatg aagatgtc 18
<210> 142
   <211> 16
   <212> DNA
   <213> Artificial Sequence
<220>
   <223> Description of Artificial Sequence:human IL-10
   antisense oligonucleotide
<400> 142
   gattttggag acctct 16
<210> 143
   <211> 16
   <212> DNA
   <213> Artificial Sequence
<220>
   <223> Description of Artificial Sequence:human IL-10
   antisense oligonucleotide
<400> 143
   tcagctatcc cagagc 16
<210> 144
   <211> 15
   <212> DNA
   <213> Artificial Sequence
<220>
   <223> Description of Artificial Sequence:human IL-10
   antisense oligonucleotide
<400> 144
   ggctgggtca gctat 15
<210> 145
   <211> 18
   <212> DNA
   <213> Artificial Sequence
<220>
   <223> Description of Artificial Sequence:human IL-10
   antisense oligonucleotide
<400> 145
   aaatcgttca cagagaag 18
<210> 146
   <211> 18
   <212> DNA
   <213> Artificial Sequence
<220>
   <223> Description of Artificial Sequence:human IL-10
   antisense oligonucleotide
<400> 146
   tctttctaaa tcgttcac 18
<210> 147
   <211> 2745
   <212> DNA
   <213> Artificial Sequence
<220>
   <223> Description of Artificial Sequence:antisense mRNA
   of human TGF-beta 1
<400> 147
   ctgcagcctt gacctcccag gatcaagtga tcctcccacc ttagcctcca gagtagctgg 60
   gaccacaggt gtacattttt taaaagtgtt ttgtagagat agggtctcac tatgttaccc 120
   aggctggtct caaatgcctg gattcaagta tcctcccatc tctgcctccc aaaagtgcta 180
   ggattacagg cgtgagcacc ccgcctggcc tgaactacta tcttttattg tcttcttcac 240
   tatcccccac taaagcaggt tcctggtggg caggaactcc tcccttaacc tctctgggct 300
   tgtttcctca acctttaaaa tgggtgttat cagagtccct gccatctcag agtgttgcta 360
   tggtgactga atgagttcat taatgtaagg cacttcaaca gtgcccaagg tgctcaataa 420
   atagatctaa ctacagtagt gttccccact ggtcccctgt gccttgatgc cgggcaaagg 480
   aatagtgcag acaggcagga ggaggcagag agggagagag agggagtggg agtgggggaa 540
   cgtcagggat ggagacccca ggcaggcgcc caatgacaca gagatccgca gtcctctctc 600
   catctttaat ggggccccag gtgggcttgg ggcacggtgt ccttaaatac agcccccatg 660
   ggcaaggcag cgggggcggg gcggggtggg gccgggcctg ccggggcggg gcggggcggg 720
   gcgggacctc agctgcactt gcaggagcgc acgatcatgt tggacagctg ctccaccttg 780
   ggcttgcggc ccacgtagta cacgatgggc agcggctcca gcgcctgcgg cacgcagcac 840
   ggcgccgccg aggcgcccgg gttatgctgg ttgtacaggg ccaggacctt gctgtactgc 900
   gtgtccaggc tccaaatgta ggggcagggc ccgaggcaga agttggcatg gtagcccttg 960
   ggctcgtgga tccacttcca gccgaggtcc ttgcggaagt caatgtacag ctgccgcacg 1020
   cagcagttct tctccgtgga gctgaagcaa tagttggtgt ccagggctcg gcggtgccgg 1080
   gagctttgca gatgctgggc cctctccagc ggggtggcca tgagaagcag gaaaggccgg 1140
   ttcatgccat gaatggtggc caggtcacct cggcggccgg tagtgaaccc gttgatgtcc 1200
   acttgcagtg tgttatccct gctgtcacag gagcagtggg cgctaaggcg aaagccctca 1260
   atttcccctc cacggctcaa ccactgccgc acaactccgg tgacatcaaa agataaccac 1320
   tctggcgagt cgctgggtgc cagcagccgg ttgctgaggt atcgccagga attgttgctg 1380
   tatttctggt acagctccac gtgctgctcc acttttaact tgagcctcct cagcagacgc 1440
   agctctgccc gggagagcaa cacgggttca ggtaccgctt ctcggagctc tgatgtgttg 1500
   aagaacatat atatgctgtg tgtactctgc ttgaacttgt catagatttc gttgtgggtt 1560
   tccaccatta gcacgcgggt gacctccttg gcgtagtagt cggcctcagg ctcgggctcc 1620
   ggttctgcac tctccccggc cacccggtcg cgggtgctgt tgtacagggc gagcacggcc 1680
   tcgggcagcg ggccgggcgg cacctccccc tggctcgggg ggctggcgag ccgcagcttg 1740
   gacaggatct ggccgcggat ggcctcgatg cgcttccgct tcaccagctc catgtcgata 1800
   gtcttgcagg tggatagtcc cgcggccggc gggccaggcg tcagcaccag tagccacagc 1860
   agcggtagca gcagcggcag cagccgcagc ccggagggcg gcatggggga ggcggcgccc 1920
   cccggcactg ccgagagcgc gaacagggct ggtgtggtgg ggaggccccg cccctgcagg 1980
   ggctgggggt ctcccggcaa aaggtaggag ggcctcgagg gaaagctgag gctcctcagg 2040
   gagaagggcg cagtggtgga ggggaggctt ggaccggggg tgtctcagta tcccacggaa 2100
   ataacctaga tgggcgcgat ctggtaccag aaggtgggtg gtcttgaata ggggatctgt 2160
   ggcaggtcgg agagagatcc gtctcctgga ggagaaaggg tctaggatgc gcgggggctc 2220
   aggagacagg ccggggatga aggcggcgtg cagggggtgc gcccgaggtc tggggaaaag 2280
   tctttgcggg aggccgggtc ggcgactccc gagggctggt ccggaatggg ggcgcctgag 2340
   ggacgccgtg tagggggcag ggagggagca agcgtccccg gcggcaaagg gaggcggtct 2400
   ggggtcccca agtcctgcct cctcgcgggg cagcgtcgcg ccaagaggtc cccgcgcctc 2460
   cggctcccag cggcaacgga aaagtctcaa aagttttttt cctcttctcc cgaccagctc 2520
   gtccctcctc ccgctcctcc tccccctcct ccccgcagtg gcgggggcgg cggcggctcg 2580
   tctcagactc tggggcctca ggctgctcct cggcgactcc ttcctccgct ccgggccgag 2640
   gccggccccg cgggcggctc agagccgggg ggggtgcccc ggacggggcg tcccccctgc 2700
   ccccggccgg ggccctcgct gtctggctgc tccgcggagg gaggt                 2745
<210> 148
   <211> 1695
   <212> DNA
   <213> Artificial Sequence
<220>
   <223> Description of Artificial Sequence:antisense mRNA
   of human TGF-beta 2
<400> 148
   tttaaaaaaa tttgcttctt gtctctctca cttacaaagt aggtgaaatg tagaataagg 60
   ccttcaactt tttttgtgtc agatgccagt tttaacaaac agaacacaaa cttccaaagt 120
   gtctgaacta gtaccgcctt ttcaaaaatt ttttaacact gatgaaccaa ggctctctta 180
   tgttttcttg ttacaagcat catcgttgtc gtcgtcatca tcattatcat catcattgtc 240
   attttggtct tgccactttt ccaagaattt tagctgcatt tgcaagactt tacaatcata 300
   ttagaaagct gttcaatctt gggtgttttg ccaatgtagt agagaatggt tagaggttct 360
   aaatcttggg acacgcagca aggagaagca gatgcttctg gatttatggt attatataag 420
   ctcaggaccc tgctgtgctg agtgtctgaa ctccataaat acgggcatgc tccagcacag 480
   aagttggcat tgtacccttt gggttcgtgt atccatttcc accctagatc cctcttgaaa 540
   tcaatgtaaa gtggacgtag gcagcaatta tcctgcacat ttctaaagca ataggccgca 600
   tccaaagcac gcttcttccg ccggttggtc tgttgtgact caagtctgta ggagggcaat 660
   aacattagca ggagatgtgg ggtcttccca ctgttttttt tcctagtgga ctttatagtt 720
   ttctgatcac cactggtata tgtggaggtg ccatcaatac ctgcaaatct tgcttctagt 780
   tcttcacttt tatttgggat gatgtaatta ttagatggta caaaagtgca gcagggacag 840
   tgtaagctta ttttaaatcc caggttcctg tctttatggt gaagccattc atgaacagca 900
   tcagttacat cgaaggagag ccattcgcct tctgctcttg ttttcacaac tttgctgtcg 960
   atgtagcgct gggttggaga tgttaaatct ttggacttga gaatctgata tagctcaatc 1020
   cgttgttcag gcactctggc ttttgggttc tgcaaacgaa agactctgaa ctctgctttc 1080
   accaaattgg aagcattctt ctccattgct gagacgtcaa atcgaacaat tctgaagtag 1140
   ggtctgtaga aagtgggcgg gatggcattt tcggagggga agaagggcgg catgtctatt 1200
   ttgtaaacct ccttggcgta gtactcttcg tcgctcctct cgcgctcgca ggcggccgcc 1260
   ctccggctcg ccttctcctg gagcaagtcc ctggtgctgt tgtagatgga aatcacctcc 1320
   ggggggactt cctcgggctc aggatagtct tctgggggac tggtgagctt cagcttgctc 1380
   aggatctgcc cgcggatcgc ctcgatcctc ttgcgcatga actggtccat atcgagtgtg 1440
   ctgcaggtag acaggctgag cgcgaccgtg accagatgca ggatcagaaa agcgctcagc 1500
   acacagtagt gcatttttta aaaaagtgga aaaaaaagtt gtttttaaaa gtcagaataa 1560
   aaaaaaagaa atcaacaatt ctcaaagtat agatcaagga gagttgtttg gttttttgtt 1620
   gttgttgttt gtttttgatg cgaaactttt gcaaacaatc tagtcaatgc ccaacagaaa 1680
   aacgtatcct gcttg                                                  1695
<210> 149
   <211> 2529
   <212> DNA
   <213> Artificial Sequence
<220>
   <223> Description of Artificial Sequence:antisense mRNA
   of human TGF-beta 3
<400> 149
<210> 150
   <211> 1259
   <212> DNA
   <213> Artificial Sequence
<220>
   <223> Description of Artificial Sequence:antisense mRNA
   of human IL-10
<400> 150
   tcaccctatg gaaacagctt aaaaacaggt gaaaataata aatattgaaa aaaattataa 60
   tattgggctt ctttctaaat cgttcacaga gaagctcagt aaataaatag aaatgggggt 120
   tgaggtatca gaggtaataa atattctata agagaggtac aataaggttt ctcaaggggc 180
   tgggtcagct atcccagagc cccagatccg attttggaga cctctaattt atgtcctaga 240
   gtctatagag tcgccaccct gatgtctcag tttcgtatct tcattgtcat gtaggcttct 300
   atgtagttga tgaagatgtc aaactcactc atggctttgt agatgccttt ctcttggagc 360
   ttattaaagg cattcttcac ctgctccacg gccttgctct tgttttcaca gggaagaaat 420
   cgatgacagc gccgtagcct cagcctgagg gtcttcaggt tctcccccag ggagttcaca 480
   tgcgccttga tgtctgggtc ttggttctca gcttggggca tcacctcctc caggtaaaac 540
   tggatcatct cagacaaggc ttggcaaccc aggtaaccct taaagtcctc cagcaaggac 600
   tcctttaaca acaagttgtc cagctgatcc ttcatttgaa agaaagtctt cactctgctg 660
   aaggcatctc ggagatctcg aagcatgtta ggcaggttgc ctgggaagtg ggtgcagctg 720
   ttctcagact gggtgccctg gcctgggctg gccctcaccc cagtcaggag gaccaggcaa 780
   cagagcagtg ctgagctgtg catgccttct tttgcaagtc tgtcttgtgg tttggttttg 840
   caagagcaac cccctgatgt gtagaccttc acctctctgt ccccctttta tattgtaagc 900
   tcagggaggc ctcttcattc attaaaaagc cacaatcaag gtttcccggc acaggatttt 960
   ttctgcttag agctcctcct tctctaacct ctctaataaa cttagttttc aatttttgca 1020
   tcgtaagcaa aaatgattgg ttgaacatga acttctgcat tacagctatt tttaggatgg 1080
   gctacctctc ttagaataat tttttagctt ctcaattaaa aaaagttgat ttcctgggga 1140
   gaacagctgt tctgtccgca gaggccctca gctgtgggtt ctcattcgcg tgttcctagg 1200
   tcacagtgac gtggacaaat tgcccattcc agaatacaat gggattgaga aataattgg  1259
<210> 151
   <211> 1765
   <212> DNA
   <213> Artificial Sequence
<220>
   <223> Description of Artificial Sequence:antisense mRNA
   of human Prostaglandin E2 Synthase
<400> 151
   ccatgagatg cctgccatga caggcgccac aaacctttcc tttattgcaa acatgtccca 60
   gtcccgggag gcttgggaag agtgggaacc aggggaaccc agggatggga ttccactgaa 120
   aacaaaccgt cctgctgtcc tgtcgagggc ccccacccac aggatgtagc catgggacag 180
   ccactgaggg tccaggaaga ggggcggcag agcagggagg cagggacagg gaggggtcgc 240
   cccagggcag tggcagggct ggaactcgtc cctaacatcc ctgagcccca gcaggtgccc 300
   tgtgttagaa gcgagagggc tggtgggggt gcgtggacaa ggggcagaat gatcctgccc 360
   ccaaccagta tcgccaggcg ctggcccagt ggccccaggc cctggcagct ggcgtcttcc 420
   gctgccttcc ctctgctctg cgcggggaca ttcagtgcgc tggggaggcc tcggtgatgg 480
   ccctctccac ccgcaggtac cagggctgga tgtgcgtgtg ctgcatcagg tcatcgaatg 540
   catccagccc ctccatcaca cgcagcacgc catacaccgc caaatcagcg agattcggct 600
   tctggccccc catgaagggc cggtccttgc ccacagcagc cacccacttg tcagcagcct 660
   catagaggtc ctcgcgcacg ttgtcctgga ggcggtgcct gctcttgagt cgcttgctga 720
   tgaggtacat ggccgctgca cccatgtact tggccacggc accctccacg gctccgaact 780
   tgccctcgcg gacaatgtag tcaaaggacg ccagagcctc ggtgggcgtg cggtacacat 840
   tgggggagat caggtgcacc agccagtcgt ccgcccactg ccgccacttc atctcctccg 900
   tcctggcctc cttcccacca tacacttgct gggcctcctt ctcgttgagc atgagccagt 960
   acttattgcc gaactcggtc acctccttgc cctgctcgtt cacagccttc atggctgggt 1020
   agtaggtgat gatctcttcc aggggctgcc ccgacaccag gtaggtcttg agggcgctga 1080
   tgatgacaga ggagtcattt agttgttgcg agctttctcc ttcctgggcc accaggatgg 1140
   gcacctttct gtaggaggag aacttgatct cagccctgcg cacagggttc acctccacca 1200
   cctggtaggg cagggcatgg aagtcgagga aggctcggac cttgctgcag aagggacacg 1260
   tcttgtactg gtacagggtc agctgcaggc ggctggacag ggagagctgc gcggctgagc 1320
   gctctgcgtg gaggtcctgg gcgcgcaggt gccaccgcgc cgtgtggtac agccccaggg 1380
   ctccccccag ggccagcgcc gcagctccca gcagccgcgg gctcccctta cgagctgcag 1440
   ccacggggct cgggccgccc gccgcccccg cgaagccagc ccggctctgc gtgggtagca 1500
   gcggctgggg gcggcctccc agcctccagg ccaaggcgca cccaccaggc cacagcgccc 1560
   gcaccacccg cgcagccggg tccatgttcg ctccgccggc gccgcgggcg ggcgcgcgaa 1620
   acgaagacgc cgaggcacgc gcggcgttta aagggccagg actctggcgc cccgcgggtt 1680
   ggccggggtg agggcgacgc taagggaacc ctcagcgctc tcgggactgg gcgtgtgccc 1740
   ggcgcccaag ttcgaaacgc ccgcc                                       1765
<210> 152
   <211> 990
   <212> DNA
   <213> Artificial Sequence
<220>
   <223> Description of Artificial Sequence:antisense mRNA
   of human VEGF
<400> 152
   cagtgtgctg gcggccgcgg tgtgtctaca ggaatcccag aaataaaact ctctaatctt 60
   ccgggctcgg tgatttagca gcaagaaaaa taaaatggcg aatccaattc caagagggac 120
   cgtgctgggt cacccgcccg ggaatgcttc cgccggagtc tcgccctccg gacccaaagt 180
   gctctgcgca gagtctcctc ttccttcatt tcaggtttct ggattaagga ctgttctgtc 240
   gatggtgatg gtgtggtggc ggcagcgtgg tttctgtatc gatcgttctg tatcagtctt 300
   tcctggtgag agatctggtt cccgaaaccc tgagggaggc tccttcctcc tgcccggctc 360
   accgcctcgg cttgtcacat ctgcaagtac gttcgtttaa ctcaagctgc ctcgccttgc 420
   aacgcgagtc tgtgtttttg caggaacatt tacacgtctg cggatcttgt acaaacaaat 480
   gctttctccg ctctgagcaa ggcccacagg gattttcttg tcttgctcta tctttctttg 540
   gtctgcattc acatttgttg tgctgtagga agctcatctc tcctatgtgc tggccttggt 600
   gaggtttgat ccgcataatc tgcatggtga tgttggactc ctcagtgggc acacactcca 660
   ggccctcgtc attgcagcag cccccgcatc gcatcagggg cacacaggat ggcttgaaga 720
   tgtactcgat ctcatcaggg tactcctgga agatgtccac cagggtctcg attggatggc 780
   agtagctgcg ctgatagaca tccatgaact tcaccacttc gtgatgattc tgccctcctc 840
   cttctgccat gggtgcagcc tgggaccact tggcatggtg gaggtagagc agcaaggcga 900
   ggctccaatg cacccaagac agcagaaagt tcatggtttc ggaggcccga ccggggccgg 960
   gccggctcgc gccgggccgc cagcacactg                                  990
<210> 153
   <211> 390
   <212> PRT
   <213> Homo sapiens
<400> 153
   Met Pro Pro Ser Gly Leu Arg Leu Leu Leu Leu Leu Leu Pro Leu Leu
   1               5                   10                  15
   Trp Leu Leu Val Leu Thr Pro Gly Arg Pro Ala Ala Gly Leu Ser Thr
               20                  25                  30
   Cys Lys Thr Ile Asp Met Glu Leu Val Lys Arg Lys Arg Ile Glu Ala
           35                  40                  45
   Ile Arg Gly Gln Ile Leu Ser Lys Leu Arg Leu Ala Ser Pro Pro Ser
       50                  55                  60
   Gln Gly Glu Val Pro Pro Gly Pro Leu Pro Glu Ala Val Leu Ala Leu
   65                  70                  75                  80
   Tyr Asn Ser Thr Arg Asp Arg Val Ala Gly Glu Ser Ala Glu Pro Glu
                   85                  90                  95
   Pro Glu Pro Glu Ala Asp Tyr Tyr Ala Lys Glu Val Thr Arg Val Leu
               100                 105                 110
   Met Val Glu Thr His Asn Glu Ile Tyr Asp Lys Phe Lys Gln Ser Thr
           115                 120                 125
   His Ser Ile Tyr Met Phe Phe Asn Thr Ser Glu Leu Arg Glu Ala Val
       130                 135                 140
   Pro Glu Pro Val Leu Leu Ser Arg Ala Glu Leu Arg Leu Leu Arg Leu
   145                 150                 155                 160
   Lys Leu Lys Val Glu Gln His Val Glu Leu Tyr Gln Lys Tyr Ser Asn
                   165                 170                 175
   Asn Ser Trp Arg Tyr Leu Ser Asn Arg Leu Leu Ala Pro Ser Asp Ser
               180                 185                 190
   Pro Glu Trp Leu Ser Phe Asp Val Thr Gly Val Val Arg Gln Trp Leu
           195                 200                 205
   Ser Arg Gly Gly Glu Ile Glu Gly Phe Arg Leu Ser Ala His Cys Ser
       210                 215                 220
   Cys Asp Ser Arg Asp Asn Thr Leu Gln Val Asp Ile Asn Gly Phe Thr
   225                 230                 235                 240
   Thr Gly Arg Arg Gly Asp Leu Ala Thr Ile His Gly Met Asn Arg Pro
                   245                 250                 255
   Phe Leu Leu Leu Met Ala Thr Pro Leu Glu Arg Ala Gln His Leu Gln
               260                 265                 270
   Ser Ser Arg His Arg Arg Ala Leu Asp Thr Asn Tyr Cys Phe Ser Ser
           275                 280                 285
   Thr Glu Lys Asn Cys Cys Val Arg Gln Leu Tyr Ile Asp Phe Arg Lys
       290                 295                 300
   Asp Leu Gly Trp Lys Trp Ile His Glu Pro Lys Gly Tyr His Ala Asn
   305                 310                 315                 320
   Phe Cys Leu Gly Pro Cys Pro Tyr Ile Trp Ser Leu Asp Thr Gln Tyr
                   325                 330                 335
   Ser Lys Val Leu Ala Leu Tyr Asn Gln His Asn Pro Gly Ala Ser Ala
               340                 345                 350
   Ala Pro Cys Cys Val Pro Gln Ala Leu Glu Pro Leu Pro Ile Val Tyr
           355                 360                 365
   Tyr Val Gly Arg Lys Pro Lys Val Glu Gln Leu Ser Asn Met Ile Val
       370                 375                 380
   Arg Ser Cys Lys Cys Ser
   385                 390
<210> 154
   <211> 20
   <212> PRT
   <213> Artificial Sequence
<220>
   <223> Description of Artificial Sequence:peptide
   fragments of human TGF-beta 1
<400> 154
   Ala Leu Asp Thr Asn Tyr Cys Phe Ser Ser Thr Glu Lys Asn Cys Cys
   1 5 10 15
   Val Arg Gln Leu
   20
<210> 155
   <211> 20
   <212> PRT
   <213> Artificial Sequence
<220>
   <223> Description of Artificial Sequence:peptide
   fragments of human TGF-beta 1
<400> 155
   Tyr Ile Asp Phe Arg Lys Asp Leu Gly Trp Lys Trp Ile His Glu Pro
   1 5 10 15
   Lys Gly Tyr His
   20
<210> 156
   <211> 20
   <212> PRT
   <213> Artificial Sequence
<220>
   <223> Description of Artificial Sequence:peptide
   fragments of human TGF-beta 1
<400> 156
   Ala Asn Phe Cys Leu Gly Pro Cys Pro Tyr Ile Trp Ser Leu Asp Thr
   1 5 10 15
   Gln Tyr Ser Lys
   20
<210> 157
   <211> 20
   <212> PRT
   <213> Artificial Sequence
<220>
   <223> Description of Artificial Sequence:peptide
   fragments of human TGF-beta 1
<400> 157
   Val Leu Ala Leu Tyr Asn Gln His Asn Pro Gly Ala Ser Ala Ala Pro
   1 5 10 15
   Cys Cys Val Pro
   20
<210> 158
   <211> 20
   <212> PRT
   <213> Artificial Sequence
<220>
   <223> Description of Artificial Sequence:peptide
   fragments of human TGF-beta 1
<400> 158
   Gln Ala Leu Glu Pro Leu Pro Ile Val Tyr Tyr Val Gly Arg Lys Pro
     1               5                  10                  15
   Lys Val Glu Gln
                20
<210> 159
   <211> 12
   <212> PRT
   <213> Artificial Sequence
<220>
   <223> Description of Artificial Sequence:peptide
   fragments of human TGF-beta 1
<400> 159
   Leu Ser Asn Met Ile Val Arg Ser Cys Lys Cys Ser
     1               5                  10
<210> 160
   <211> 20
   <212> PRT
   <213> Artificial Sequence
<220>
   <223> Description of Artificial Sequence:peptide
   fragments of human TGF-beta 1
<400> 160
   Thr Glu Lys Asn Cys Cys Val Arg Gln Leu Tyr Ile Asp Phe Arg Lys
     1               5                  10                  15
   Asp Leu Gly Trp
                20
<210> 161
   <211> 20
   <212> PRT
   <213> Artificial Sequence
<220>
   <223> Description of Artificial Sequence:peptide
   fragments of human TGF-beta 1
<400> 161
   Lys Trp Ile His Glu Pro Lys Gly Tyr His Ala Asn Phe Cys Leu Gly
    1               5                  10                  15
   Pro Cys Pro Tyr
               20
<210> 162
   <211> 19
   <212> PRT
   <213> Artificial Sequence
<220>
   <223> Description of Artificial Sequence:peptide
   fragments of human TGF-beta 1
<400> 162
   Trp Ser Leu Asp Thr Gln Tyr Ser Lys Val Leu Ala Leu Tyr Asn Gln
    1               5                  10                  15
   His Asn Pro
<210> 163
   <211> 20
   <212> PRT
   <213> Artificial Sequence
<220>
   <223> Description of Artificial Sequence:peptide
   fragments of human TGF-beta 1
<400> 163
   Gly Ala Ser Ala Ala Pro Cys Cys Val Pro Gln Ala Leu Glu Pro Leu
    1               5                  10                  15
   Pro Ile Val Tyr
               20
<210> 164
   <211> 22
   <212> PRT
   <213> Artificial Sequence
<220>
   <223> Description of Artificial Sequence:peptide
   fragments of human TGF-beta 1
<400> 164
   Tyr Val Gly Arg Lys Pro Lys Val Glu Gln Leu Ser Asn Met Ile Val
    1               5                  10                  15
   Arg Ser Cys Lys Cys Ser
               20
<210> 165
   <211> 40
   <212> PRT
   <213> Artificial Sequence
<220>
   <223> Description of Artificial Sequence:peptide
   fragments of human TGF-beta 1
<400> 165
   Gln Tyr Ser Lys Val Leu Ala Leu Tyr Asn Gln His Asn Pro Gly Ala
    1              5                  10                  15
   Ser Ala Ala Pro Cys Cys Val Pro Gln Ala Leu Glu Pro Leu Pro Ile
               20                  25                  30
   Val Tyr Tyr Val Gly Arg Lys Pro
           35                  40
<210> 166
   <211> 40
   <212> PRT
   <213> Artificial Sequence
<220>
   <223> Description of Artificial Sequence:peptide
   fragments of human TGF-beta 1
<220>
   <221> DISULFID
   <222> (21)
   <223> intermolecular disulfide bridge with SEQ ID No.
   219
<400> 166
   Gln Tyr Ser Lys Val Leu Ala Leu Tyr Asn Gln His Asn Pro Gly Ala
    1              5                  10                  15
   Ser Ala Ala Pro Cys Cys Val Pro Gln Ala Leu Glu Pro Leu Pro Ile
               20                  25                  30
   Val Tyr Tyr Val Gly Arg Lys Pro
           35                  40
<210> 167
   <211> 112
   <212> PRT
   <213> Artificial Sequence
<220>
   <223> Description of Artificial Sequence:peptide
   fragments of human TGF-beta 1
<400> 167
   Ala Leu Asp Thr Asn Tyr Cys Phe Ser Ser Thr Glu Lys Asn Cys Cys
   1               5                   10                  15
   Val Arg Gln Leu Tyr Ile Asp Phe Arg Lys Asp Leu Gly Trp Lys Trp
               20                  25                  30
   Ile His Glu Pro Lys Gly Tyr His Ala Asn Phe Cys Leu Gly Pro Cys
           35                  40                  45
   Pro Tyr Ile Trp Ser Leu Asp Thr Gln Tyr Ser Lys Val Leu Ala Leu
       50                  55                  60
   Tyr Asn Gln His Asn Pro Gly Ala Ser Ala Ala Pro Cys Cys Val Pro
   65                  70                  75                  80
   Gln Ala Leu Glu Pro Leu Pro Ile Val Tyr Tyr Val Gly Arg Lys Pro
                   85                  90                  95
   Lys Val Glu Gln Leu Ser Asn Met Ile Val Arg Ser Cys Lys Cys Ser
               100                 105                 110
<210> 168
   <211> 30
   <212> PRT
   <213> Artificial Sequence
<220>
   <223> Description of Artificial Sequence:peptide
   fragments of human TGF-beta 1
<400> 168
   Ala Leu Asp Thr Asn Tyr Cys Phe Ser Ser Thr Glu Lys Asn Cys Cys
   1               5                   10                  15
   Val Arg Gln Leu Tyr Ile Asp Phe Arg Lys Asp Leu Gly Trp
               20                  25                  30
<210> 169
   <211> 30
   <212> PRT
   <213> Artificial Sequence
<220>
   <223> Description of Artificial Sequence:peptide
   fragments of human TGF-beta 1
<400> 169
   Lys Trp Ile His Glu Pro Lys Gly Tyr His Ala Asn Phe Cys Leu Gly
   1               5                   10                  15
   Pro Cys Pro Tyr Ile Trp Ser Leu Asp Thr Gln Tyr Ser Lys
               20                  25                  30
<210> 170
   <211> 30
   <212> PRT
   <213> Artificial Sequence
<220>
   <223> Description of Artificial Sequence:peptide
   fragments of human TGF-beta 1
<400> 170
   Val Leu Ala Leu Tyr Asn Gln His Asn Pro Gly Ala Ser Ala Ala Pro
   1               5                   10                  15
   Cys Cys Val Pro Gln Ala Leu Glu Pro Leu Pro Ile Val Tyr
               20                  25                  30
<210> 171
   <211> 22
   <212> PRT
   <213> Artificial Sequence
<220>
   <223> Description of Artificial Sequence:peptide
   fragments of human TGF-beta 1
<400> 171
   Tyr Val Gly Arg Lys Pro Lys Val Glu Gln Leu Ser Asn Met Ile Val
   1               5                   10                  15
   Arg Ser Cys Lys Cys Ser
               20
<210> 172
   <211> 30
   <212> PRT
   <213> Artificial Sequence
<220>
   <223> Description of Artificial Sequence:peptide
   fragments of human TGF-beta 1
<400> 172
   Cys Val Arg Gln Leu Tyr Ile Asp Phe Arg Lys Asp Leu Gly Trp Lys
   1               5                   10                  15
   Trp Ile His Glu Pro Lys Gly Tyr His Ala Asn Phe Cys Leu
               20                  25                  30
<210> 173
   <211> 30
   <212> PRT
   <213> Artificial Sequence
<220>
   <223> Description of Artificial Sequence:peptide
   fragments of human TGF-beta 1
<400> 173
   Gly Pro Cys Pro Tyr Ile Trp Ser Leu Asp Thr Gln Tyr Ser Lys Val
   1               5                   10                  15
   Leu Ala Leu Tyr Asn Gln His Asn Pro Gly Ala Ser Ala Ala
               20                  25                  30
<210> 174
   <211> 30
   <212> PRT
   <213> Artificial Sequence
<220>
   <223> Description of Artificial Sequence:peptide
   fragments of human TGF-beta 1
<400> 174
   Pro Cys Cys Val Pro Gln Ala Leu Glu Pro Leu Pro Ile Val Tyr Tyr
   1               5                   10                  15
   Val Gly Arg Lys Pro Lys Val Glu Gln Leu Ser Asn Met Ile
               20                  25                  30
<210> 175
   <211> 413
   <212> PRT
   <213> Homo sapiens
<400> 175
   Met His Tyr Cys Val Leu Ser Ala Phe Leu Ile Leu His Leu Val Thr
   1               5                   10                  15
   Val Ala Leu Ser Leu Ser Thr Cys Ser Thr Leu Asp Met Asp Gln Phe
               20                  25                  30
   Met Arg Lys Arg Ile Glu Ala Ile Arg Gly Gln Ile Leu Ser Lys Leu
           35                  40                  45
   Lys Leu Thr Ser Pro Pro Glu Asp Tyr Pro Glu Pro Glu Glu Val Pro
       50                  55                  60
   Pro Glu Val Ile Ser Ile Tyr Asn Ser Thr Arg Asp Leu Leu Gln Glu
   65                  70                  75                  80
   Lys Ala Ser Arg Arg Ala Ala Ala Cys Glu Arg Glu Arg Ser Asp Glu
                   85                  90                  95
   Glu Tyr Tyr Ala Lys Glu Val Tyr Lys Ile Asp Met Pro Pro Phe Phe
               100                 105                 110
   Pro Ser Glu Asn Ala Ile Pro Pro Thr Phe Tyr Arg Pro Tyr Phe Arg
           115                 120                 125
   Ile Val Arg Phe Asp Val Ser Ala Met Glu Lys Asn Ala Ser Asn Leu
       130                 135                 140
   Val Lys Ala Glu Phe Arg Val Phe Arg Leu Gln Asn Pro Lys Ala Arg
   145                 150                 155                 160
   Val Pro Glu Gln Arg Ile Glu Leu Tyr Gln Ile Leu Lys Ser Lys Asp
                   165                 170                 175
   Leu Thr Ser Pro Thr Gln Arg Tyr Ile Asp Ser Lys Val Val Lys Thr
               180                 185                 190
   Arg Ala Glu Gly Glu Trp Leu Ser Phe Asp Val Thr Asp Ala Val His
           195                 200                 205
   Glu Trp Leu His His Lys Asp Arg Asn Leu Gly Phe Lys Ile Ser Leu
       210                 215                 220
   His Cys Pro Cys Cys Thr Phe Val Pro Ser Asn Asn Tyr Ile Ile Pro
   225                 230                 235                 240
   Asn Lys Ser Glu Glu Leu Glu Ala Arg Phe Ala Gly Ile Asp Gly Thr
                   245                 250                 255
   Ser Thr Tyr Thr Ser Gly Asp Gln Lys Thr Ile Lys Ser Thr Arg Lys
               260                 265                 270
   Lys Asn Ser Gly Lys Thr Pro His Leu Leu Leu Met Leu Leu Pro Ser
           275                 280                 285
   Tyr Arg Leu Glu Ser Gln Gln Thr Asn Arg Arg Lys Arg Ala Leu Asp
       290                 295                 300
   Ala Ala Tyr Cys Phe Arg Asn Val Gln Asp Asn Cys Cys Leu Arg Pro
   305                 310                 315                 320
   Leu Tyr Ile Asp Phe Lys Arg Asp Leu Gly Trp Lys Trp Ile His Glu
                   325                 330                 335
   Pro Lys Gly Tyr Asn Ala Asn Phe Cys Ala Gly Ala Cys Pro Tyr Leu
               340                 345                 350
   Trp Ser Ser Asp Thr Gln His Ser Arg Val Leu Ser Leu Tyr Asn Thr
           355                 360                 365
   Ile Asn Pro Glu Ala Ser Ala Ser Pro Cys Cys Val Ser Gln Asp Leu
       370                 375                 380
   Glu Pro Leu Thr Ile Leu Tyr Tyr Ile Gly Lys Thr Pro Lys Ile Glu
   385                 390                 395                 400
   Gln Leu Ser Asn Met Ile Val Lys Ser Cys Lys Cys Ser
                   405                 410
<210> 176
   <211> 20
   <212> PRT
   <213> Artificial Sequence
<220>
   <223> Description of Artificial Sequence:peptide
   fragments of human TGF-beta 2
<400> 176
   Ala Leu Asp Ala Ala Tyr Cys Phe Arg Asn Val Gln Asp Asn Cys Cys
    1               5                  10                  15
   Leu Arg Pro Leu
               20
<210> 177
   <211> 20
   <212> PRT
   <213> Artificial Sequence
<220>
   <223> Description of Artificial Sequence:peptide
   fragments of human TGF-beta 2
<400> 177
   Tyr Ile Asp Phe Lys Arg Asp Leu Gly Trp Lys Trp Ile His Glu Pro
    1               5                  10                  15
   Lys Gly Tyr Asn
               20
<210> 178
   <211> 20
   <212> PRT
   <213> Artificial Sequence
<220>
   <223> Description of Artificial Sequence:peptide
   fragments of human TGF-beta 2
<400> 178
   Ala Asn Phe Cys Ala Gly Ala Cys Pro Tyr Leu Trp Ser Ser Asp Thr
    1               5                  10                  15
   Gln His Ser Arg
               20
<210> 179
   <211> 20
   <212> PRT
   <213> Artificial Sequence
<220>
   <223> Description of Artificial Sequence:peptide
   fragments of human TGF-beta 2
<400> 179
   Val Leu Ser Leu Tyr Asn Thr Ile Asn Pro Glu Ala Ser Ala Ser Pro
   1 5 10 15
   Cys Cys Val Ser
   20
<210> 180
   <211> 20
   <212> PRT
   <213> Artificial Sequence
<220>
   <223> Description of Artificial Sequence:peptide
   fragments of human TGF-beta 2
<400> 180
   Gln Asp Leu Glu Pro Leu Thr Ile Leu Tyr Tyr Ile Gly Lys Thr Pro
   1 5 10 15
   Lys Ile Glu Gln
   20
<210> 181
   <211> 12
   <212> PRT
   <213> Artificial Sequence
<220>
   <223> Description of Artificial Sequence:peptide
   fragments of human TGF-beta 2
<400> 181
   Leu Ser Asn Met Ile Val Lys Ser Cys Lys Cys Ser
   1 5 10
<210> 182
   <211> 20
   <212> PRT
   <213> Artificial Sequence
<220>
   <223> Description of Artificial Sequence:peptide
   fragments of human TGF-beta 2
<400> 182
   Val Gln Asp Asn Cys Cys Leu Arg Pro Leu Tyr Ile Asp Phe Lys Arg
   1 5 10 15
   Asp Leu Gly Trp
   20
<210> 183
   <211> 20
   <212> PRT
   <213> Artificial Sequence
<220>
   <223> Description of Artificial Sequence:peptide
   fragments of human TGF-beta 2
<400> 183
   Lys Trp Ile His Glu Pro Lys Gly Tyr Asn Ala Asn Phe Cys Ala Gly
     1               5                  10                  15
   Ala Cys Pro Tyr
                20
<210> 184
   <211> 20
   <212> PRT
   <213> Artificial Sequence
<220>
   <223> Description of Artificial Sequence:peptide
   fragments of human TGF-beta 2
<400> 184
   Leu Trp Ser Ser Asp Thr Gln His Ser Arg Val Leu Ser Leu Tyr Asn
     1               5                  10                  15
   Thr Ile Asn Pro
                20
<210> 185
   <211> 20
   <212> PRT
   <213> Artificial Sequence
<220>
   <223> Description of Artificial Sequence:peptide
   fragments of human TGF-beta 2
<400> 185
   Glu Ala Ser Ala Ser Pro Cys Cys Val Ser Gln Asp Leu Glu Pro Leu
     1               5                  10                  15
   Thr Ile Leu Tyr
                20
<210> 186
   <211> 22
   <212> PRT
   <213> Artificial Sequence
<220>
   <223> Description of Artificial Sequence:peptide
   fragments of human TGF-beta 2
<400> 186
   Tyr Ile Gly Lys Thr Pro Lys Ile Glu Gln Leu Ser Asn Met Ile Val
     1               5                  10                  15
   Lys Ser Cys Lys Cys Ser
               20
<210> 187
   <211> 41
   <212> PRT
   <213> Artificial Sequence
<220>
   <223> Description of Artificial Sequence:peptide
   fragments of human TGF-beta 2
<400> 187
   Gln His Ser Arg Val Leu Ser Leu Tyr Asn Thr Ile Asn Pro Glu Ala
     1               5                  10                  15
   Ser Ala Ser Pro Cys Cys Val Ser Gln Asp Leu Glu Pro Leu Thr Ile
                20                  25                  30
   Leu Tyr Tyr Ile Gly Lys Thr Pro Lys
           35                  40
<210> 188
   <211> 41
   <212> PRT
   <213> Artificial Sequence
<220>
   <223> Description of Artificial Sequence:peptide
   fragments of human TGF-beta 2
<220>
   <221> DISULFID
   <222> (21)
   <223> intermolecular disulfide bridge with SEQ ID No.
   220
<400> 188
   Gln His Ser Arg Val Leu Ser Leu Tyr Asn Thr Ile Asn Pro Glu Ala
     1               5                  10                  15
   Ser Ala Ser Pro Cys Cys Val Ser Gln Asp Leu Glu Pro Leu Thr Ile
                20                  25                  30
   Leu Tyr Tyr Ile Gly Lys Thr Pro Lys
           35                  40
<210> 189
   <211> 112
   <212> PRT
   <213> Artificial Sequence
<220>
   <223> Description of Artificial Sequence:peptide
   fragments of human TGF-beta 2
<400> 189
   Ala Leu Asp Ala Ala Tyr Cys Phe Arg Asn Val Gln Asp Asn Cys Cys
     1               5                  10                  15
   Leu Arg Pro Leu Tyr Ile Asp Phe Lys Arg Asp Leu Gly Trp Lys Trp
                20                  25                  30
   Ile His Glu Pro Lys Gly Tyr Asn Ala Asn Phe Cys Ala Gly Ala Cys
            35                  40                  45
   Pro Tyr Leu Trp Ser Ser Asp Thr Gln His Ser Arg Val Leu Ser Leu
        50                  55                  60
   Tyr Asn Thr Ile Asn Pro Glu Ala Ser Ala Ser Pro Cys Cys Val Ser
    65                  70                  75                  80
   Gln Asp Leu Glu Pro Leu Thr Ile Leu Tyr Tyr Ile Gly Lys Thr Pro
                    85                  90                  95
   Lys Ile Glu Gln Leu Ser Asn Met Ile Val Lys Ser Cys Lys Cys Ser
               100                 105                 110
<210> 190
   <211> 30
   <212> PRT
   <213> Artificial Sequence
<220>
   <223> Description of Artificial Sequence:peptide
   fragments of human TGF-beta 2
<400> 190
   Ala Leu Asp Ala Ala Tyr Cys Phe Arg Asn Val Gln Asp Asn Cys Cys
     1               5                  10                  15
   Leu Arg Pro Leu Tyr Ile Asp Phe Lys Arg Asp Leu Gly Trp
                20                  25                  30
<210> 191
   <211> 30
   <212> PRT
   <213> Artificial Sequence
<220>
   <223> Description of Artificial Sequence:peptide
   fragments of human TGF-beta 2
<400> 191
   Lys Trp Ile His Glu Pro Lys Gly Tyr Asn Ala Asn Phe Cys Ala Gly
     1               5                  10                  15
   Ala Cys Pro Tyr Leu Trp Ser Ser Asp Thr Gln His Ser Arg
   20 25 30
<210> 192
   <211> 30
   <212> PRT
   <213> Artificial Sequence
<220>
   <223> Description of Artificial Sequence:peptide
   fragments of human TGF-beta 2
<900> 192
<210> 193
   <211> 22
   <212> PRT
   <213> Artificial Sequence
<220>
   <223> Description of Artificial Sequence:peptide
   fragments of human TGF-beta 2
<400> 193
   Tyr Ile Gly Lys Thr Pro Lys Ile Glu Gln Leu Ser Asn Met Ile Val
   1 5 10 15
   Lys Ser Cys Lys Cys Ser
   20
<210> 194
   <211> 30
   <212> PRT
   <213> Artificial Sequence
<220>
   <223> Description of Artificial Sequence:peptide
   fragments of human TGF-beta 2
<400> 194
   Cys Leu Arg Pro Leu Tyr Ile Asp Phe Lys Arg Asp Leu Gly Trp Lys
   1 5 10 15
   Trp Ile His Glu Pro Lys Gly Tyr Asn Ala Asn Phe Cys Ala
   20 25 30
<210> 195
   <211> 30
   <212> PRT
   <213> Artificial Sequence
<220>
   <223> Description of Artificial Sequence:peptide
   fragments of human TGF-beta 2
<400> 195
   Gly Ala Cys Pro Tyr Leu Trp Ser Ser Asp Thr Gln His Ser Arg Val
     1               5                  10                  15
   Leu Ser Leu Tyr Asn Thr Ile Asn Pro Glu Ala Ser Ala Ser
                20                  25                  30
<210> 196
   <211> 30
   <212> PRT
   <213> Artificial Sequence
<220>
   <223> Description of Artificial Sequence:peptide
   fragments of human TGF-beta 2
<400> 196
   Pro Cys Cys Val Ser Gln Asp Leu Glu Pro Leu Thr Ile Leu Tyr Tyr
     1               5                  10                  15
   Ile Gly Lys Thr Pro Lys Ile Glu Gln Leu Ser Asn Met Ile
                20                  25                  30
<210> 197
   <211> 412
   <212> PRT
   <213> Homo sapiens
<400> 197
   Met Lys Met His Leu Gln Arg Ala Leu Val Val Leu Ala Leu Leu Asn
     1               5                  10                  15
   Phe Ala Thr Val Ser Leu Ser Leu Ser Thr Cys Thr Thr Leu Asp Phe
                20                  25                  30
   Gly His Ile Lys Lys Lys Arg Val Glu Ala Ile Arg Gly Gln Ile Leu
            35                  40                  45
   Ser Lys Leu Arg Leu Thr Ser Pro Pro Glu Pro Thr Val Met Thr His
        50                  55                  60
   Val Pro Tyr Gln Val Leu Ala Leu Tyr Asn Ser Thr Arg Glu Leu Leu
    65                  70                  75                  80
   Glu Glu Met His Gly Glu Arg Glu Glu Gly Cys Thr Gln Glu Asn Thr
                    85                  90                  95
   Glu Ser Glu Tyr Tyr Ala Lys Glu Ile His Lys Phe Asp Met Ile Gln
               100                 105                 110
   Gly Leu Ala Glu His Asn Glu Leu Ala Val Cys Pro Lys Gly Ile Thr
           115                 120                 125
   Ser Lys Val Phe Arg Phe Asn Val Ser Ser Val Glu Lys Asn Arg Thr
       130                 135                 140
   Asn Leu Phe Arg Ala Glu Phe Arg Val Leu Arg Val Pro Asn Pro Ser
   145                 150                 155                 160
   Ser Lys Arg Asn Glu Gln Arg Ile Glu Leu Phe Gln Ile Leu Arg Pro
                   165                 170                 175
   Asp Glu His Ile Ala Lys Gln Arg Tyr Ile Gly Gly Lys Asn Leu Pro
               180                 185                 190
   Thr Arg Gly Thr Ala Glu Trp Leu Ser Phe Asp Val Thr Asp Thr Val
           195                 200                 205
   Arg Glu Trp Leu Leu Arg Arg Glu Ser Asn Leu Gly Leu Glu Ile Ser
       210                 215                 220
   Ile His Cys Pro Cys His Thr Phe Gln Pro Asn Gly Asp Ile Leu Glu
   225                 230                 235                 240
   Asn Ile His Glu Val Met Glu Ile Lys Phe Lys Gly Val Asp Asn Glu
                   245                 250                 255
   Asp Asp His Gly Arg Gly Asp Leu Gly Arg Leu Lys Lys Gln Lys Asp
               260                 265                 270
   His His Asn Pro His Leu Ile Leu Met Met Ile Pro Pro His Arg Leu
           275                 280                 285
   Asp Asn Pro Gly Gln Gly Gly Gln Arg Lys Lys Arg Ala Leu Asp Ala
       290                 295                 300
   Ala Tyr Cys Phe Arg Asn Val Gln Asp Asn Cys Cys Leu Arg Pro Leu
   305                 310                 315                 320
   Tyr Ile Asp Phe Lys Arg Asp Leu Gly Trp Lys Trp Ile His Glu Pro
                   325                 330                 335
   Lys Gly Tyr Asn Ala Asn Phe Cys Ala Gly Ala Cys Pro Tyr Leu Trp
               340                 345                 350
   Ser Ser Asp Thr Gln His Ser Arg Val Leu Ser Leu Tyr Asn Thr Ile
           355                 360                 365
   Asn Pro Glu Ala Ser Ala Ser Pro Cys Cys Val Ser Gln Asp Leu Glu
       370                 375                 380
   Pro Leu Thr Ile Leu Tyr Tyr Ile Gly Lys Thr Pro Lys Ile Glu Gln
   385                 390                 395                 400
   Leu Ser Asn Met Ile Val Lys Ser Cys Lys Cys Ser
                   405                 410
<210> 198
   <211> 20
   <212> PRT
   <213> Artificial Sequence
<220>
   <223> Description of Artificial Sequence:peptide
   fragments of human TGF-beta 3
<400> 198
   Ala Leu Asp Thr Asn Tyr Cys Phe Arg Asn Leu Glu Glu Asn Cys Cys
     1               5                  10                  15
   Val Arg Pro Leu
               20
<210> 199
   <211> 20
   <212> PRT
   <213> Artificial Sequence
<220>
   <223> Description of Artificial Sequence:peptide
   fragments of human TGF-beta 3
<400> 199
   Tyr Ile Asp Phe Arg Gln Asp Leu Gly Trp Lys Trp Val His Glu Pro
     1               5                  10                  15
   Lys Gly Tyr Tyr
               20
<210> 200
   <211> 20
   <212> PRT
   <213> Artificial Sequence
<220>
   <223> Description of Artificial Sequence:peptide
   fragments of human TGF-beta 3
<400> 200
   Ala Asn Phe Cys Ser Gly Pro Cys Pro Tyr Leu Arg Ser Ala Asp Thr
     1               5                  10                  15
   Thr His Ser Thr
               20
<210> 201
   <211> 20
   <212> PRT
   <213> Artificial Sequence
<220>
   <223> Description of Artificial Sequence:peptide
   fragments of human TGF-beta 3
<400> 201
   Val Leu Gly Leu Tyr Asn Thr Leu Asn Pro Glu Ala Ser Ala Ser Pro
   1 5 10 15
   Cys Cys Val Pro
   20
<210> 202
   <211> 20
   <212> PRT
   <213> Artificial Sequence
<220>
   <223> Description of Artificial Sequence:peptide
   fragments of human TGF-beta 3
<400> 202
   Gln Asp Leu Glu Pro Leu Thr Ile Leu Tyr Tyr Val Gly Arg Thr Pro
   1 5 10 15
   Lys Val Glu Gln
   20
<210> 203
   <211> 12
   <212> PRT
   <213> Artificial Sequence
<220>
   <223> Description of Artificial Sequence:peptide
   fragments of human TGF-beta 3
<400> 203
   Leu Ser Asn Met Val Val Lys Ser Cys Lys Cys Ser
   1 5 10
<210> 204
   <211> 20
   <212> PRT
   <213> Artificial Sequence
<220>
   <223> Description of Artificial Sequence:peptide
   fragments of human TGF-beta 3
<400> 204
   Asn Leu Glu Glu Asn Cys Cys Val Arg Pro Leu Tyr Ile Asp Phe Arg
   1 5 10 15
   Gln Asp Leu Gly
   20
<210> 205
   <211> 20
   <212> PRT
   <213> Artificial Sequence
<220>
   <223> Description of Artificial Sequence:peptide
   fragments of human TGF-beta 3
<400> 205
   Trp Lys Trp Val His Glu Pro Lys Gly Tyr Tyr Ala Asn Phe Cys Ser
    1               5                  10                  15
   Gly Pro Cys Pro
               20
<210> 206
   <211> 20
   <212> PRT
   <213> Artificial Sequence
<220>
   <223> Description of Artificial Sequence:peptide
   fragments of human TGF-beta 3
<400> 206
   Tyr Leu Arg Ser Ala Asp Thr Thr His Ser Thr Val Leu Gly Leu Tyr
    1               5                  10                  15
   Asn Thr Leu Asn
               20
<210> 207
   <211> 20
   <212> PRT
   <213> Artificial Sequence
<220>
   <223> Description of Artificial Sequence:peptide
   fragments of human TGF-beta 3
<400> 207
   Pro Glu Ala Ser Ala Ser Pro Cys Cys Val Pro Gln Asp Leu Glu Pro
    1               5                  10                  15
   Leu Thr Ile Leu
               20
<210> 208
   <211> 23
   <212> PRT
   <213> Artificial Sequence
<220>
   <223> Description of Artificial Sequence:peptide
   fragments of human TGF-beta 3
<400> 208
   Tyr Tyr Val Gly Arg Thr Pro Lys Val Glu Gln Leu Ser Asn Met Val
   1 5 10 15
   Val Lys Ser Cys Lys Cys Ser
   20
<210> 209
   <211> 41
   <212> PRT
   <213> Artificial Sequence
<220>
   <223> Description of Artificial Sequence:peptide
   fragments of human TGF-beta 3
<400> 209
   Thr His Ser Thr Val Leu Gly Leu Tyr Asn Thr Leu Asn Pro Glu Ala
   1 5 10 15
   Ser Ala Ser Pro Cys Cys Val Pro Gln Asp Leu Glu Pro Leu Thr Ile
   20 25 30
   Leu Tyr Tyr Val Gly Arg Thr Pro Lys
   35 40
<210> 210
   <211> 41
   <212> PRT
   <213> Artificial Sequence
<220>
   <223> Description of Artificial Sequence:peptide
   fragments of human TGF-beta 3
<220>
   <221> DISULFID
   <222> (21)
   <223> intermolecular disulfide bridge to SEQ ID No. 221
<400> 210
   Thr His Ser Thr Val Leu Gly Leu Tyr Asn Thr Leu Asn Pro Glu Ala
   1 5 10 15
   Ser Ala Ser Pro Cys Cys Val Pro Gln Asp Leu Glu Pro Leu Thr Ile
   20 25 30
   Leu Tyr Tyr Val Gly Arg Thr Pro Lys
   35 40
<210> 211
   <211> 112
   <212> PRT
   <213> Artificial Sequence
<220>
   <223> Description of Artificial Sequence:peptide
   fragments of human TGF-beta 3
<400> 211
   Ala Leu Asp Ala Ala Tyr Cys Phe Arg Asn Val Gln Asp Asn Cys Cys
   1               5                   10                  15
   Leu Arg Pro Leu Tyr Ile Asp Phe Lys Arg Asp Leu Gly Trp Lys Trp
               20                  25                  30
   Ile His Glu Pro Lys Gly Tyr Asn Ala Asn Phe Cys Ala Gly Ala Cys
           35                  40                  45
   Pro Tyr Leu Trp Ser Ser Asp Thr Gln His Ser Arg Val Leu Ser Leu
       50                  55                  60
   Tyr Asn Thr Ile Asn Pro Glu Ala Ser Ala Ser Pro Cys Cys Val Ser
   65                  70                  75                  80
   Gln Asp Leu Glu Pro Leu Thr Ile Leu Tyr Tyr Ile Gly Lys Thr Pro
                   85                  90                  95
   Lys Ile Glu Gln Leu Ser Asn Met Ile Val Lys Ser Cys Lys Cys Ser
               100                 105                 110
<210> 212
   <211> 30
   <212> PRT
   <213> Artificial Sequence
<220>
   <223> Description of Artificial Sequence:peptide
   fragments of human TGF-beta 3
<400> 212
   Ala Leu Asp Ala Ala Tyr Cys Phe Arg Asn Val Gln Asp Asn Cys Cys
   1               5                   10                  15
   Leu Arg Pro Leu Tyr Ile Asp Phe Lys Arg Asp Leu Gly Trp
               20                  25                  30
<210> 213
   <211> 30
   <212> PRT
   <213> Artificial Sequence
<220>
   <223> Description of Artificial Sequence:peptide
   fragments of human TGF-beta 3
<400> 213
   Lys Trp Ile His Glu Pro Lys Gly Tyr Asn Ala Asn Phe Cys Ala Gly
     1               5                  10                  15
   Ala Cys Pro Tyr Leu Trp Ser Ser Asp Thr Gln His Ser Arg
                20                  25                  30
<210> 214
   <211> 30
   <212> PRT
   <213> Artificial Sequence
<220>
   <223> Description of Artificial Sequence:peptide
   fragments of human TGF-beta 3
<400> 214
   Val Leu Ser Leu Tyr Asn Thr Ile Asn Pro Glu Ala Ser Ala Ser Pro
     1               5                  10                  15
   Cys Cys Val Ser Gln Asp Leu Glu Pro Leu Thr Ile Leu Tyr
                20                  25                  30
<210> 215
   <211> 22
   <212> PRT
   <213> Artificial Sequence
<220>
   <223> Description of Artificial Sequence:peptide
   fragments of human TGF-beta 3
<400> 215
   Tyr Ile Gly Lys Thr Pro Lys Ile Glu Gln Leu Ser Asn Met Ile Val
     1               5                  10                  15
   Lys Ser Cys Lys Cys Ser
                20
<210> 216
   <211> 30
   <212> PRT
   <213> Artificial Sequence
<220>
   <223> Description of Artificial Sequence:peptide
   fragments of human TGF-beta 3
<400> 216
   Cys Leu Arg Pro Leu Tyr Ile Asp Phe Lys Arg Asp Leu Gly Trp Lys
     1               5                  10                  15
   Trp Ile His Glu Pro Lys Gly Tyr Asn Ala Asn Phe Cys Ala
                20                  25                  30
<210> 217
   <211> 30
   <212> PRT
   <213> Artificial Sequence
<220>
   <223> Description of Artificial Sequence:peptide
   fragments of human TGF-beta 3
<400> 217
   Gly Ala Cys Pro Tyr Leu Trp Ser Ser Asp Thr Gln His Ser Arg Val
    1               5                  10                  15
   Leu Ser Leu Tyr Asn Thr Ile Asn Pro Glu Ala Ser Ala Ser
               20                  25                  30
<210> 218
   <211> 30
   <212> PRT
   <213> Artificial Sequence
<220>
   <223> Description of Artificial Sequence:peptide
   fragments of human TGF-beta 3
<400> 218
   Pro Cys Cys Val Ser Gln Asp Leu Glu Pro Leu Thr Ile Leu Tyr Tyr
    1               5                  10                  15
   Ile Gly Lys Thr Pro Lys Ile Glu Gln Leu Ser Asn Met Ile
               20                  25                  30
<210> 219
   <211> 40
   <212> PRT
   <213> Artificial Sequence
<220>
   <223> Description of Artificial Sequence:peptide
   fragments of human TGF-beta 1
<220>
   <221> DISULFID
   <222> (21)
   <223> intermolecular disulfide bridge with SEQ ID No. 166
<400> 219
   Gln Tyr Ser Lys Val Leu Ala Leu Tyr Asn Gln His Asn Pro Gly Ala
    1               5                  10                  15
   Ser Ala Ala Pro Cys Cys Val Pro Gln Ala Leu Glu Pro Leu Pro Ile
               20                  25                  30
   Val Tyr Tyr Val Gly Arg Lys Pro
           35                  40
<210> 220
   <211> 41
   <212> PRT
   <213> Artificial Sequence
<220>
   <221> DISULFID
   <222> (21)
   <223> intermolecular disulfide bridge to SEQ ID No. 188
<220>
   <223> Description of Artificial Sequence:peptide
   fragments of human TGF-beta 2
<400> 220
   Gln His Ser Arg Val Leu Ser Leu Tyr Asn Thr Ile Asn Pro Glu Ala
     1               5                  10                  15
   Ser Ala Ser Pro Cys Cys Val Ser Gln Asp Leu Glu Pro Leu Thr Ile
                20                  25                  30
   Leu Tyr Tyr Ile Gly Lys Thr Pro Lys
            35                  40
<210> 221
   <211> 41
   <212> PRT
   <213> Artificial Sequence
<220>
   <223> Description of Artificial Sequence:peptide
   fragments of human TGF-beta 3
<220>
   <221> DISULFID
   <222> (21)
   <223> intermolecular disulfide bridge to SEQ ID No. 210
<400> 221
   Thr His Ser Thr Val Leu Gly Leu Tyr Asn Thr Leu Asn Pro Glu Ala
     1               5                  10                  15
   Ser Ala Ser Pro Cys Cys Val Pro Gln Asp Leu Glu Pro Leu Thr Ile
                20                  25                  30
   Leu Tyr Tyr Val Gly Arg Thr Pro Lys
            35                  40

## Description

### Field of the invention

This invention relates to pharmaceutical compositions and their use in treating neoplasms, in one preferred embodiment to neoplasms of the brain such as glioma, glioblastoma or astrocytoma.

Antineoplastic chemotherapeutic agents and radiation are the most common agents and methods, besides surgery for the treatment of neoplasms. Antineoplastic chemotherapeutic agents comprise e.g. alkylating agents, antimetabolites and alkaloids derived from plants. The common effect of these antineoplastic chemotherapeutic agents and radiation is the unspecific inhibition of the cell proliferation and the unspecific induction of cell death respectively, by a wide range of different mechanisms not completely discovered so far.

The inhibition of cell proliferation and the induction of cell death, respectively, primarily influence rapidly growing cells such as tumor cells. But at the same time the proliferation of other rapidly growing cells such as cells of the hair follicle, colon mucosa cells and also immune cells is inhibited. The immune cells inhibited are for example T-lymphocytes, B-lymphocytes, natural killer cells, granulocytes, macrophages, microglia cells as well as the respective precursor cells of the bone marrow. The administration of antineoplastic chemotherapeutic agents unspecifically inhibiting cell growth is therefore associated with severe side effects and general suppression of the function of the immune system, which has been proven by a lot of "in vitro" and "in vivo" results. For example, dacarbazine which cannot be clearly classified according to standard classification so far is reported to inhibit the humoral and cell-mediated immune response in mouse cells (Giampietri 1978, Nardelli 1984). The same results can be found for temozolomide which is an active metabolite of dacarbazine. Further "in vitro" studies of temozolomide show inhibition of cytotoxicity of lymphocyte activated killer cells (Alvino et al. 1999). CCNU (lomustine) as a representative for alkylating antineoplastic agents was shown to suppress both T-cells and B-cells (Bernego et al. 1984) by e.g. suppressing T-cell mediated cytotoxicity and further suppresses T-cell mediated cytotoxicity (Einstein et al. 1975). Further "in vitro" comparative studies of the alkylating agent cyclophosphamide, the antimetabolite 5-fluorouracil, the alkaloid vincristin and the antibiotic doxorubicine have in common to clearly show suppression of the cytotoxic T-cell function (Gereis et al. 1987).

Whereas the chemotherapy of some neoplasms is very successful, many neoplasms are accompanied by a poor life expectancy.

Another approach in the therapy of neoplasms is the stimulation of the immune system. There is a wide range of stimulators of the function of the immune system and/or the immune cells e.g. immune cell attracting substances, viruses and molecules involved in antigen processing, presentation or transporting, fusion cells of dendritic and tumor cells. Antagonists of substances downregulating the function of the immune system are regarded as stimulators of the immune system as well.

As a common principle these immune stimulators employ the ability of the immune system to selectively kill "foreign" tumor cells while sparing other fast growing "self" cells. This is of course a superior approach for treating neoplasms compared to unspecific inhibition of all growing cells or unspecific destruction of cells of an organism, respectively, which is the principle of the above mentioned antineoplastic chemotherapeutic agents as well as of radiation.

One example for a potent inhibitor of the immune system is TGF-beta (transforming growth factor-beta) mediating the neoplasms escape from immunosurveillance (Wojtowicz-Praga, S. 1997). Cellular immunity is highly suppressed in patients suffering from neoplasms producing high levels of TGF-beta (de Visser, K.E. et al. 1999).

Using a substance specifically inhibiting the TGF-beta production and thus stimulating the immune system is a promising approach for the treatment of neoplasms (Stauder, G. et al. 2003).

Despite these promising results the tumor therapy with immunostimulators seems to have margins at least in very quick growing tumors.

Therefore there is an urgent need for the development of new therapeutics also for the treatment of fast growing neoplasms that are more reliable, have less side effects and increase the life spans of patients suffering from neoplasms.

In a clinical phase I/II study, upon administration of an immunostimulatory agent (antagonist of the immunosuppressor TGF-beta), we surprisingly recognized that the median overall survival of patients treated with an antineoplastic chemotherapeutic agent before the treatment with this immunostimulatory agent was clearly longer compared to patients not pre-treated with all antineoplastic chemotherapeutic agent.

Since the antineoplastic agents suppress the immune system by inhibiting the proliferation of the immunocompetent cells, as described above, up to now the approach of combining these antineoplastic agents with stimulators of the immune system in human beings were deemed not to be an appropriate approach for tumor therapy or tumor medicaments. In the literature reporting about neoplasm therapy by stimulation of the immune system it is emphasized that there has to be a sufficient time delay between the administration of a chemotherapeutic agent and a substance stimulating an immune response (e.g. Timmermann, J. M. 2002).

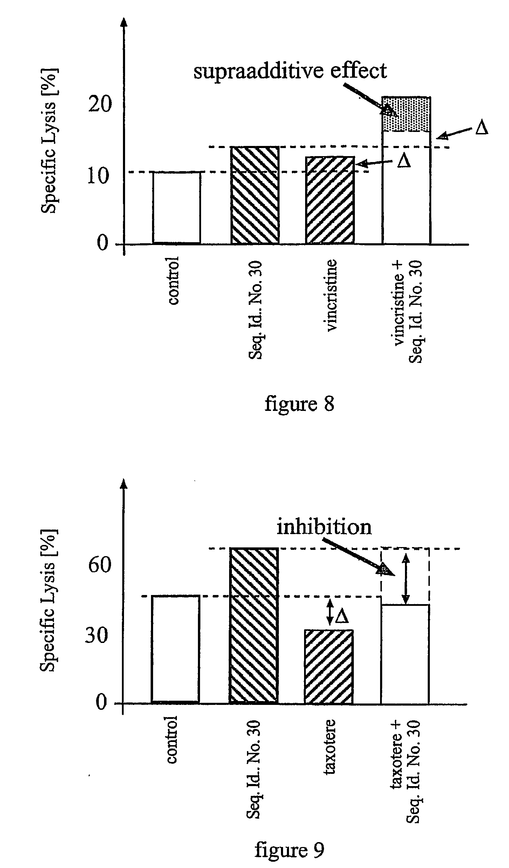

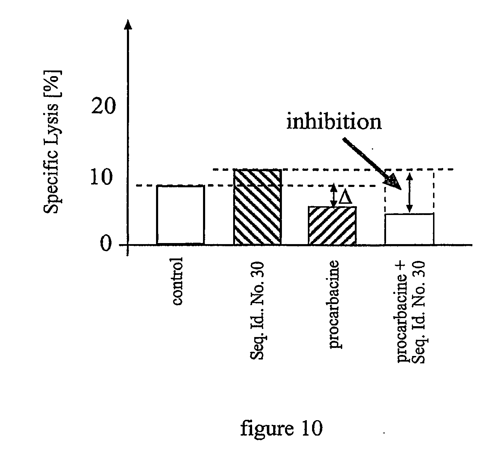

This inhibitory effect of chemotherapeutics on cells of the immune system also was proven in experiments. In these experiments cells of the immune system were treated with antineoplastic therapeutics and stimulators of the immune system, namely antisense oligonucleotides inhibiting TGF-beta. These assays are described in experiment 4 and the results are depicted in figures 9 and 10.

### Summary of the invention

Surprisingly patients treated with a combination of an antineoplastic chemotherapeutic agent selected from the group consisting of BCNU, CCNU, gemcitabine, temozolomide and vincristine and at least one TGF-beta2 antisense oligonucleotide hybridising with an area of the messenger RNA (m-RNA) and/or DNA encoding TGF-beta2 selected from the group consisting of SEQ ID NO: 28 to 30 according to this invention showed significant longer life spans compared to patients treated with either of these therapeutics.

Thus, the invention comprises a pharmaceutical composition with a stimulator of the function of the immune system and/or immune cells and substances inhibiting cell proliferation and/or inducing cell death.

The invention is a pharmaceutical composition comprising at least one antagonist of TGF-beta which is a TGF-beta2 antisense oligonucleotide selected from the group consisting of SEQ ID No. 28 to 30 and at least one antineoplastic chemotherapeutic agent selected from the group consisting of BCNU, CCNU, gemcitabine, temozolomide and vincristine. The at least two substances are mixed or are separate.

The TGF-beta antagonist is selected from the group of TGF-beta2 antisense oligonucleotides hybridising with an area of the messenger RNA (m-RNA) and/or DNA encoding TGF-beta2.

Pharmaceutical composition of the present invention is used in treating neoplasms. The substances for use in stimulating the function of the immune system and/or the immune cells are administered with substances inhibiting cell proliferation and/or inducing cell death according to the present inevntion. The substances are administered by any known route in the art for administering medicaments.

The at least two substances of the pharmaceutical compositions according to this invention are mixed together or separately, optionally in the same carrier formulation or in separate pharmaceutical carriers.

The treatment of a patient suffering from unwanted neoplasms with a pharmaceutical composition as described above, in a preferred embodiment additionally with radiation is also part of this invention.

The at least two substances of the pharmaceutical compositions according to this invention are administered in parallel, in sequence, through the same route or different routes, together with the radiation, before or after the radiation.

Surprisingly patients suffering from neoplasms that were treated with at least one substance stimulating the immune system and/or the immune cells together with a substance inhibiting the cell proliferation and/or inducing cell death, and/or radiation according to the present invention show clearly longer life spans compared to patients treated with each of these medicaments and/or therapies alone.

This can lead to a reduction of the dosage of one of these medicaments and/or therapies being administered and thus to the reduction of potential undesireable side effects.

### Figures

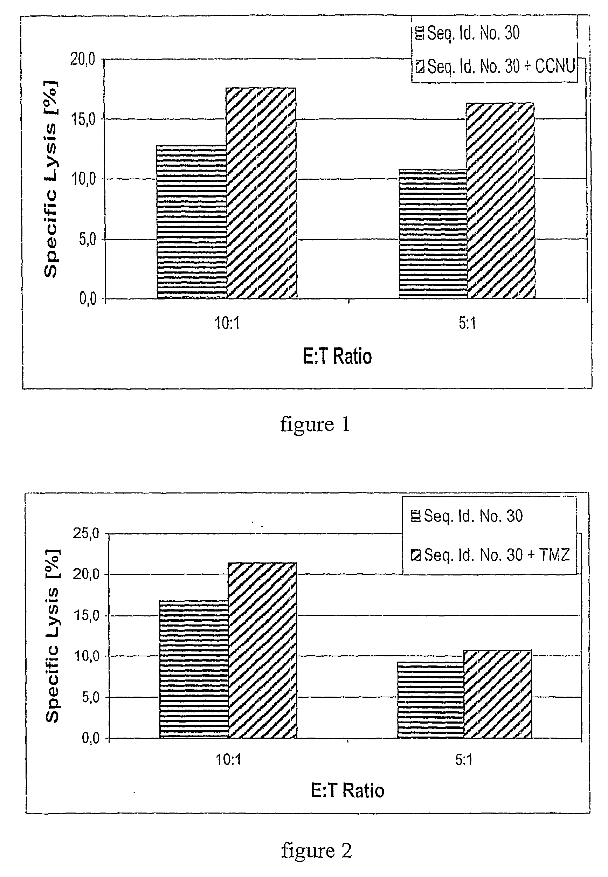

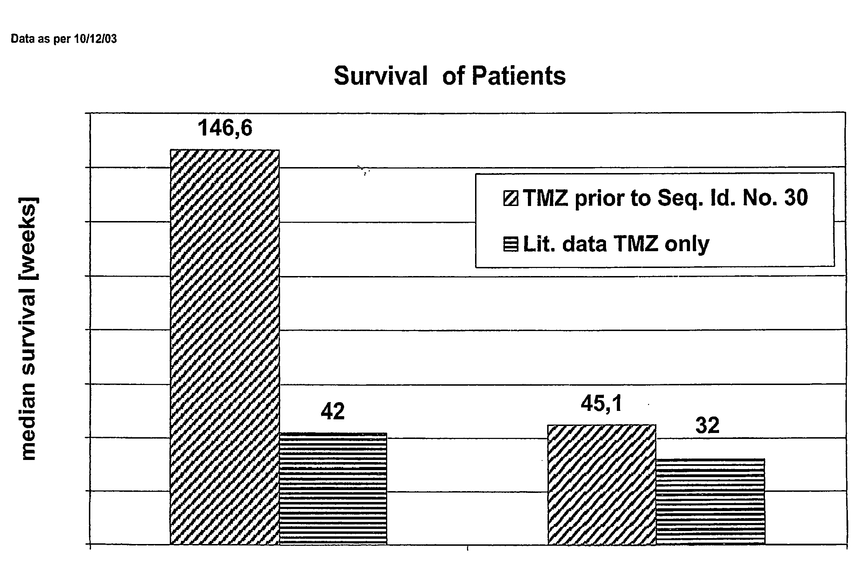

**Figure 1** depicts a comparative study of lymphokine activated killer cell (LAK cell) mediated cytotoxicity on glioma cells. One part of the cells was incubated with the TGF-beta 2 specific antisense oligonucleotide with Seq. Id. No. 30, the other part was additionally treated with CCNU. The figure clearly points out that the cytotoxic activity of LAK cells treated with CCNU in combination with TGF-beta 2 specific antisense oligonucleotide with Seq. Id. No. 30 is superior compared to LAK cells treated with only TGF-beta 2 specific antisense oligonucleotide with Seq. Id. No. 30. 5x10⁶ PBMC (peripheral blood mononuclear cells) were cultivated in 4 µL RPMI 1640 medium supplemented with 10% fetal calf serum, in the presence of 10 ng/ml rh IL-2 (recombinant human interleukin 2), in 5% CO2 atmosphere at 37°C. The first 3 days 5 µM TGF-beta 2 specific antisense oligonucleotide with Seq. Id. No. 30 was added. After that one part of the cells was incubated with 10 µM CCNU for an additional 6 h. Cell-mediated cytotoxicity, quantified by CARE-LASS assay (Lichtenfels et al. 1994), of LAK cells treated with TGF-beta 2 specific antisense oligonucleotide with Seq. Id. No. 30 (horizontal hachures) was compared to LAK cells treated with TGF-beta 2 specific antisense oligonucleotide with Seq. Id. No. 30 in combination with CCNU (diagonal hachures). Indicated are means ± SD of quadruplicates.
**Figure 2** depicts a comparative study of lymphokine activated killer cell (LAK cell) mediated cytotoxicity on glioma cells. One part of the cells was incubated with the TGF-beta 2 specific antisense oligonucleotide with Seq. Id. No. 30 the other part was subsequently treated with Temozolomid (TMZ). The figure clearly points out that the cytotoxic activity of LAK cells treatment with temozolomid after the treatment with TGF-beta 2 specific antisense oligonucleotide with Seq. Id. No. 30 is superior compared to LAK cells treated only with TGF-beta 2 specific antisense oligonucleotide with Seq. Id. No. 30. 5x10⁶ LAK were cultivated in RPMI medium supplemented with 10% fetal calf serum, in the presence of 10 ng/ml rh IL-2 (recombinant human interleukin 2), in 5% CO2 atmosphere at 37°C. The first 3 days 5 µM TGF-beta 2 specific antisense oligonucleotide with Seq. Id. No. 30 was added. Cell-mediated cytotoxicity was then quantified by CARE-LASS assay (Lichtenfels et al. 1994) in one part of the cells without further treatment (only TGF-beta 2 specific antisense oligonucleotide with Seq. Id. No. 30, horizontal hachures), in the other part in the presence of 30 µM temozolomid (TMZ, diagonal hachures). Indicated are means ± SD of quadruplicates.
**Figure 3** depicts survival data of patients treated with the TGF-beta antisense oligonucleotide with Seq. Id. No. 30 after treatment with temozolomide according to standard schedule compared to the median overall survival time of patients treated with temozolomide only according to standard schedule. Survival time is given from start of first chemotherapy after tumor recurrence. Median overall survival time in the clinical study is evaluated from 3 patients with anaplastic astrocytoma and 10 patients with glioblastoma. The survival data are compared to the survival data of the literature. Our data reveal longer median overall survival times if applying TGF-beta 2 specific antisense oligonucleotide with Seq. Id. No. 30 following temozolomide than the comparable published data for temozolomide alone: 146.6 weeks vs. 42.0 weeks for patients suffering from anaplastic astrocytoma and 45.1 weeks versus 32.0 weeks for patients suffering from glioblastoma.

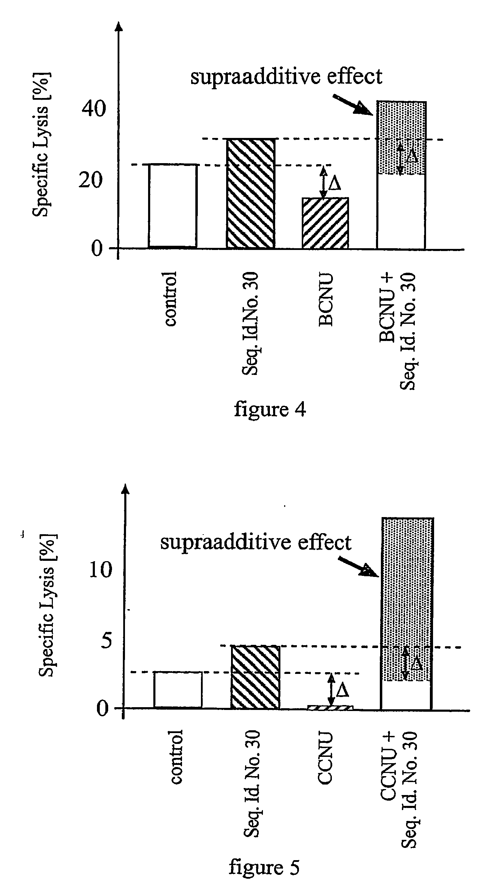

**Figure 4** depicts the specific lysis of tumor cells in an in vitro assay with A-172 cell line performed according to descriptions in example 7 in a ratio of effector cells to target cells of 20 : 1. Antisense oligonucleotide with Sequence Id. No. 30 was under test at a concentration of 5 micrMol enhancing cell lysis. In contrast BCNU at a concentration of 4 µM inhibited LAK cell induced cell lysis, which indicates its immunosuppressive effect. Surprisingly, the cytolytic effect of Seq. Id. No. 30 (5 microM) was enlarged supraadditively in combination with BCNU (4 µM) (specific cell lysis of control: 25.2 %, BCNU 15.6 %, Seq. Id. No. 30: 29.4 %, Seq. Id. No. 30 in combination with BCNU: 40.7 %).
**Figure 5** depicts the specific lysis of tumor cells in an in vitro assay with A-172 cell line performed according to descriptions in example 7 in a ratio of effector cells to target cells of 1.25 : 1. Antisense oligonucleotide with Sequence Id. No. 30 was under test at a concentration of 5 microMol enhancing cell lysis. In contrast CCNU at a concentration of 10 µM inhibited LAK cell induced cell lysis, which indicates its immunosuppressive effect. Surprisingly, the cytolytic effect of Seq. Id. No. 30 (5 microM) was enlarged supraadditively in combination with CCNU (10 µM) (specific cell lysis of control:2.6 %, CCNU 0.5 %, Seq. Id. No. 30: 4.4 %, Seq. Id. No. 30 in combination with CCNU: 13.3 %).

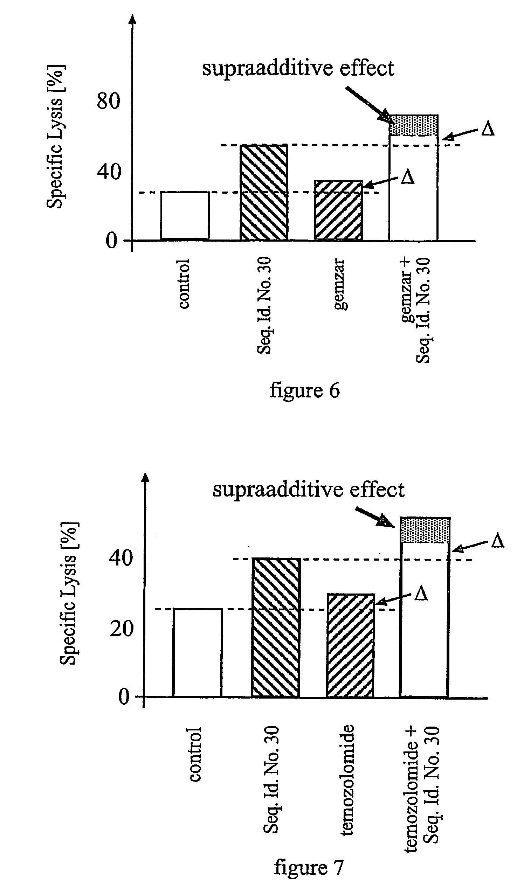

**Figure 6** depicts the specific lysis of tumor cells in an in vitro assay with Hup-T3 cell line performed according to descriptions in example 7 in a ratio of effector cells to target cells of 10:1. Antisense oligonucleotide with Sequence Id. No. 30 was under test at a concentration of 5 microM enhancing cell lysis. In contrast gemzar at a concentration of 20 µg/ml inhibited LAK cell induced cell lysis, which indicates its immunosuppressive effect. Surprisingly, the cytolytic effect of Seq. Id. No. 30 (5 microM) was enlarged supraadditively in combination with gemzar (20 µg/ml) (specific cell lysis of control: 32.9 %, gemzar 34.5 %, Seq. Id. No. 30: 59.5 %, Seq. Id. No. 30 in combination with gemzar: 75.4 %).
**Figure 7** depicts the specific lysis of tumor cells in an in vitro assay with A-172 cell line performed according to descriptions in example 7 in a ratio of effector cells to target cells of 10 :1. Antisense oligonucleotide with Sequence Id. No. 30 was under test at a concentration of 5 microMol enhancing cell lysis. A very small increase of LAK cell induced cell lysis could be observed with temozolomide at a concentration of 50 µM. But, surprisingly, the cytolytic effect of Seq. Id. No. 30 (5 microM) was enlarged supraadditively in combination with temozolomide (50 µM) (specific cell lysis of control: 25.2 %, temozolomide 31.3 %, Seq. Id. No. 30: 39.2 %, Seq. Id. No. 30 in combination with temozolomide: 50.4 %).
**Figure 8** depicts the specific lysis of tumor cells in an in vitro assay with A-172 cell line performed according to descriptions in example 7 in a ratio of effector cells to target cells of 2.5 :1. Antisense oligonucleotide with Sequence Id. No. 30 was under test at a concentration of 5 microMol enhancing cell lysis. A very small increase of LAK cell induced cell lysis could be observed with vincristine at a concentration of 0.04 pmol/ml. But, surprisingly, the cytolytic effect of Seq. Id. No. 30 (5 microM) was enlarged supraadditively in combination with vincristine (0.04 pmol/ml) (specific cell lysis of control: 10.1 %, vincristine 12.6 %, Seq. Id. No. 30: 13.9 %, Seq. Id. No. 30 in combination with vincristine: 20.5 %).
**Figure 9** depicts the specific lysis of tumor cells in an in vitro assay with NCL-H661 cell line performed according to descriptions in example 7 in a ratio of effector cells to target cells of 1.25 :1. Antisense oligonucleotide with Sequence Id. No. 14 was under test at a concentration of 5 microMol enhancing cell lysis. In contrast taxotere reduced LAK induced cell lysis at a concentration of 0.37 µg/ml. But in this case the cytolytic effect of Seq. Id. No. 14 (5 microM) was reduced in combination with taxotere (0.37 µg/ml) (specific cell lysis of control: 49.6 %, taxotere 30.5 %, Seq. Id. No. 14: 65.3 %, Seq. Id. No. 30 in combination with taxotere: 39.7 %).
**Figure 10** depicts the specific lysis of tumor cells in an in vitro assay with A-172 cell line performed according to descriptions in example 7 in a ratio of effector cells to target cells of 5 :1. Antisense oligonucleotide with Sequence Id. No. 30 was under test at a concentration of 5 microMol enhancing cell lysis. In contrast procarbacine reduced LAK induced cell lysis at a concentration of 3 nmol/ml. But in this case the cytolytic effect of Seq. Id. No. 30 (5 microM) was reduced in combination with procarbacine (3 nmol/ml). (specific cell lysis of control: 8.31 %, procarbacine 6.1 %, Seq. Id. No. 14: 16.4 %, Seq. Id. No. 30 in combination with procarbacine: 5.7 %).

### Detailed description of the invention

The pharmaceutical composition of the present invention is for use in any mammal. Examples of mammal to which the composition may be usefully applied include laboratory animals, including rodents such as mice, rats and guinea pigs; farm animals such as cows, sheep, pigs and oats; pet animals such as dogs and cats; and primates such as monkeys, apes and humans. The invention is most preferably applied in human clinical situations, particularly where the patient is undergoing immunosuppressive therapy after organ or tissue transplantation, or any other form of surgery where suppression of the immune system of the patient is indicated. However, other mammals may also benefit from the practice of the invention. These other high value animals such as horses and fur animals such as mink.

In one embodiment of this invention the at least one stimulator of the function of the immune cells and/or the immune system and at least one substance inhibiting the cell proliferation and/or inducing cell death of the present invention is a mixture of these at least two components pure or in a pharmaceutical acceptable carrier also herein referred to as combination.

In another embodiment of this invention the at least one stimulator of the function of the immune cells and/or the immune system and at least one substance inhibiting the cell proliferation and/or inducing cell death are separate in one pharmaceutical composition of the present invention. Each of these parts being pure or in a pharmaceutical acceptable carrier. The at least two parts of the pharmaceutical composition have the same or different pharmaceutical acceptable carriers. To these separate parts of a pharmaceutical composition is also referred to herein as combination.

Immune cells are lymphoid cells, such as T cells, B cells, NK cells (natural killer cells), NK T cells (natural killer T cells), granulocytes, such as neutrophils, eosinophils, basophils, and mononuclear cells such as monocytes, macrophages, dendritic cells and mast cells.

TGF-beta2 antagonists inhibiting the production of TGF-beta are antisense oligonucleotides hybridising with an area of the messenger RNA (mRNA) of TGF-beta2 and/or the DNA encoding TGF-beta2 and by this inhibit the production of TGF-beta.

The terms "nucleic acid" and "oligonucleotide" refer to multiple nucleotides (i.e. molecules comprising a sugar, e.g. ribose or deoxyribose) linked to a phosphate group and to a variable organic base, which is either a substituted pyrimidine, e.g. cytosine (C), thymine (T) or uracil (U) or a substituted purine, e.g. adenine (A) or guanine (G) or a modification thereof. As used herein, the terms refer to oligoribonucleotides as well as oligodeoxyribonucleotides. The terms shall also include oligonucleosides (i.e. a oligonucleotide without the phosphate) and any other organic base containing polymer. The nucleic acids may be double-stranded or single-stranded. Double-stranded molecules may be more stable in vivo, while single-stranded molecules may have increased activity. The nucleotides have lengths between about 12 to about 32 nucleotides.

As used herein with respect to linked units of a nucleic acid, "linked" or "linkage" means two entities are bound to one another by any physicochemical means. Any linkage known to those of ordinary skill in the art, covalent or noncovalent, is embraced. Natural linkages, which are those ordinarily found in nature connecting the individual units of a nucleic acid, are most common. The individual units of a nucleic acid may be linked, however, by synthetic or modified linkages.

In one embodiment the respective ends of this linear polymeric structure can be further joined to form a circular structure. However, open linear structures are generally preferred. Within the oligonucleotides structure, the phosphate groups are commonly referred to as forming the internucleoside backbone of the oligonucleotide. The normal linkage or backbone of RNA and DNA is a 3'to 5'phosphodiester linkage.

In one embodiment the terms "nucleic acids", "nucleotides", "oligonucleotides" respectively "antisense oligonucleotides" are substances stimulating the function of the immune system and/or the immune cells and/or are antagonists of TGF-beta2 as described herein They comprise DNA- or RNA-fragments coding for TGF-beta2 and are the respective antisense nucleotides.

TGF-beta2 antisense oligonucleotides of the present invention include at least one sequence set forth as SEQ ID NOs: 28-30.

Oligonucleotides or nucleic acids include oligonucleotides having non-naturally occurring portions with similar function. Such modified or substituted oligonucleotides are often preferred over native forms because of desirable properties such as, for example, enhanced cellular uptake, enhanced affinity for nucleic acid target (e.g. protein), altered intracellular localization and increased stability in the presence of nucleases. Modifications of the oligonucleotides as used herein comprises any chemical modifications of the sugar, the base moiety and/or the internucleoside linkage.

In one embodiment nucleic acids or oligonucleotides with a covalently modified base and/or sugar include for example nucleic acids having backbone sugars which are covalently attached to low molecular weight organic groups other than a hydroxyl group at the 3' and/or 2' position and other than a phosphate group at the 5' position. Thus modified nucleic acids may include a 2'-O-alkylated ribose group. In yet another embodiment modified nucleic acids include sugars such as arabinose instead of ribose. Thus the nucleic acids may be heterogeneous in backbone composition thereby containing any possible combination of polymer units linked together such as peptide-nucleic acids (which have amino acid backbone with nucleic acid bases). In some embodiments the nucleic acids are homogeneous in backbone composition.

The substituted purines and pyrimidines of the nucleic acids include standard purines and pyrimidines such as cytosine as well as base analogs such as substituted bases (Wagner et al. 1996). Purines and pyrimidines include but are not limited to adenine, cytosine, guanine, thymine, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The single nucleotides in each oligonucleotide may contain the same modifications, may contain combinations of these modifications, or may combine these modifications with phosphodiester linkages. Methods of rendering oligonucleotide nuclease resistant include, but are not limited to, covalently modifying the purine or pyrimidine bases. For example, bases may be methylated, hydroxymethylated, or otherwise substituted (e.g., glycosylated) such that the oligonucleotides or polynucleotides are rendered substantially acid and nuclease resistant.

In a preferred embodiment, at least one end-block on the oligonucleotide is a biotin, biotin analog, avidin, or avidin analog. These molecules have the ability to both block the degradation of the protected oligonucleotide and provide means for high affinity attachment of the modified nucleic acids to the solid support. Avidin and biotin derivatives which can be used to prepare the reagents of this invention include streptavidin, succinylated avidin, monomeric avidin, biocytin (biotin-epsilon-N-lysine), biocytin hydrazide, amine or sulfhydryl derivatives of 2-iminobiotin and biotinyl-epsilon-aminocaproic acid hydrazide. Additional biotin derivatives, such as biotin-N-hydroxysuccinimide ester, biotinyl-epsilon-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)biocytin, can also be used as end-blocking groups on the polynucleotides of the present invention.

In another embodiment the ring structure of the ribose group of the nucleotides in the modified oligonucleotide has an oxygen in the ring structure substituted with N-H, N-R (with R being an alkyl or aryl substituent), S and/or methylene.

In yet another embodiment the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, U.S. Pat. Nos.: 5,539,082 ; 5,714,331; and 5,719,262 , each of which is herein incorporated by reference. Further teaching of PNA compounds can be found in Nielsen et al. (1991).

Further modified oligonucleotide backbones include, for example, phosphorothioates, chiral phospherothioates, phosphorodithioates, phosphorotriesters, aminoalkylphosphorotriesters, methyl- and other alky-phosphonates including 3'-alkylene phophonates and chiral phosphonates, phosphinates, phosphoramidates, including 3'-aminophosphoramidate and aminoalkylphosphoramidates, thionophosphoramidates, thionoalkylphosphonates, thionoalkylphosphotriesters, and boranophosphates having norm 3'-5'linkages, 2'-5'linked analogs of these, and those having inverted polarity wherein the adjacent pairs of nucleoside units are linked 3'-5'to 5'-3' or 2'-5' to 5'-2'. Various salts, mixed salts, and free acid forms are also included.

In embodiments at least one nucleotide of an oligonucleotide is modified as described in one of the modifications above. The modification can either cover the oligonucleotide continuously or irregularly.

In yet another embodiment at least two modifications as described above are combined within one oligonucleotide.

In another embodiment the 1 to about 12 or 1 to about 8 or 1 to about 4 or 1 to about 2 oligonucleotides and/or nucleotide linkages at the 3' and/or 5'end of the oligonucleotide are modified as described above.

The oligonucleotides of this invention are hybridizing TGF-beta2 selected from the group consisting of SEQ ID No. 28 to 30. Comprising in the context of this invention means that one of the oligonucleotides of the sequence listing is part of the antisense oligonucleotide of the respective m-RNA. In one embodiment even the complete antisense oligonucleotide of the m-RNA of the target is an immunostimulator in the meaning of this invention. In yet another embodiment any part of the antisense m-RNA of a target negatively influencing the function of the immune system is within the scope of this invention.

For use in the instant invention, the nucleic acids can be synthesized de novo using any of a number of procedures well known in the art. Such compounds are referred to as 'synthetic nucleic acids.' For example, the b-cyanoethyl phosphoramidite method (Beaurage et al. 1981); nucleoside H-phosphonate method (Garegg et al. 1986, Froehler et al. 1986, Garegg et al. 1986, Gaffney et al. 1988). These chemistries can be performed by a variety of automated oligonucleotide synthesizers available in the market.

Alternatively, nucleic acids can be produced on a large scale in plasmids, (see, e.g., Sambrook, et al. 1989) and separated into smaller pieces or administered whole. Nucleic acids can be prepared from existing nucleic acid sequences (e.g., genomic or cDNA) using known techniques, such as those employing restriction enzymes, exonucleases or endonucleases. Nucleic acids prepared in this manner are referred to as isolated nucleic acids. The term "antineoplastic nucleic acid" encompasses both synthetic and isolated antineoplastic nucleic acids.

Modified backbone nucleic acids, such as those having phosphorothioates bonds may be synthesized using automated techniques employing, for example, phosphoramidate or H-phosphonate chemistries. Aryl- and alkyl-phosphonates can be made, e.g., as described in U.S. Pat. No. 4,469,863. Alkyiphosphotriesters, in which the charged oxygen moiety is alkylated as described in U.S. Pat. No. 5,023,243 and European Patent No. 092,574, can be prepared by automated solid phase synthesis using commercially available reagents. Methods for making other nucleic acid backbone modifications and substitutions have been described (Uhlmann et al. 1990, Goodchild 1990).

Phosphorothioates may be synthesized using automated techniques employing either phosphoramidate or H-phosphonate chemistries. Aryl- and alkyl-phosphonates can be made, e.g., as described in U.S. Pat. No. 4,469,863; and alkylphosphotriesters (in which the charged oxygen moiety is alkylated as described in U.S. Pat. No. 5,023,243 and European Patent No. 092,574) can be prepared by automated solid phase synthesis using commercially available reagents. Methods for making other DNA backbone modifications and substitutions have been described (Uhlmann, E. et al. 1990, Goodchild, J. 1990).

As used herein, the term "neoplasm" means new and abnormal growth or formation of tissue and/or blood cells in the body of a organism. The unwanted neoplasms include, but are not limited to, solid tumors; blood born tumors such as leukemias, acute or chronic myelotic or lymphoblastic leukemia; tumor metastasis; benign tumors, for example hemangiomas, acoustic neuromas, neurofibromas, trachomas, and pyogenic granulomas; pre-malignant tumors; astrocytoma, comprising pilocyt. astrocytoma WHO I, astrocytoma WHO II, astrocytoma WHO III, blastoma, chordoma, craniopharyngioma, ependymoma, Ewing's tumor, germinoma, glioma, glioblastoma, hemangioblastoma, hemangioperycatioma, Hodgkins lymphoma, medulloblastoma, leukaemia, mesothelioma, neuroblastoma, n on-Hodgkins lymphoma, pinealoma, retinoblastoma, sarcoma (including angiosarcoma, chondrosarcoma, endothelial sarcoma, fibrosarcoma, leiomyosarcoma, liposarcoma, lymphangioandotheliosarcoma, lyphangiosarcoma, medulloblastoma, melanoma, meningioma, myosarcoma, neurinoma, oligodendroglioma, osteogenic sarcoma, osteosarcoma), seminoma, subependymoma, Wilm's tumor, or is selected from the group of bile duct carcinoma, bladder carcinoma, brain tumor, breast carcinoma, bronchogenic carcinoma, carcinoma of the kidney, cervical carcinoma, choriocarcinoma, cystadenocarcinome, embryonal carcimoma, epithelial carcinoma, esophageal carcinoma, cervical carcinoma, colon carcinoma, colorectal carcinoma, endometrial carcinoma, gallbladder carcinoma, gastric carcinoma, head and neck carcinoma, liver carcinoma, lung carcinoma, medullary carcinoma, non-small cell bronchogenic/lung carcinoma, ovarian carcinoma, pancreas carcinoma, papillary carcinoma, papillary adenocarcinoma, prostate carcinoma, small intestine carcinoma, rectal carcinoma, renal cell carcinoma, skin carcinoma, small-cell bronchogenic/lung carcinoma, squamous cell carcinoma, sebaceous gland carcinoma, testicular carcinoma, uterine carcinoma. Pharmaceutical compositions of this invention beside the immunostimulator comprise at least one substance inhibiting cell proliferation and/or inducing cell death.

An "antineoplastic chemotherapeutic agent" as used herein is a substance inhibiting cell proliferation and/or inducing cell death and in a preferred embodiment further inhibits the formation of metastases not by stimulating the immune cells and/or the function of the immune system as described herein.

Antineoplastic substance of the present invention are selected from the group consisting of temozolomid, BCNU, CCNU, gemcitabine and vincristine.

BCNU is Bischloroethylnitrosourea, the chemical name is N,N'-bis(2-chlorethyl)-N-nitroso-urea, other names are BiCNU, carmustine.

CCNU is 1-(2-Chloroethy)-3-cyclohexyl-1-nitrosourea. Synonyms are N-(2-chloroethyl)-N'-cyclohexyl-N-nitroso-urea, Belustine, Cee NU, Chloroethylcyclohexymitrosourea, ICIG 1109, Lomustine, NSC 79037. One chemical name for temozolomide is 3,4-dihydro-3-methyl-4-oxoimidazo--5,1d'1,2,3,4-tetrazin-8-carboximide. Other names for temozolomide are Temodal, Temodar, methazolastone, CCRG81045, SCH52365, NSC362856, M&B39836.

In one embodiment the antagonist of the purine and pyrimidine bases is gemcitabine.

Since a common but tolerable side effect of antineoplastic agents is nausea and vomiting it is obvious to someone skilled in the art that these effects can be avelliated by administering an antiemetic in conjunction with the antineoplastic agent inducing nausea and/or vomiting. E.g. Ondansetron may be given p.o. in a dose of about 8 mg about 30 minutes before the nausea/vomiting inducing antineoplastic agent is administered. Of course other anti-emtics such as Hasaldol, Benadryl, and Ativan may also be used as needed.

The antineoplastic chemotherapeutic agent of this invention are commercialley available. For the synthesis of e.g. temozolomid see for example Stevens et al. (1984) or Wang et. al (1994).

Radiation is applied in dosages of about 1 Gy to about 100 Gy, more preferred from about 20 to about 80 Gy and most preferred, e.g. for the treatment of astrocytomas, glioblastomas and gliomas from about 40 to about 60 Gy.

The dosage in preferred embodiments is fractionated which means that, from about 0.1 to about 10 Gy or from about 1 Gy to about 5 Gy or from about 1 Gy to about 2 Gy are applied in one session which is repeated several times during about 1 to about 20 weeks, about 2 to about 10 weeks or 4 to about 8 weeks. The antagonist and/or the substance inhibiting cell proliferation and/or inducing cell death of this invention can be administered before, after or together with the radiation. One cycle of radiation therapy as well as several cycles of radiation are possible, dependent of the reduction of tumor size.

The radiation usually is performed with 60Co. Radiation with neutrons, protons, negative pimesones or neutrone capture are applicable as well.

It is clear to someone skilled in the art that the dosage is further dependant on the size of the tumor, the build of the patient and the kind of radiation applied. In special embodiments the dosage is about 2 to about 100 fold higher or lower as described above also dependant from the number of fractions the dosage is applied with.

In one embodiment the combination of at least one immunostimulator and at least one antineoplastic agent according to the present invention is useful in the treatment of unwanted neoplasms such as but not limited solid tumors; blood born tumors such as leukemias, acute or chronic myelotic or lymphoblastic leukemia; tumor metastasis; benign tumors, for example hemangiomas, acoustic neuromas, neurofibromas, trachomas, and pyogenic granulomas; premalignant tumors; astrocytoma, blastoma, chordoma, craniopharyngioma, ependymoma, Ewing's tumor, germinoma, glioma, glioblastoma, hemangioblastoma, hemangioperycatioma, Hodgkins lymphoma, medulloblastoma, leukaemia, mesothelioma, neuroblastoma, non-Hodgkins lymphoma, pinealoma, retinoblastoma, sarcoma (including angiosarcoma, chondrosarcoma, endothelial sarcoma, fibrosarcoma, leiomyosarcoma, liposarcoma, lymphangioandotheliosarcoma, lyphangiosarcoma, medulloblastoma, melanoma, meningioma, myosarcoma, neurinoma, oligodendroglioma, osteogenic sarcoma, osteosarcoma), seminoma, subependymoma, Wilm's tumor, or is selected from the group of bile duct carcinoma, bladder carcinoma, brain tumor, breast carcinoma, bronchogenic carcinoma, carcinoma of the kidney, cervical carcinoma, choriocarcinoma, cystadenocarcinome, embryonal carcinoma, epithelial carcinoma, esophageal carcinoma, cervical carcinoma, colon carcinoma, colorectal carcinoma, endometrial carcinoma, gallbladder carcinoma, gastric carcinoma, head and neck carcinoma, liver carcinoma, lung carcinoma, medullary carcinoma, non-small cell bronchogenic/lung carcinoma, ovarian carcinoma, pancreas carcinoma, papillary carcinoma, papillary adenocarcinoma, prostate carcinoma, small intestine carcinoma, rectal carcinoma, renal cell carcinoma, skin carcinoma, small-cell bronchogenic/lung carcinoma, squamous cell carcinoma, sebaceous gland carcinoma, testicular carcinoma, uterine carcinoma.

In another embodiment the composition of at least one agonist and at least one antineoplastic agent according to the present invention may be used in combination with other procedures for the treatment of diseases. For example, a tumor may be treated conventionally with surgery and/or radiation and then the composition of an immunostimulator and antineoplastic chemotherapeutic agent according to this invention may be subsequently administered to the patient to extend the dormancy of micrometastases and to stabilize respectively reduce any residual unwanted neoplasm.

In a preferred embodiment a combination of at least one antineoplastic agent and at least one antagonist according to the present invention is administered to a site likely to harbor a metastatic lesion (that may or may not be clinically discernible at the time). A sustained release formulation implanted specifically at the site (or the tissue) where the metastatic lesion is likely to be would be suitable in these latter instances.

The embodiments of the combination of at least one stimulator of the immune cells and/or the immune system and at least one substance inhibiting cell proliferation, and/or inducing cell death according to the present invention is delivered in effective amounts. In general, the term "effective amount" of an antagonist and/or antineoplastic agent refers to the amount necessary or sufficient to realize a desired biologic effect. Specifically, the effective amount is that amount that reduces the rate or inhibits altogether formation of neoplasms. For instance, when the subject bears a tumor, an effective amount is that amount which decreases or eliminates the unwanted neoplasm. Additionally, an effective amount may be that amount which prevents an increase or causes a decrease in new unwanted neoplasms.

The effective amount varies depending upon whether the combination is used in single or multiple dosages. Dosages given in this writing are for adults. It is quite clear to someone skilled in the art that these dosages have to be adapted if the human being is a child, a person stressed by a further illness or other circumstances. The effective dosage is dependent also on the method and means of delivery, which can be localized or systemic. For example, in some applications, as in the treatment of skin carcinoma or ophthalmic carcinoma the combination is preferably delivered in a topical or ophthalmic carrier.

In one embodiment subject doses of the compounds described herein typically range from about 0.1 µg to about 10 mg per administration, which depending on the application could be given hourly, daily, weekly, or monthly and any other amount of time therebetween. In yet another embodiment the doses range from about 10 µg to about 5 mg per administration or from about 100 µg to about 1 mg, with 1-10 administrations being spaced hours, days or weeks apart. In some embodiments, however, doses may be used in a range even 2 to 100 fold higher or lower than the typical doses described above.

In one embodiment of this invention the at least one immunostimulator of a pharmaceutical composition according to this invention is an antisense oligonucleotide of TGF-beta2 which is administered in a dose range from about 1 µg/kg/day to about 100 mg/kg/day or from about 10 µg/kg/day to about 10 mg/kg/day or from about 100 µg/kg/day to about 1 mg/kg/day.

In a further preferred embodiment of the pharmaceutical composition described herein the at least one immunostimulator, a TGF-beta2 antisense oligonucleotide, is administered with a catheter directly into the unwanted neoplasm. The concentrations of these antinsense oligonucleotides are from about 0.1 µM/L to about 1 M/L, more preferred from about 1 µM/L to about 500µM/L and even more preferred from about 10 to about 200 µM/L or from about 50 µM/L to about 150 µM/L in a sterile aqueous solution. In yet another preferred embodiment this solution is administered with a flow of about 0.1 µL/min to about 50 µL/min or about 2 µL/min to about 12 µL/min or about 3 µL/min to about 10 µL/min into the neoplasm.

In yet another embodiment the at least one antineoplastic chemotherapeutic agent is selected from the group of BCNU, and/or CCNU in combination with at least one immunostimulator of the present invention is administerd in dose range from about 1 mg/m² to about 1000 mg/m², more preferred in a dose of about 50 mg/m² to about 500 mg/m² and most preferred in a single dosis of about 150 mg/m² to 200 mg/m² intravenously every 6 weeks. It may be given as a single dose or divided into daily injections such as about 75 mg/m² to about100 mg/m² on two successive days.

In yet another embodiment in the treatment of neoplasms the antineoplastic chemotherapeutic agent is gemcitabine and is administered with at least one immunostimulator of the present invention in a dosage of about 10 mg/m² to about 10 g/m², more preferred from about 100 mg to about 5g/m² and most preferred from about 500 mg/m² to about 2000 mg/m².

In another embodiment the dosage of gemcitabine is administered within about 10 min to about 120 min, more preferred from about 15 min to about 60 min and most preferred from about 20 min to about 40 min. In yet another embodiment this single dose is administered repeatedly within about 4 to about 10 days, respectively about 5 to about 8 days and most preferred within about 7 days. About 1 to about 8, more preferred about 2 to about 6 most preferred about 3 to about 4 single doses are administered. After this a therapy free interval of about 2 to about 60 days, more preferred about 5 to about 30 days and most preferred from about 10 to about 20 days is applied. Several repetitions of these cycles are possible.

In yet another embodiment at least one antineoplastic chemotherapeutic agent is temozolomide and is administered with a total dose of about 500 to about 1200 mg/m², over a period from about 2 to about 28 consecutive days, more preferable over a period of from about 4 to about 7 consecutive days, and most preferably over a period of about 5 consecutive days. Thus if the total dose is to be about 1000 mg/m² administered over a period of about 5 days, the daily dose for this period is about 200 mg/m²/day. Temozolomide must be administered more than once per day. Preferably dosing regimes would be twice per day, three times per day or four times per day. After a period of about 28 to about 42 days, or about about 28 to about 35 days, or more preferably 28 days, from the first day of temozolomide administration, another administration cycle may be started.

In yet another embodiment the temozolomide may be administered for a much longer period at reduced dosage. For example, the temozolomide could be administered more than once daily for up to six weeks at a daily dosage of about 50 mg/m²/day to about 150 mg/m² preferably at about 75 mg/m²/day. More preferred these daily doses are split about evenly into two or more doses to be administered two ore more times per day.

In a further embodiment vincristin is administered at a dose of about 0.1 mg/m² to 10 mg/m² more preferred in a dose of about 0.5 mg/m² to about 5 mg/m² and more preferred at about 0.8 mg/m² to about 2 mg/m² about once a week whereas the neurotoxicity is the dosage limiting factor. Most commonly solution of vincristin sulfate from about 0.1 mg/mL to about 10 mg/mL are administered with single doses of about 0.1 mg/m² to about 50 mg/m² more preferred in a dose of about 0.5 mg/m² to about 10 mg/m² and even more preferred from about 1 mg/m² to about 5.0 mg/m²_{.}

In one embodiment a pharmaceutical composition for the treatment of glioma, glioblastoma and/or anaplastic astrocytoma comprises a combination of at least one TGF-beta2 antisense oligonucleotide hybridising with an area of the messenger RNA (m-RNA) and/or DNA encoding TGF-beta2 selected from the group consisting of SEQ ID NO: 28 to 30and at least one substance inhibiting cell proliferation and/or inducing cell death preferably selected from the group of temozolomide, , BCNU, CCNU, vincristine, and gemicitabine.

In a further preferred embodiment the at least one TGF-beta2 antisense oligonucleotide hybridising with an area of the messenger RNA (m-RNA) and/or DNA encoding TGF-beta2 selected from the group consisting of SEQ ID NO: 28 to 30 and temozolomide are the parts of a pharmaceutical composition. In this case the dosage of temozolomide for the treatment of unwanted neoplasms more preferred glioma, glioblastoma and/or anaplastic astrocytoma is from about 120 to about 180 mg/m², p.o. on day 1 to 5 of a cycle. In a more preferred embodiment the immunostimulator is administered from about 1 µg/kg/day to about 50 mg/kg/day. The cycle is repeated after about 3 to 5 weeks.

In a further preferred embodiment for the treatment of glioma radiation is further administered according to standard schedules as described above. In one embodiment the radiation is applied together with the administration of the combination as described above. In other embodiments the radiation is applied before or after the administration of the pharmaceutical compositions according to this invention.

In yet another embodiment for the treatment of neoplasms such as pancreatic neoplasms the at least one antineoplastic chemotherapeutic agent of a pharamaceutical composition according to this invention is gemcitabine. Gemcitabine is administered in dosages of about 800 mg/m² to about 1200 mg/m², more preferred about 1000 mg/m² iv. Within about 10 min to about 60 min, more preferred within about 12 min to about 20 min. This application is repeated for about 5 to about 10 days.

In another embodiment further to the adminstration of the pharmaceutical compositions of the present invention, radiotherapy is applied according to standard schedules as described above.

Non-small cell lung carcinoma (NSCLC)

In yet another embodiment for the treatment of NSCLC further radiation is applied according to schedules as described above.

In a more preferred embodiment of the above mentioned embodiments for the treatment of NSCLC the immunostimulator is a TGF-beta2 antisense oligonucleotide hybridising with an area of the messenger RNA (m-RNA) and/or DNA encoding TGF-beta2 selected from the group consisting of SEQ ID NO: 28 to 30which are administered according to schedules as described above.

In even more preferred embodiments of the above mentioned embodiments for the treatment of gastrointestinal neoplasms the antagonist is a TGF-beta2 antisense oligonucleotide hybridising with an area of the messenger RNA (m-RNA) and/or DNA encoding TGF-beta2 selected from the group consisting of SEQ ID NO: 28 to 30.

In a further preferred embodiment for the treatment of neoplasms such as gastrointestinal neoplasams radiation is additionally applied with a total dosage of about 40 Gy to about 60 Gy within one cycle. Even more preferred this dosage is fractioned into about 5 times about 1 Gy to about 2 Gy per week. The cycle is repeated after about 20 to about 40 days, after about 25 to about 35 days or after about 30 days.

Further preferred embodiments are pharmaceutical compositions according to this invention for the treatment of neoplasms such as melanomas, wherein the at least one substance inhibiting cell proliferation and/or inducing cell death is selected from the group of BCNU, CCNU, and temozolomide.

In even more preferred embodiments of the above mentioned embodiments for the treatment of melanoma the immunostimulator is a TGF-beta2 antisense oligonucleotide hybridising with an area of the messenger RNA (m-RNA) and/or DNA encoding TGF-beta2 selected from the group consisting of SEQ ID NO: 28 to 30.

In even more preferred embodiments of the above mentioned embodiments for the treatment of neoplasms such as prostate cancer the antagonist is a TGF-beta2 antisense oligonucleotide hybridising with an area of the messenger RNA (m-RNA) and/or DNA encoding TGF-beta2 selected from the group consisting of SEQ ID NO: 28 to 30.

The pharmaceutical composition of an antagonist according to the present invention is delivered solely or in mixtures with the at least one substance inhibiting cell proliferation and/or inducing cell death according to the present invention. A mixture may consist of several antineoplastic agents in addition to TGF beta2 antisense oligonucleotides hybridising with an area of the messenger RNA (m-RNA) and/or DNA encoding TGF-beta2 selected from the group consisting of SEQ ID NO: 28 to 30. These at least two substances herein is also referred to as compounds.

In one embodiment the at least two compounds are mixed and pure or in a pharmaceutically acceptable carrier. In yet another embodiment the at least two compounds of the pharmaceutical composition are separate and pure or are separate and in a pharmaceutically acceptable carrier. In one embodiment the at least two components are in the same pharmaceutically acceptable carrier, in yet another embodiment the at least two components are in different pharmaceutically acceptable carriers.

"Administering" the pharmaceutical compositions of the present invention may be accomplished by any means known to a person skilled in the art. Routes of administration include but are not limited to oral, intranasal, intratracheal, ocular, pulmonal, vaginal, rectal, parenteral (e.g. intramuscular, intradermal, intravenous, intratumoral or subcutaneous or direct injection), topical, transdermal.

In one embodiment of a pharmaceutical composition for the treatment of unwanted neoplasms, the combination of at least one substance inhibiting cell proliferation and/or inducing cell death and the at least one immunostimulator according to the present invention are delivered by means of a biodegradable, polymeric implant or implanted catheters. The term "pharmaceutical composition" implicates that the liquids or substances of this composition are pure and/or combined with pharmaceutically acceptable carriers.

The term "pharmaceutical acceptable carrier" means one or more compatible solid or liquid filler, diluents or encapsulating substances which are suitable for administration to a human or other manual. The term "carrier" denotes an organic or inorganic ingredient, natural or synthetic, with which the active ingredient is combined to facilitate the application. The components of the pharmaceutical compositions of the present invention also are capable of being commingled with the compounds of the present invention, and with each other, in a manner such that there is no interaction which would substantially impair the desired pharmaceutical efficacy.

Such carriers enable the compounds of the invention to be formulated as tablets, coated tablets, evervescent tablets, granules, lozenge, powders, pills, dragees, (micro)capsules, liquids, gels, syrups, slurries, suspensions, emulsions and the like, for oral ingestion by a subject to be treated.

The pharmaceutical compositions according to the present invention may also include granules, powders, tablets, coated tablets, (micro)capsules, suppositories, syrups, emulsions, suspensions, creams, drops, coated onto microscopic gold particles or preparations with protracted release of active compounds, in whose preparation excipients and additives and/or auxiliaries such as disintegrants, binders, coating agents, swelling agents, lubricants, flavorings, sweeteners or solubilizers are customarily used as described above.

For a brief review of present methods for drug delivery, see Langer (1990).

For oral administration, the compounds (i.e., at least one immunostimulator and at least one substance inhibiting cell proliferation and/or inducing cell death according to the present invention) are delivered alone without any pharmaceutical carriers or formulated readily by combining the compound(s) with pharmaceutical acceptable carriers.

In one embodiment pharmaceutical preparations for oral use are obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP).

In yet another embodiment disintegrating agents are added, such as the cross-linked polyvinyl pyrrolidone, agar, or alginic acid or a salt thereof such as sodium alginate. Optionally the oral formulations may also be formulated in saline or buffers for neutralizing internal acid conditions.

In yet another embodiment dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures.

In yet another embodiment dyestuffs or pigments are added to the tablets or dragee coatings for identification or to characterize different combinations of active compound doses.

In another embodiment pharmaceutical preparations which can be used orally "vegicaps" include push-fit capsules made of gelatin, as well as soft, sealed capsules made of gelatin and a plasticizer, such as glycerol or sorbitol.

In one embodiment the push-fit capsules containes the active ingredient in a mixture with filler such as lactose, binders such as starches, and/or lubricants such as talc or magnesium stearate and, optionally, stabilizers. In another embodiment of the soft capsules, the active compounds are dissolved or suspended in suitable liquids, such as fatty oils, liquid paraffin, or liquid polyethylene glycols. In addition, stabilizers may be added.

In yet another embodiment microspheres formulated for oral administration are used, wellknown to someone skilled in the art.

The formulations for oral administration are in dosages suitable for such administration.

In yet another embodiment for buccal administration, the compositions may take the form of tablets or lozenges formulated in conventional manner.

In yet another embodiment for the administration by inhalation, the compounds for use according to the present invention may be conveniently delivered in the form of an aerosol spray, from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Suitable pharmaceutical carriers are, for example, aqueous or saline solutions for inhalation, microencapsulated, encochleated, contained in liposomes, nebulized, aerosols.

In yet another embodiment the pharmaceutical acceptable carriers of the compounds for parenteral, intrathecal, intraventricular or intratumoral administration include sterile aqueous solutions which may also contain buffers, diluents and other suitable additives such as, but not limited to, penetration enhancers, carrier compounds and other pharmaceutical acceptable carriers or excipients.

In yet another embodiment for the systemic delivery of the compounds they are in pharmaceutical carriers for parenteral administration by injection (e.g., by bolus injection or continuous infusion). Formulations for injection may be presented in unit dosage form, e.g., in ampoules or in multi-dose containers, with an added preservative. The pharmaceutical compositions take such forms as suspensions, solutions or emulsions in oily or aqueous vehicles, and contain formulatory agents such as suspending, stabilizing and/or dispersing agents. In one embodiment pharmaceutical carriers for parenteral administration include aqueous solutions of the active compounds in water-soluble form.

In yet another embodiment a suspension of the compounds is prepared as appropriate oily injection suspension. Suitable lipophilic solvents or vehicles include fatty oils such as sesame oil, or synthetic fatty acid esters, such as ethyl oleate or triglycerides, or liposomes. Aqueous injection suspensions comprise substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In yet another embodiment the active compounds may be in powder form for constitution with a suitable vehicle, e.g., sterile pyrogen-free water, before use or dried onto a sharp object to be scratched into the skin. In yet another embodiment the compounds are formulated in rectal or vaginal compositions such as suppositories or retention enemas or tablets, e.g., containing conventional suppository bases such as cocoa butter or other glycerides.

In yet another embodiment the compounds are formulated as a depot preparation. In one embodiment such long acting formulations are formulated with suitable polymeric or hydrophobic materials (for example as an emulsion in an acceptable oil) or ion exchange resins, or as sparingly soluble derivatives, for example as a sparingly soluble salt.

In other embodiments delivery systems include time-release, delayed release or sustained release delivery systems. Such systems can avoid repeated administrations of the compounds, increasing convenience to the subject and the physician. Many types of release delivery systems are available and known to those of ordinary skill in the art.

In one embodiment the delivery systems include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109 .

In another embodiment the delivery systems include non-polymer systems that are e.g. lipids including sterols such as cholesterol, cholesterol esters and fatty acids or neutral fats such as mono-, di- and triglycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Pat. No. 4,452,775, 4,675,189, and 5,736,152, and (b) diffusional systems in which an active component permeates at a controlled rate from a polymer such as described in U.S. Pat. No. 3,854,480, 5,133,974 and 5,407,686. In addition, pump-based hardware delivery systems can be used, some of which are adapted for implantation.

In still other embodiments, the antagonist and antineoplastic agent are formulated with GELFOAM^{®}, a commercial product consisting of modified collagen fibers that degrade slowly.

In one embodiment the pharmaceutical compositions also comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols. In one embodiment the immunostimulators and substances inhibiting cell proliferation and/or inducing cell death are administered neat or in the form of a pharmaceutical acceptable salt. The salts have to be pharmaceutical acceptable, but non-pharmaceutical acceptable salts may conveniently be used to prepare pharmaceutical acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

In one embodiment suitable buffering agents include but are not limited to: acetic acid and a salt (1-2% w/v); citric acid and a salt (1-3% w/v); boric acid and a salt (0.5-2.5% w/v); and phosphoric acid and a salt (0.8-2% w/v).

Suitable preservatives include benzalkonium chloride (0.003-0.03% w/v); chlorobutanol (0.3-0.9% w/v); parabens (0.01-0.25% w/v) and thimerosal (0.004-0.02% w/v).

In one embodiment the pharmaceutically acceptable carrier for topical administration for the at least two compounds of a pharmaceutical composition according to this invention include transdermal patches, ointments, lotions, creams, gels, drops, suppositories, sprays, liquids and powders. Conventional pharmaceutical carriers, aqueous, powder or oily bases, thickeners and the like may be necessary or desirable. In yet another embodiment coated condoms, gloves and the like are useful.

In yet another embodiment the pharmaceutical compositions also include penetration enhancers in order to enhance the alimentary delivery. Penetration enhancers may be classified as belonging to one of five broad categories, i.e., fatty acids, bile salts, chelating agents, surfactants and non-surfactants (Lee et al. 1991, Muranishi 1990). One or more penetration enhancers from one or more of these broad categories may be included.

Various fatty acids and their derivatives which act as penetration enhancers include, for example, oleic acid, lauric acid, capric acid, myristic acid, palmitic acid, stearic acid, linoleic acid, linolenic acid, dicaprate, tricaprate, recinleate, monoolein (a.k.a. 1-monooleoyl-rac-glycerol), dilaurin, caprylic acid, arichidonic acid, glyceryl 1-monocaprate, 1-dodecylazacycloheptan-2-one, acylcarnitines, acylcholines, mono- and diglycerides and physiologically acceptable salts thereof (i.e., oleate, laurate, caprate, myristate, palmitate, stearate, linoleate, etc.) (Lee et al. 1991, Muranishi 1990, El-Hariri et aL 1992). Examples of some presently preferred fatty acids are sodium caprate and sodium laurate, used singly or in combination at concentrations of 0.5 to 5%.

The physiological roles of bile include the facilitation of dispersion and absorption of lipids and fat-soluble vitamins (Brunton 1996). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), generally used at concentrations of 0.5 to 2%.

Complex formulations comprising one or more penetration enhancers may be used. For example, bile salts may be used in combination with fatty acids to make complex formulations. Preferred combinations include CDCA combined with sodium caprate or sodium laurate (generally 0.5 to 5%).

In one embodiment additionally chelating agents are used that include, but are not limited to, disodium ethylenediaminetetraacetate (EDTA), citric acid, salicylates (e.g., sodium salicylate, 5-methoxysalicylate and homovanillate), N-acyl derivatives of collagen, laureth-9 and N-amino acyl derivatives of beta-diketones (enamines) (Lee et al. 1991; Muranishi 1990; Buur et al. 1990). Chelating agents have the added advantage of also serving as DNase inhibitors.

In yet another embodiment additionally surfactants are used. Sufactants include, for example, sodium lauryl sulfate, polyoxyethylene-9-lauryl ether and polyoxyethylene-20-cetyl ether (Lee et al. 1991); and perfluorochemical emulsions, such as FC-43 (Takahashi et al. 1988).

Non-surfactants include, for example, unsaturated cyclic ureas, 1-alkyl- and 1-alkenylazacyclo-alkanone derivatives (Lee et al. 1991); and non-steroidal anti-inflammatory agents such as diclofenac sodium, indomethacin and phenylbutazone (Yamashita et al. 1987).

In one embodiment the pharmaceutical compositions of the present invention additionally contain other adjunct components conventionally found in pharmaceutical compositions, at their art-established usage levels. Thus, for example, the compositions may contain additional compatible pharmaceutically active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents and stabilizers. However, such materials, when added, should not unduly interfere with the biological activities of the components of the compositions of the invention.

### Examples

Clinical studies represented herein were primarily designed as safety studies and were approved by the local ethic committees and performed in accordance with the current international declaration of Helsinki for human experimentation and GCP and had to sign a written informed consent prior to recruitment.

The treatment with the antineoplastic agent followed routine schedules if nothing else is mentioned. Before the treatment with an TGF-beta antagonist, the antisense oligonucleotide of TGF-beta, with Seq. No. 30 the patients were selected according to the following criteria.

Patients had high grade gliom, either anaplastic astrocytome, WHO grade III, or glioblastoma, WHO grade IV, refractory to or recurrent after standard therapy (surgery, radiotherapy and different therapies with antineoplastic substances). Patients had not received antineoplastic agents within 10 days prior to the administration of the antagonist. Patients were between 18 and 75 years old. Karnofsky performance status (KPS) was at least 70%. Patients with clinically significant acute infections, cardiovascular abnormalities or poorly controlled seizures and pregnant and lactating females were excluded.

Surgical planning was based on computer tomography or magnetic resonance images. The perforated part of the catheter was placed in the solid, enhancing area of the tumor. Ventricles, cysts, resection cavities from prior surgical interventions, blood vessels and eloquent brain areas had to be avoided by the catheter trajectory. The catheter was introduced through a standard burr hole into the center of the largest tumor lesion. The distal end of the catheter was passed several centimetres under the galea through the skin and filled with saline. TGF-beta 2 specific antisense oligonucleotide with Seq. Id. No. 30 was administered intratumorally as a continuous high-flow microperfusion using an external pump system, Graseby 3200 (Smith Medical, London, GBM). The application system was removed after the end of the infusion. For safety assessment patients were followed up for 28 days. Post-study MRI and survival data until death were collected by the investigators.

### 1. Example

47 years old male who was diagnosed with a histologically grade III anaplastic astrocytoma received a combination therapy of several antineoplastic agents and TGF-beta 2 specific antisense oligonucleotide with Seq. Id. No. 30. The antineoplastic agents administered were ACNU together with tenoposide, temozolomide, and PEG-ylated liposomal doxorubicin (Caelyx^{®}). ACNU was administered partly parallel with tenoposide with 90 mg/m² ACNU on the first day of each cycle and 60 mg/m² of tenoposide on days 1-3 of each cycle. Each cycle comprised 42 days, 4 of these cycles were realized. About 2 years later the patient was treated with 3 cycles of temozolomide. Each cycle of 28 days started with the administration of temozolomide 75 mg/m² from day 1-5. About 8 months after this treatment PEG-ylated liposomal doxorubicin (Caelyx^{®} was administered in 5 cycles of 42 days, with 20 mg/m² on day 4 and day 14 of the cycle, followed by a week with 160 mg tamoxifen administration in the morning and in the evening.

The therapy with these antineoplastic agents according to standard schedules was finally without success and therefore the patient was included into the study with TGF-beta 2 specific antisense oligonucleotide with Seq. Id. No. 30 showing surprising success. At the start point of this study the magnetic resonance imaging showed three tumors in the left frontal lobe and an additional tumor in the right hemisphere and an overall edema. After the chemotherapy with the above mentioned antineoplastic agents one cycle of TGF-beta 2 specific antisense oligonucleotide with Seq. Id. No. 30 (10 µM in sterile pyrogen free isotonic 0.9% NaCl solution, 4 µL/min, total of 1.42 mg in 4 days) was applied intratumorally by an implanted catheter into the largest nodule. Six months after start of TGF-beta 2 specific antisense oligonucleotide with Seq. Id. No. 30 a clear reduction of the largest tumor lesion could be diagnosed. Although not individually targeted by the catheter, the three smaller tumors also disappeared completely. Additionally, the edema had decreased. 17 months after the first application of TGF-beta 2 specific antisense oligonucleotide with Seq. Id. No. 30 the largest tumor was hardly measurable anymore. Four months later a complete response was assessed by 3 independent specialists. These findings were accompanied by clinical improvement. The patient died due to a myocardial infarction without signs of tumor recurrence and had experienced an overall survival of 195 weeks after first recurrence and 208 weeks after diagnosis of anaplastic astrocytoma.

### 2. Example

Male patient 45 years old was diagnosed with anaplastic astrocytoma (WHO grade III). The diagnosis was followed by surgery and radiotherapy. 3 times 200 mg/m² Temozolomide was administered according to a standard schedule during two months. Again this therapy was without success. Therefore the patient was included into the study with TGF-beta 2 specific antisense oligonucleotide with Seq. Id. No. 30. Two cycles of this oligonucleotide with a concentration of 80 µM and a flow of 8 µl/min was administered for each 4 days through a catheter placed inside the tumor tissue. Afterwards the patient received ten additional cycles within four months. Following the last cycle of the oligonucleotide, approximately 10 months after the first oligonucleotide treatment, the patient received seven cycles of liposomal doxorubicin (Caelyx^{®}).

A planned 8th cycle could not be started, as the chemotherapy had to be discontinued due to cardiotoxicity (ventricular tachycardia). From that time the patient did not receive any anti-tumor therapy or corticosteroids. The lastmagnetic resonance image was taken 19.4 months after the start of oligonucleotide treatment. These images were evaluated and showed in the central reading a significant partial response (83% tumor reduction) and an overall survival time which was not so far reported in literature.

This is a further prove that surprisingly the coadministration of radiotherapy, antineoplastic agents and antagonists clearly show synergistic effects in the treatment of tumors, such as e.g. glioma, glioblastoma and/or astrocytoma.

### 3. Example

Comparison of survival data of patients treated with antineoplastic agents in combination with antagonists of factors negatively influencing the immune system (here: an antisense oligonucleotide of TGF-beta with the sequence Id. No. 30) to literature data for treatment with antineoplastic alone. Survival time is given from start of first chemotherapy after tumor recurrence. Median overall survival time of all patients treated with antineoplastic agents and TGF-beta 2 specific antisense oligonucleotide with Seq. Id. No. 30 (anaplastic astrocytom: 8 patients, glioblastoma, 23 patients) are compared to the most current literature data (Theodosopoulos, P.V. et al. 2001). Table 1: Demographic data and patients' characteristics

**Table 1: Demographic data and patients' characteristics**

| Patient | Histology¹ | Age (years) | Sex² | KPS³ at baseline | Tumor size⁴ volume (cm³) | Previous therapy⁶ (after 1st recurrence) | Seq. Id. No. 30 (study group)⁷ (cyle s) | | Steroid⁸ |
|---|---|---|---|---|---|---|---|---|---|
| 01 | AA | 41 | M | 70 | 204.0 | TMZ | 1/1; 2/1 | 2x | 40 mg MP |
| 02 | AA | 46 | M | 90 | 216.0 | Surg+TMZ, CaeTam | 1/1 | 1x | - |
| 03 | GBM | 61 | M | 70 | 73.5 | CaeTam+Surg | 1/1 | 1x | 8mgMP |
| 04 | AA | 46 | M | 80 | 11.5⁵ | Surg+TMZ | 1/2 | 1x | 12 mg DEX |
| 06 | GBM | 51 | F | 70 | 76.8 | Surg+TMZ | 1/2 | 1x | - |
| 07 | GBM | 53 | M | 70 | 54.9 | Surg+TMZ, Surg+lxotene | 1/2 | 1x | 24 mg DEX |
| 08 | GBM | 56 | M | 70 | 101.0 | TMZ | 1/2 | 1x | 2 mg DEX |
| 09 | GBM | 63 | F | 70 | 67.7^{s} | - | 1/3 | 1x | 3 mg DEX |
| 10 | GBM | 30 | M | 90 | 160.7 | Surg+TMZ, CaeTam | 1/3 | 1x | 12-8 mg DEX |
| 11 | GBM | 43 | M | 70 | 16.7⁵ | TMZ | 1/3 | 1x | 2 mg DEX |
| 12 | GBM | 58 | M | 70 | n/e | Surg+TMZ | 1/3 | 1x | 6 mg DEX |
| 13 | GBM | 58 | F | 90 | 47.1⁵ | - | 1/4 | 1x | - |
| 14 | AA | 54 | M | 80 | 7.2 | Surg+TMZ | 1/4 | 1x- | - |
| 15 | GBM | 42 | M | 70 | 33.6 | Surg+TMZ, 2x Surg | 1/4 | 1x- | - |
| 16 | GBM | 45 | M | 90 | 27.0 | - | 1/4 | 1x | - |
| 17 | AA | 44 | M | 100 | 6.2⁵ | Surg | 1/5; 2/2 | 2x - | - |
| 18 | GBM | 46 | M | 70 | n/e | TMZ + Surg | 1/5 | 1x- | - |
| 19 | GBM | 41 | F | 70 | 58.8 | Surg+TMZ | 1/5 | 1x- | - |

| Patient | Histology¹ | Age (years) | Sex² | KPS³ at baseline | Tumor size⁴ volume (cm³) | Previous therapy⁶ (after 1st recurrence) | Seq. Id. No. 30 (study group)⁷ (cyle s) | Steroid⁸ | |
|---|---|---|---|---|---|---|---|---|---|
| Median | | 46 | | 70 | 56.85 | | | | |
| 1 AA, anaplastic astrocytoma; GBM, glioblastoma multiforme | | | | | | | | | |
| 2 F, female; M, male | | | | | | | | | |
| 3 kips, Karnofsky performance status | | | | | | | | | |
| 4 Tumor size at baseline; n/e = not evaluable | | | | | | | | | |
| 5 Multiple lesions, the total volume of all lesions is presented | | | | | | | | | |
| 6 TMZ, temozolomide; CaeTam, Cacly® + Tamoxifen; Surg, surgery | | | | | | | | | |
| 7 For details see Table 1 | | | | | | | | | |
| 8 DEX, dexamethasone; MP, methylprednisolone | | | | | | | | | |

Summary of patients' characteristics from the study. Patients 01, 13 and 16 received each two cycles of pegylated liposomal doxorubicin (Caelyx^{®}), patient 14 two cycles of PCV (procarbazine, lomustine (CCNU), vincristine) after TGF-beta 2 specific antisense oligonucleotide with Seq. Id. No. 30 treatment, all other patients had no anti-tumor therapy after oligonucleotide treatment Patient 17 received 10 additional oligonucleotide cycles. After the last cycle of the oligonucleotide the patient received 7 cycles of pegylated liposomal doxorubicin.

Reduction of tumor volumes of patients 04 and 17 was more than 80%. Tumor volume was assessed by measurement of the largest cross-sectional diameter of the enhancing lesion in the first layer and the largest cross-sectional diameter perpendicular to the first in the same plane and layer. For the third dimension, the largest cross-sectional diameter of all further planes perpendicular to the first one was determined.

Compared to literature data for the treatment with antineoplastic agents alone the survival data show clearly enhanced survival of patients treated with one or more antineoplastic agents (e.g. temozolomide and/or procarbazine) before the administration of TGF-beta 2 specific antisense oligonucleotide with Seq. Id. No. 30.

The data are calculated after start of chemotherapy. According to this approach the median overall survival in our study was 147 weeks for AA and 42.4 weeks for GBM. The data reveal longer median overall survival times if applying the oligonucleotide following chemotherapy (mainly temozolomide) than the comparable published data for temozolomide alone, for which the most recent and accurate survival data are available: about 147 weeks versus 42 (Theodosopoulos, P.V. et al. 2001) weeks for anaplastic astrocytoma, and 45 weeks versus about 32 weeks (Theodosopoulos, P.V. et al. 2001; Yung, W.K. et al. 2000; Yung, W.K. 2000; Brandes, A.A. et al. 2001) for GBM, respectively.

These results surprisingly show that there is a clear survival advantage of patients treated with a combination of the antagonist, TGF-beta 2 specific antisense oligonucleotide with Seq. Id. No. 30 and at least one further antineoplastic agent (e.g. temozolomide) in patients suffering from neoplasm, e.g. AA (mean overall survival of 146.6 weeks versus 90 weeks for all anaplastic astrocytoma patients).

### Example 4

Temozolomide may be administered orally in capsule form wherein it is admixed with conventional pharmaceutical carriers. An example for a temozolomide capsule formulation is:

| **Ingredient** | **mg**/**capsule** | | | |
|---|---|---|---|---|
| Temozolomide | 5 | 20 | 100 | 250 |
| Anhydrous Lactose NF | 132.8 | 182.2 | 175.7 | 154.3 |
| Sodium Starch Glycolate NF | 7.5 | 11.0 | 15.0 | 22.5 |
| Colloidal Silicon Diozide NF | 0.2 | 0.2 | 0.3 | 0.7 |
| Tartaric Acid NF | 1.5 | 2.2 | 3.0 | 9.0 |
| Steric Acid NF | 3.0 | 4.4 | 6.0 | 13.5 |
| Capsule Size^{*} | 3 | 2 | 1 | 0 |
| *) white opaque, preservative free, two piece hard gelatin capsules | | | | |

The TGF-beta2 antisense oligonucleotide identified by the Seq. No. 30 is solved under sterile conditions in a sterile, pyrogene-free 0.9% NaCl solution and is ready for administration into a catheter surgically implanted with its perforated end placed in the tumor. The catheter is connected with a commercially available port system into which the AP12009 solution is administered.

Example 5 (wherein only antisense m-RNA of the human transforming growth factor TGF-beta2 belongs to the present invention)

Antisense m-RNA for the human transforming growth factor TGF-beta 1:

Antisense m-RNA of the human transforming growth factor TGF-beta2:

Antisense of m-RNA of the human TGF-beta 3

Antisense of m-RNA of human Interleukin 10

Antisense m-RNA of human Prostaglandin E2 Synthase tttttttttttttttttttttttttttttttttttttttttttttttttttCCATGAGATGCCTGCCATGACAGGCGCCACAAACCTTTCCT

Antisense m-RNA of human VEGF

### Example 6 (describes technical background and does not belong to the present invention)

Small molecules inhibiting TGF-beta
SB-431542 TBRI kinase inhibitor from GlaxoSmithKline (Callahan et al. 2002, Laping et al. 2002, Inman et al. 2002)
NPC30345 TBRI kinase inhibitor from Scios, Inc. (Dumont & Arteaga 2003)
SD-093 TBR-I kinase inhibitor (Subramanian, G. et al. 2003)
LY364947 TBRI kinase inhibitor from Lilly Inc. (Sawyer et al. 2003).
Decorin a small chondroitin-dcrmatan sulfate proteoglycan that binds various forms of active TGF-B (Border et al. 1992).
Proteins inhibiting TGF-beta
Endoglin a TGF-B binding 95 kDa glycoprotein (Gougos et al. 1992).

Antibodies binding TGF-beta
CAT-192 humanized TGF-beta1 mAB from Genzyme/CAT (Benigni et al. 2003).
CAT-152 humanized TGF-beta2 mAB from Genzyme/CAT (Siriwardena et al. 2002).
1D11 TGF-beta1, 2, 3 mAB from Genzyme/CAT (Ananth et al. 1999).
2G7 TGF-beta1, 2, 3 monoclonal IgG2 from Genentech., (Arteaga et al. 1993).
Antibodies against TGF-beta1/2/3 from R&D
see e.g. catalog 614 R&D systems, McKinley Place NE, Minneapolis, MN USA 55413
rabbit anti-TGF-beta2 LAP: (Schlotzer-Schrehardt, U. et al. 2001).

Soluble Receptors
sTBRII:Fc (RII/Fc hu IgGI fusion protein) from Biogen (Muraoka et al. 2002, Rowland-Goldsmith et al. 2001)
sT8RII:Fc (Yang, Y.A. et al. 2002)
Betaglycan (recombinant soluble TBRIII) (Bandyopadhyay et al. 2002)

### Example 7 (describes technical background and does not belong to the present invention)

Cell-mediated cytotoxicity assay:
Cell-mediated cytotoxicity was quantified by the CARE-LASS assay (Lichtenfels et al. 1994) using the NSCLC (non small cell lung carcinoma cell) line NCI-H661, the glioma cell line A-172, and the pancreatic cancer cell line Hup-T3 as target cells. NCI-H661 cells were pretreated with 5 µM TGF (transforming growth factor)-beta 1 specific antisense phosphorothioate oligodeoxynucleotide (PTO) Seq. Id. No. 14. A-172 and Hup-T3 cells were pretreated with 5 µM TGF-beta 2 specific antisense phosphorothioate oligodeoxynucleotide Seq. Id. No. 30 in medium at 5% CO2 and 37°C for 3 days according to the cell line suppliers' instructions. Additionally, for Hup-T3 cells 3 µg/ml Lipofectin^{®} were used to enhance cellular uptake of the PTO. Untreated cells and cells treated with 3 µg/ml Lipofectin^{®} were used as controls. Lymphokine activated killer cells (LAK cells) were used as effector cells. LAK cells were generated by a 3 day pretreatment of 5x106 PBMC from healthy volunteers with 10 ng/ml rh IL-2 (recombinant human interleukin 2) in 4 ml RPMI 1640 medium supplemented with 10% fetal calf serum at 5% CO2 and 37°C. One part of the effector cells was incubated additionally with rh TGF-b to mimic the presence of tumor cells (2000 pg/ml rh TGF-b1 for NCI-H661, 500 pg/ml rh TGF-b2 for A-172 and Hup-T3). The other part of cells was incubated without additional treatment.

Effector cells were incubated with the target cells with and without cytostatic drugs for 4 h. The supraaditive effect respectively the inhibition was calculated by subtracting the specific cell lysis of the control from the specific cell lysis of the chemotherapeutic agent. Taking into account the sign this difference is summed up with the specific lysis of the inhibitor of TGF-beta, as depicted in the figures. In case the specific lysis of the combination of a chemotherapeutic agent with the TGF-beta inhibitor was higher than this sum, this was interpreted as supraaditive effect. In case the specific lysis of this combination was smaller than the sum this was interpreted as inhibition.

### Example 8 (describes technical background and does not belong to the present invention)

Presented are the amino acid sequences of TGF-beta1, TGF-beta2 and TGF-beta 3 with the international one letter abbrevation for amino acids.
RXXR: cleavage site of the mature (active) part (XX may be anything)
ASPC: the C of this motif is the C for the intermolecular cystine bridge that links the two monomers into a functional dimer
C C C: intramolecular cystein bridges (cystein knot motif)
mature protein of TGF-beta 1, 2 and 3 contains 112 amino acids from the end of this listing TGF-beta 1 preferred amino acid sequences of TGF-beta1:
1) ALDTNYCFSSTEKNCCVRQL
2) YIDFRKDLGWKWIHEPKGYH
3) ANFCLGPCPYIWSLDTQYSK
4) VLALYNQHNPGASAAPCCVP
5) QALEPLPIVYYVGRKPKVEQ
6) LSNMIVRSCKCS
7) TEKNCCVRQLYIDFRKDLGW
8) KWIHEPKGYHANFCLGPCPY
9) WSLDTQYSKVLALYNQHNP
10) GASAAPCCVPQALEPLPIVY
11) YVGRKPKVEQLSNMIVRSCKCS
12) QYSKVLAYNQHNPGASAAPCCVPQALEPLPIVYYVGRKP
13) QYSKVLALYNQHNPGASAAPCCVPQALEPLPIVYYVGRKP I
   QYSKVLALYNQHNPGASAAPCCVPQALEPLPIVYYVGRKP
   (dimer of the TGF-beta1 amino acid sequence No.12 coupled by an s-s bridge at the Cytosins of the AAPC motif)
14)ALDTNYCFSSTEKNCCVRQLYIDFRKDLGWKWIHEPKGYHANFCLGPCPYIWSLDTQYSKVLAL YNQHNPGASAAPCCVPQALEPLPIVYYVGRKPKVEQLSNMIVRSCKCS
15) ALDTNYCFSSTEKNCCVRQLYIDFRKDLGW
16) KWIHEPKGYHANFCLGPCPYIWSLDTQYSK
17) VLALYNQHNPGASAAPCCVPQALEPLPIVY
18) YVGRKPKVEQLSNMIVRSCKCS
19) CVRQLYIDFRKDLGWKWIHEPKGYHANFCL
20) GPCPYIWSLDTQYSKVLALYNQHNPGASAA
21) PCCVPQALEPLPIVYYVGRKPKVEQLSNMI

TGF-beta 2

Preferred amino acid sequences of TGF-beta2
1) ALDAAYCFRNVQDNCCLRPL
2) YIDFKRDLGWKWIHEPKGYN
3) ANFCAGACPYLWSSDTQHSR
4) VLSLYNTINPEASASPCCVS
5) QDLEPLTILYYIGKTPKIEQ
6) LSNMIVKSCKCS
7) VQDNCCLRPLYIDFKRDLGW
8) KWIHEPKGYNANFCAGACPY
9) LWSSDTQHSRVLSLYNTINP
10) EASASPCCVSQDLEPLTILY
11) YIGKTPKIEQLSNMIVKSCKCS
12) QHSRVLSLYNTINPEASASPCCVSQDLEPLTILYYIGKTPK
13) QHSRVLSLYNTINPEASASPCCVSQDLEPLTILYYIGKTPK I
   QHSRVLSLYNTINPEASASPCCVSQDLEPLTILYYIGKTPK
   (dimer of the TGF-beta2 amino acid sequence No.12 coupled by an s-s bridge at the Cytosins of the ASPC motif)
14) ALDAAYCFRNVQDNCCLRPLYIDFKRDLGWKWIHEPKGYNANFCAGACPYLWSSDTQHSRVLS LYNTINPEASASPCCVSQDLEPLTILYYIGKTPKIEQLSNMIVKSCKCS
15) ALDAAYCFRNVQDNCCLRPLYIDFKRDLGW
16) KWIIHEPKGYNANFCAGACPYLWSSDTQHSR
17) VLSLYNTINPEASASPCCVSQDLEPLTILY
18) YIGKTPKIEQLSNMIVKSCKCS
19) CLRPLYIDFKRDLGWKWIHEPKGYNANFCA
20) GACPYLWSSDTQHSRVLSLYNTINPEASAS
21) PCCVSQDLEPLTILYYIGKTPKIEQLSNMI

TGF-beta3 preferred amino acid sequences of TGF-beta3:
1) ALDTNYCFRNLEENCCVRPL
2) YIDFRQDLGWKWVHEPKGYY
3) ANFCSGPCPYLRSADTTHST
4) VLGLYNTLNPEASASPCCVP
5) QDLEPLTILYYVGRTPKVEQ
6) LSNMVVKSCKCS
7) NLEENCCVRPLYIDFRQDLG
8) WKWVHEPKGYYANFCSGPCP
9)YLRSADTTHSTVLGLYNTLN
10) PEASASPCCVPQDLEPLTIL
11) YYVGRTPKVEQLSNMVVKSCKCS
12) THSTVLGLYNTLNPEASASPCCVPQDLEPLTILYYVGRTPK
13) THSTVLGLYNTLNPEASASPCCVPQDLEPLTILYYVGRTPK I
   THSTVLGLYNTLNPEASASPCCVPQDLEPLTILYYVGRTPK
   (dimer of the TGF-beta3 amino acid sequence No.12 coupled by an s-s bridge at the cytosins of the ASPC motif)
14)ALDAAYCFRNVQDNCCLRPLYIDFKRDLGWKWRIEPKGYNANFCAGACPYLWSSDTQHSRVLS LYNTINPEASASPCCVSQDLEPLTILYYIGKTPKIEQLSNMIVKSCKCS
15) ALDAAYCFRNVQDNCCLRPLYIDFKRDLGW
16) KWIHEPKGYNANFCAGACPYLWSSDTQHSR
17) VLSLYNTINPEASASPCCVSQDLEPLTILY
18) YIGKTPKIEQLSNMIVKSCKCS
19) CLRPLYIDFKRDLGWKWIHEPKGYNANFCA
20) GACPYLWSSDTQHSRVLSLYNTINPEASAS
21) PCCVSQDLEPLTILYYIGKTPKIEQLSNMI

### References

### Patents and Patent Applications

EP Pat. No. 069 53 54 Schlingensiepen et al. Jan. 09, 2002
EP Pat. No. 100 86 49 Schlingensiepen et al. Mar. 26, 2003
EP Pat. No. 009 25 74 Tallis Dec. 27,2000
US Pat. No. 6,455,689 Schlingensiepen et al. Sep. 24, 2002
US Pat. No 5,539,082 Nielsen et al. July 23,1996
US Pat. No 5,714,331 Buchardt , deceased, et al. Feb. 03,1998
US Pat. No 5,719,262 Buchardt , deceased, et al. Feb. 17, 1998
US Pat. No 4,469,863 Ts'o et al. Sep. 04, 1984
US Pat. No 5,023,243 Tullis June 11, 1991
US Pat. No 5,075,109 Tice et al. Dec. 24,1991
US Pat. No 4,452,775 Kent June 05, 1984
US Pat. No 4,675,189 Kent et al. June 23, 1987
US Pat. No 5,736,152 Dunn April 07, 1998
US Pat. No 3,854,480 Zaffaroni Dec. 17, 1974
US Pat. No 5,133,974 Paradissis et al. July 28, 1992
US Pat. No 5,407,686 Patel, et al. April 18, 1995
WO 98/33 904 Schlingensiepen et al. published Aug. 06,1998
WO 99/63 975 Schlingensiepen et al. published Dec. 16, 1999
WO 01/68 146 Schlingensiepen et al. published Dec. 20,2001

### Other References

Alvino E. et al., J. Pharmacol. Exp. Ther. 291: 1292-1300, 1999
Ananth et al., Carcinoma Res. 59(9): 2210-6,1999
Arteaga et al., J Clin Invest. 92(6): 2569-76, 1993
Bandyopadhyay et al., Carcinoma Res. 62: 4690-4695, 2002
Beaucage, S. L., Caruthers, M. H., Tet. Let. 22:1859,1981
Benigni et al., J. Am. Soc. Nephrol. 2003 Jul; 14(7):1816-24, 2003
Bernego et al. 1984
Border et al., Nature 360:361-364,1992
Brandes, A.A. et al., Ann Oncol 12: 255-257, 2001
Brunton, Chapter 38 In: Goodman & Gilman's, The Pharmacological Basis of Therapeutics, 9th Ed .
Buur et al., J. Control Rel. 14: 43-51, 1990
Callahan, J.F. et al., J. Med. Chem. 45: 999-1001, 2002
Carrington L., Allamby D., McLeod D., Boulon M., Incest. Ophthalmol. Vis. Sci. 39: 566, 1998
Cooper, H.M. & Paterson, Y., Current protocols in Immunology 2.4.1-2.5.17,1995
Dumont, Arteaga. Carcinoma Cell, 3: 531-536, 2003
Einstein A. B. Jr., Fass L., Fefer A., Carcinoma Res. 35(3): 492-496, 1975
El-Hariri et al., J. Pharm. Pharmacol. 44: 651-654,1992
Froehler et al., Nucl. Acid. Res. 14: 5399-5407,1986
Gaffney et al., Tet. Let. 29:2619-2622, 1988
Garegg et al., Tet. Let. 27:4051-4054,1986
Gereis M., Burford-Mason A. P., Watkins S. M., Suppression of in vitro peripheral blood lymphocyte mitogenesis by cytotoxic drugs commonly used in the treatment of breast carcinoma, 1987
Giampietri A., Bonmassar E., Goldin A., J. Immunopharmacol 791(1), 61-86, 1978
Goodchild, J., Bioconjugate Chem. 1:165,1990
Gougos et al., Int Immunol. 4(1): 83-92, 1992
Hardman et al., eds., McGraw-Hill, New York, N.Y.: 934-935,1996
Hayashi, T. et al., Clin. Immunol. 104: 14-20, 2002
Inman et al., Mol. Pharmacol. 62: 65-74, 2002
Jager, E., Jager, D., Knuth, A., Int. J. Carcinoma 106: 817-820,2003
Jantscheff, P., Spagnoli, G., Zajac, P., Rochlitz, C.F., Carcinoma Immunol Immunother 51: 367-375, 2002 Langer, Science 249: 1527-1533,1990
Laping et al., Mol. Pharmacol. 62: 58-64, 2002
Lee et al., Critical Reviews in Therapeutic Drug Carrier Systems 8:2, 91-192, 1991
Lichtenfels et al.; Journal of Immunological Methods 172: 227-239,1994
Lieberman, D.M., Laske, D.W., Morrison, P.F., Bankiewicz, K.S., Oldfield, E. H. J Neurosurg 82: 1021-1029, 1995
Mittl p., Priestle J. P., Cox D.A., McMaster G., Cerletti N., Grütter G., The crystal structure of TGF-beta 3 and comparison to TGF-beta 2: Implications for receptor binding, Protein Science 5 1261-1271,1996
Morrison, P.F., Laske, D.W., Bobo, H., Oldfield, E.H. & Dedrick, R.L., Am. J. Physiol. 266: R 292-305, 1994
Muranishi, Critical Reviews in Therapeutic Drug Carrier Systems, 7:1,1-33, 1990
Muraoka et al., J. Clin. Immunol. Cl 109: 1551-1559, 2002
Nardelli B., Puccetti p., Romani L., Sava G., Bonmassar E., Fioretti M. C., Carcinoma Immunol.
Immunother. 17(3) 213-217, 1984
Nielsen et al., Science 254: 1497-1500,1991
Parkhurst, MR., DePan, C., Riley, J.P., Rosenberg, S.A., Shu, S., J. Immunol.170, 5317-5325, 2003 Phan, V. et al., Nat Med 9: 1215-1219, 2003
Preiß, Dornhoff, Hagmann, Schmieder, Empfehlungen zur Therapy, 11. Auflage, W. Zuckscherdt Verlag GmbH, München, Bern, Wien, New York, 2002
Rowland-Goldsmith et al., Clin. Carcinoma Res. 7: 2931-2940, 2001
Sambrook, et al., Molecular Cloning: A Laboratory Manual, Cold Spring Harbor Laboratory Press, New York, 1989
Sawyer et al., J. Med. Chem. 46(19): 3953-3956,2003
Schlotzer-Schrehardt, U., Zenkel, M., Kuchle, M., Sakai, L.Y., Naumann, G.O., Exp. Eye Res. 73, 765-780,2001
Schneider, T., Gerhards, R., Kirches, E. Firsching, R., J Neurooncol 53: 39-46,2001
Siriwardena et al., Ophtalmology 109: 427-431, 2002
Stauder, G. et al. Proc. Am. Soc. Clin. Oncol. 22: 109 (abstr 436), 2003
Stevens et al., J. Med. Chem. 27: 196-201,1984
Subramanian, G., Schwarz, R., Liu, D., Reiss, M., Roles in the pathogenesis of carcinoma and other diseases B 26, La Jolla, 2003
Takahashi et al., J. Pharm. Pharmacol., 40: 252-257, 1988
Theodosopoulos, P.V. et al., Proc Annu Meet Am Soc Clin Oncol, 20 Abstract 2059 (San Francisco, 2001) Timmermann J.M., Czerwinski D. K., Davis T. A., Hsu F.J., Benike C., Hao, Z.M., Taidi B.,
Fajapaksa R., Caspar C.B., Okada C. Y., van Beckhoven A., Liles T.M., Eengleman E. G., Levy R., Blood 99(5): 1517-1528,2002
Uhlmann, E., Peyman, A., Chem. Rev. 90: 544, 1990
de Visser, K. E., Kast, W. M., Leukemia 13:1188-99,1999
Wagner et al., Nature Biotechnology 14: 840-844, 1996
Wang et al., J. Chem. Commun. 1687-1688,1994
Wilkenson, KA., Martin, T. D., Reba S. M.,Aung H., Redline R. W., Boom W. H., Toossi Z., Fulton S. A., Infect. immune6508,2000
Wojtowicz-Praga, S., J. Immunother. 20,165-77,1997
Wojtowicz-Praga, S., Investigational New Drugs 21: 21-32, 2003
Yamashita et al., J. Pharm. Pharmacol. 39:621-626,1987
Yang, Y.A. et al. J. Clin. Invest. 109: 1607-1615, 2002
Yung, W.K. et al., Br. J. Carcinoma 83: 588-893, 2000
Yung, W.K., Semin Oncol 27:27-34, 2000

### Sequenzen:

| | Seq. No. | Id.Sequences | Length | No. int. | Bez. int. |
|---|---|---|---|---|---|
| TGF-beta 1 | 1 | CGATAGTCTRGCAG | 14 | 1 | |
| | 2 | GTCGATAGTCTTGC | 14 | 2 | |
| | 3 | CTTGGACAGGATCT | 14 | 3 | |
| | 4 | CCAGGAATTGTTGC | 14 | 4 | |
| | 5 | CCTCAATITCCCCT | 14 | 5 | |
| | 6 | GATGTCCACTTGCA | 14 | 6 | |
| | 7 | CTCCAAATGTAGGG | 14 | 7 | |
| | 8 | ACCTTGCTGTACTG | 14 | 8 | |
| | 9 | GTAGTACACGATGG | 14 | 9 | |
| | 10 | CACGTAGTACACGA | 14 | 10 | |
| | 11 | CATGTFGGACAOCT | 14 | 11 | |
| | 12 | GCACGATCATGTTG | 14 | 12 | |
| | 13 | TGTACTCTGCTTGAAC | 16 | 13 | |
| | 14 | CTGATGTGTTGAAGAACA | 18 | 14 | |
| | 15 | CTCTGATGTGTTGAAG | 16 | 15 | |
| | 16 | GGAAGTCAATGTACAG | 16 | 16 | |
| | 17 | CATGTCGATAGTCTTGCA | 18 | 17 | |
| | 18 | AGCTGAAGCAATAGTROO | 18 | 18 | |
| | 19 | GTCATAGATTTCGTTGTG | 18 | 19 | |
| | 20 | CTCCACTTTTAACTTGAG | 18 | 20 | |
| | 21 | TGCTGTATTTCTGGTACA | 18 | 21 | |
| TGF-beta | 222 | CACACAGTAGTGCA | 14 | 1 | |
| | 23 | GCACACAGTAGTGC | 14 | 2 | |
| | 24 | GCTTGCTCAGGATCTGC | 17 | 3 | |
| | 25 | TACTCTTCGTCGCT | 14 | 4 | |
| | 26 | CTTGGCGTAGTACT | 14 | 5 | |
| | 27 | GTAAACCTCCTTGG | 14 | 6 | |
| | 28 | GTCTATTTTGTAAACCTCC | 19 | 7 | |
| | 29 | GCATGTCTATTTTGTAAACC | 20 | 8 | |
| | 30 | CGGCATGTCTATTTTGTA | 18 | 9 | |
| | 31 | GGCATCAAGGTACC | 14 | 10 | |
| | 32 | CTGTAGAAAGTGGG | 14 | 11 | |
| | 33 | ACAATTCTGAAGTAGGGT | 18 | 12 | |
| | 34 | TCACCAAATTGGAAGCAT | 18 | 13 | |
| | 35 | GCTTTCACCAAATTGGAAGC | 20 | 14 | |
| | 36 | CTGGCTTTTGGGTT | 14 | 15 | |
| | 37 | TCTGATATAGCTCAATCC | 18 | 16 | |
| | 38 | TCCTAGTGGACTTTATAG | 18 | 17 | |
| | 39 | TTTTTCCTAGTGGACT | 16 | 18 | |
| | 40 | CAATTATCCTGCACATTTC | 19 | 19 | |
| | 41 | GCAATTATCCTGCACA | 16 | 20 | |
| | 42 | GCAGCAATTATCCTGC | 16 | 21 | |
| | 43 | TGGCATTGTACCCT | 14 | 22 | |
| | 44 | TGTGCTGAGTGTCT | 14 | 23 | |
| | 45 | CCTGCTGTGCTGAGTG | 16 | 24 | |
| | 46 | CTTGGGTGTTTTGC | 14 | 25 | |
| | 47 | TTTAGCTGCATTTGCAAG | 18 | 26 | |
| | 48 | GCCACTTTTCCAAG | 14 | 27 | |
| TGF-beta | 349 | TCGAGCTTCCCCCA | 14 | 107 | TGF-β3-98-1 |
| | 50 | CCCCGAGCCCAAGG | 14 | 108 | TGF-β3-98-2 |
| | 51 | CCCGACGAGCCGG | 13 | 109 | TGF-β3-98-3 |
| | 52 | ACGCACCAAGGCGA | 14 | 110 | TGF-β3-98-4 |
| | 53 | CGGGTTGTCGAGCCC | 15 | 111 | TGF-β3-98-5 |
| | 54 | CGGCAGTGCCCCG | 13 | 112 | TGF-β3-98-6 |
| | 55 | CGCAATTCTGCTCG | 14 | 113 | TGF-β3-98-7 |
| | 56 | TTCGTTGTGCTCCC | 14 | 114 | TGF-β3-98-8 |
| | 57 | ATTCCGACTCGGTG | 14 | 115 | TGF-β3-98-9 |
| | 58 | ACGTGCGTCATCACCGT | 17 | 116 | TGF-β3-98-10 |
| | 59 | CCAAGAAGCC | 10 | 117 | TGF-β3-98-11 |
| | 60 | CCTAATGCCTTCCA | 14 | 118 | TGF-β3-312 |
| | 61 | TCAGCAGGGCCAGG | 14 | 187 | GF-β-3rwk-1 |
| | 62 | GCAAAGTTCAGCAGGGC | 17 | 188 | GF-β-3rwk-2 |
| | 63 | GGCAAAGTTCAGCAGG | 16 | 189 | GF-β-3rwk-3 |
| | 64 | GTGGCAAAGTTCAGCAGG | 18 | 190 | GF-β-3rwk-4 |
| | 65 | GTGGCAAAGTTCAG | 14 | 191 | GF-β-3rwk-5 |
| | 66 | GACCGTGGCAAAGTTCAG | 18 | 192 | GF-β-3rwk-6 |
| | 67 | AGAGAGGCTGACCGT | 15 | 193 | GF-β-3rwk-7 |
| | 68 | GAGAGAGAGAGGCTGAC | 17 | 194 | GF-β-3rwk-8 |
| | 69 | ACAGAGAGAGGCTGA | 15 | 195 | GF-β-3rwk-9 |
| | 70 | GTGGACAGAGAGAGG | 15 | 196 | GF-β-3rwk-10 |
| | 71 | CAACTGGACAGAGAGAGG | 18 | 197 | GF-β-3rwk-11 |
| | 72 | TCTTCTTGATGTGGCC | 16 | 198 | GF-β-3rwk-12 |
| | 73 | CCCTCTTCTTCTTGATG | 17 | 199 | GF-β-3rwk-13 |
| | 74 | CACCCTCTTCTTCT | 14 | 200 | GF-β-3rwk-14 |
| | 75 | ATGGATTTCTTTGGCAT | 17 | 201 | GF-β-3rwk-15 |
| | 76 | GGATTTCTTTGGC | 13 | 202 | GF-β-3rwk-16 |
| | 77 | AAGTTGGACTCTCTTCTC | 18 | 203. | GF-β-3rwk-17 |
| | 78 | TAAGTTGGACTCTCTTCT | 18 | 204. | GF-β-3rwk-18 |
| PGE | 79 | TAGGAGTGGTTGAGGC | 16 | 1539 | Prostaglan.Rec.EP3-1 |
| | 80 | GTGTAGGAGTGGTTGAG | 17 | 1540 | Prostaglan.Rec.EP3-2 |
| | 81 | CTGTGTAGGAGTGG | 14 | 1541 | Prostaglan.Rec.EP3-3 |
| | 82 | CCCACATGCCTGTG | 14 | 1542 | Prostaglan.Rec.EP3-4 |
| | 83 | CGATGAACAACGAG | 14 | 1543 | Prostaglan.Rec.EP3-5 |
| | 84 | CTGGCGATGAACAACG | 16 | 1544 | Prostaglan.Rec.EP3-6 |
| | 85 | CGCTGGCGATGAAC | 14 | 1545 | Prostaglan.Rec.EP3-7 |
| | 86 | GAGCTAGTCCCGTTG | 15 | 1546 | Prostaglan.Rec.EP3-8 |
| | 87 | GCGAAGAGCTAGTCC | 15 | 1547 | Prostaglan.Rec.EP3-9 |
| | 88 | CCAGTTATGCGAAGAGC | 17 | 1548 | Prostaglan.Rec.EP3-10 |
| | 89 | CCCCAGTTATGCGAAG | 16 | 1549 | ProstaglanRec.EP3-11 |
| VEGF | 90 | CGGCCGCGGTGTGT | 14 | 119 | VEGF-98-1 |
| | 91 | CGGGAATGCTTCCGCCG | 17 | 120 | VEGF-98-2 |
| | 92 | CGGCTCACCGCCTCGGC | 17 | 121 | VEGF-98-3 |
| | 93 | CACGTCTGCGGATC | 14 | 122 | VEGF-98-4 |
| | 94 | CCCCGCATCGCATCAGGG | 18 | 123 | VEGF-98-5 |

| Seq. No. | Id.Sequences | Length | | No. int. | Bez. int. |
|---|---|---|---|---|---|
| 95 | CG CCTTG CAACG CG | 14 | | 124 | VEGF-98-6 |
| 96 | CCGACCGGGGCCGG | 14 | | 125 | VEGF-98-7 |
| 97 | GTTCATGGTTTCGG | 14 | | 126 | VEGF-49 |
| 98 | GCAGAAAGTTCATGG | 15 | | 127 | VEGF-55 |
| 99 | GCTGATAGACATCC | 14 | | 128 | VEGF-188 |
| 100 | GCGCTGATAGACAT | 14 | | 129 | VEGF-190 |
| 101 | GTAGCTGCGCTGATAG | 16 | | 130 | VEGF-194 |
| 102 | CTCGATCTCATCAG | 14 | | 131 | VEGF-253 |
| 103 | ATGTACTCGATCTCATC | 17 | | 132 | VEGF-255 |
| 104 | GAAGATGTACTCGATC | 16 | | 133 | VEGF-260 |
| 105 | CTTGAAGATGTACTCG | 16 | | 134 | VEGF-263 |
| 106 | GCATCGCATCAGGG | 14 | | 135 | VEGF-292 |
| 107 | CCGCATCGCATCAG | 14 | | 136 | VEGF-294 |
| 108 | CATTTGTTGTGCTGTAGG | 18 | | 137 | VEGF-422 |
| 109 | GGTCTGCATTCACATTTG | 18 | | 138 | VEGF-434 |
| 110 | CTTTGGTCTGCATTC | 15 | | 139 | VEGF-441 |
| 111 | CTTTCTTTGGTCTGC | 15 | | 140 | VEGF-445 |
| 112 | GCTCTATCTTTCTTTGG | 17 | | 141 | VEGF-450 |
| 113 | GTCTTGCTCTATCTTTC | 17 | | 142 | VEGF-455 |
| 114 | CTTGTCTTGCTCTATC | 16 | | 143 | VEGF-459 |
| 115 | CATCTGCAAGTACGTTCG | 18 | | 144 | VEGF-596 |
| 116 | CACATCTGCAAGTACGTT | 18 | | 145 | VEGF-598 |
| 117 | GTCACATCTGCAAGTACG | 18 | | 146 | VEGF-600 |
| 118 | CATCTGCAAGTACG | 14 | | 147 | VEGF-600-2 |
| 119 | CACATCTGCAAGTAC | 15 | | 148 | VEGF-601 |
| 120 | GTCACATCTGCAAG | 14 | | 149 | VEGF-604 |
| 121 | CTTGTCACATCTGC | 14 | | 150 | VEGF-607 |
| 122 | GGCTTGTCACATCTGC | 16 | | 151 | VEGF-607-2 |
| 123 | CTCGGCTTGTCACATC | 16 | | 152 | VEGF-610 |
| 124 | CTCCTTCCTCCTGC | 14 | | 153 | VEGF-638 |
| 125 | GCTTGAAGATGTACCTCG | 16 | | 154 | VEGF-766 |
| 126 | CGTTGCTCTCCGACG | 15 | | 155 | VEGF-r-1062 |
| 127 | GTAAAACTG GATCATCTC | 16 | | 156 | U16720 |
| 128 | CTTCTTTTGCAAGTCTGT | 18 | | | |
| 129 | TGAGCTGTGCATGCCTTC | 18 | | | |
| 130 | AGTCAGGAGGACCAG | 15 | | | |
| 131 | TGGGTGCCCTGGCCT | 15 | | | |
| 132 | CATGTTAGGCAGGTT | 15 | | | |
| 133 | AGGCATCTCGGAGATCT | 17 | | | |
| 134 | AAAGTCTTCACTCTGC | 16 | | | |
| 135 | AACAAGTTGTCCAGCTG | 17 | | | |
| 136 | CATCACCTCCTCCAG | 15 | | | |
| 137 | GGGTCTTCAGGTTCTCCC | 18 | | | |
| 138 | CACGGCCTTGCTCTTGTT | 18 | | | |
| 139 | TTATTAAAGGCATTCTTC | 18 | | | |
| 140 | AAGATGTCAAACTCACTC | 18 | | | |
| 141 | GTAGTTGATGAAGATGTC | 18 | | | |
| 142 | GATTTTGGAGACCTCT | 16 | | | |
| 143 | TCAGCTATCCCAGAGC | 16 | | | |
| 144 | GGCTGGGTCAGCTAT | 15 | | | |

| Seq. No. | Id.Sequences | Length | No. int. | | Bez. int. |
|---|---|---|---|---|---|
| 145 | AAATCGTTCACAGAGAAG | 18 | | | |
| 146 | TCTTTCTAAATCGTTCAC | 18 | | | |

### Preferred embodiments

1. A pharmaceutical composition comprising at least one TGF-beta antagonist, selected from the group of TGF-beta2 oligonucleotides hybridising with an area of the messenger RNA (m-RNA) and/or DNA encoding TGF-beta2 of SEQ ID No. 28 - 30, and at least one substance inhibiting cell proliferation and/or inducing cell death, selected from the group of temozolomide, BCNU, CCNU" gemcitabine and vincristine.
2. The pharmaceutical composition of embodiment 1 wherein the at least one TGF-beta antagonist and the at least one substance inhibiting cell proliferation and/or inducing cell death are mixed together.
3. The pharmaceutical composition of embodiment 1 wherein the at least one TGF-beta antagonist and the at least one substance inhibiting cell proliferation and/or inducing cell death are separate.
4. The pharmaceutical composition according to embodiment 4 wherein at least one nucleotide of the oligonucleotide is modified at the sugar moiety, the base and/or the internucleotide linkage.
5. The pharmaceutical composition according to embodiment 5 wherein at least one modified internucleotide linkage is a phosphorothioate linkage.
6. Use of a composition comprising at least one TGF-beta antagonist, selected from the group of group of TGF-beta2 oligonucleotides hybridising with an area of the messenger RNA (m-RNA) and/or DNA encoding TGF-beta2 of SEQ ID No. 28 - 30, and at least one substance inhibiting cell proliferation and/or inducing cell death, selected from the group of temozolomide, BCNU, CCNU,, gemcitabine and vincristine.
7. Use of a composition according to embodiment 6 comprising at least one stimulator of the function of the immune system and/or or immune cells and at least one substance inhibiting the cell proliferation and/or inducing cell death for the preparation of a pharmaceutical composition for the treatment of neoplasms.
8. Use according to embodiment 6 or 7 for the treatment of neoplasms selected from the group of solid tumors; blood born tumors such as leukemias, acute or chronic myelotic or lymphoblastic leukemia; tumor metastasis ; benign tumors, for example hemangiomas, acoustic neuromas, neurofibromas, trachomas, and pyogenic granulomas; pre-malignant tumors; astrocytoma, blastoma, chorooma, craniopharyngioma, ependymoma, Ewing's tumor, germinoma, glioma, glioblastoma, hemangioblastoma, hemangioperycatioma, Hodgkins lymphoma, medulloblastoma, leukaemia, mesothelioma, neuroblastoma, non-Hodgkins lymphoma, pinealoma, retinoblastoma, sarcoma (including angiosarcoma, chondrosarcoma, endothelial sarcoma, fibrosarcoma, leiomyosarcoma, liposarcoma, lymphangioandotheliosarcoma, lyphangiosarcoma, medulloblastoma, melanoma, meningioma, myosarcoma, neurinoma, oligodendroglioma, osteogenic sarcoma, osteosarcoma), seminoma, subependymoma, Wilm's tumor, or is selected from the group of bile duct carcinoma, bladder carcinoma, brain tumor, breast carcinoma, bronchogenic carcinoma, carcinoma of the kidney, cervical carcinoma, choriocarcinoma, cystadenocarcinome, embryonal carcinoma, epithelial carcinoma, esophageal carcinoma, cervical carcinoma, colon carcinoma, colorectal carcinoma, endometrial carcinoma, gallbladder carcinoma, gastric carcinoma, head and neck carcinoma, liver carcinoma, lung carcinoma, medullary carcinoma, non-small cell bronchogenic/lung carcinoma, ovarian carcinoma, pancreas carcinoma, papillary carcinoma, papillary adenocarcinoma, prostate carcinoma, small intestine carcinoma, rectal carcinoma, renal cell carcinoma, skin carcinoma, small-cell bronchogenic/lung carcinoma, squamous cell carcinoma, sebaceous gland carcinoma, testicular carcinoma, uterine carcinoma.
9. Use according to embodiment 7 or 8 for the treatment of glioma, glioblastoma and/or anaplastic astrocytom.

## Claims

1. A pharmaceutical composition comprising at least one TGF-beta2 antisense oligonucleotide hybridising with an area of the messenger RNA (m-RNA) and/or DNA encoding TGF-beta2 selected from the group consisting of SEQ ID NO: 28 to 30, and at least one substance inhibiting cell proliferation and/or inducing cell death, selected from the group of BCNU, CCNU, gemcitabine, temozolomide, and vincristine.

2. The pharmaceutical composition of claim 1 wherein the at least one TGF-beta antagonist and the at least one substance inhibiting cell proliferation and/or inducing cell death are mixed together or are separate.

3. The pharmaceutical composition according to any of claims 1 to 2 wherein at least one nucleotide of the oligonucleotide is modified at the sugar moiety, the base and/or the internucleotide linkage.

4. The pharmaceutical composition according to claim 3 wherein at least one modified internucleotide linkage is a phosphorothioate linkage.

5. A composition comprising at least one TGF-beta2 oligonucleotide hybridising with an area of the messenger RNA (m-RNA) and/or DNA encoding TGF-beta2 selected from the group consisting of SEQ ID NO: 28 to 30, and at least one substance inhibiting cell proliferation and/or inducing cell death, selected from the group of BCNU, CCNU, gemcitabine, temozolomide, and vincristine for use in the treatment of neoplasms.

6. The composition for use in the treatment of neoplasms according to claim 5 wherein the neoplasm is selected from the group of solid tumors; blood born tumors such as leukemias, acute or chronic myelotic or lymphoblastic leukemia; tumor metastasis ; benign tumors, for example hemangiomas, acoustic neuromas, neurofibromas, trachomas, and pyogenic granulomas; pre-malignant tumors; astrocytoma, blastoma, chordoma, craniopharyngioma, ependymoma, Ewing's tumor, germinoma, glioma, glioblastoma, hemangioblastoma, hemangioperycatioma, Hodgkins lymphoma, medulloblastoma, leukaemia, mesothelioma, neuroblastoma, non-Hodgkins lymphoma, pinealoma, retinoblastoma, sarcoma (including angiosarcoma, chondrosarcoma, endothelial sarcoma, fibrosarcoma, leiomyosarcoma, liposarcoma, lymphangioandotheliosarcoma, lyphangiosarcoma, medulloblastoma, melanoma, meningioma, myosarcoma, neurinoma, oligodendroglioma, osteogenic sarcoma, osteosarcoma), seminoma, subependymoma, Wilm's tumor, or is selected from the group of bile duct carcinoma, bladder carcinoma, brain tumor, breast carcinoma, bronchogenic carcinoma, carcinoma of the kidney, cervical carcinoma, choriocarcinoma, cystadenocarcinome, embryonal carcinoma, epithelial carcinoma, esophageal carcinoma, cervical carcinoma, colon carcinoma, colorectal carcinoma, endometrial carcinoma, gallbladder carcinoma, gastric carcinoma, head and neck carcinoma, liver carcinoma, lung carcinoma, medullary carcinoma, non-small cell bronchogenic/lung carcinoma, ovarian carcinoma, pancreas carcinoma, papillary carcinoma, papillary adenocarcinoma, prostate carcinoma, small intestine carcinoma, rectal carcinoma, renal cell carcinoma, skin carcinoma, small-cell bronchogenic/lung carcinoma, squamous cell carcinoma, sebaceous gland carcinoma, testicular carcinoma, uterine carcinoma, and/or anaplastic astrocytom.

## Patentansprüche

1. Pharmazeutische Zusammensetzung umfassend mindestens ein TGF-beta2 Antisenseoligonukleotid, das an einen Bereich der Boten-RNA (m-RNA) und/oder DNA hybridisiert, kodierend TGF-beta2 ausgewählt aus der Gruppe bestehend aus SEQ ID NO: 28 bis 30, und mindestens eine Substanz, die Zellproliferation hemmt und/oder Zelltod induziert, ausgewählt aus der Gruppe von BCNU, CCNU, Gemcitabin, Temozolomid, und Vincristin.

2. Pharmazeutische Zusammensetzung gemäß Anspruch 1, wobei der mindestens eine TGF-beta Antagonist und die mindestens eine Substanz, die Zellproliferation hemmt und/oder Zelltod induziert, miteinander vermischt werden oder getrennt sind.

3. Pharmazeutische Zusammensetzung gemäß einem der Ansprüche 1 bis 2, wobei mindestens ein Nukleotid des Oligonukleotids an der Zuckereinheit, der Base und/oder der Internukleotidbindung modifiziert ist.

4. Pharmazeutische Zusammensetzung gemäß Anspruch 3, wobei mindestens eine modifizierte Internukleotidbindung eine Phosphorthioatbindung ist.

5. Zusammensetzung umfassend mindestens ein TGF-beta2 Oligonukleotid, das an einen Bereich der Boten-RNA (m-RNA) und/oder DNA hybridisiert, kodierend TGF-beta2 ausgewählt aus der Gruppe bestehend aus SEQ ID NO: 28 bis 30, und mindestens eine Substanz, die Zellproliferation hemmt und/ oder Zelltod induziert, ausgewählt aus der Gruppe von BCNU, CCNU, Gemcitabin, Temozolomid, und Vincristin zur Verwendung bei der Behandlung von Neoplasmen.

6. Zusammensetzung gemäß Anspruch 5 zur Verwendung bei der Behandlung von Neoplasmen ausgewählt aus der Gruppe von festen Tumoren; blutbasierten Tumoren wie Leukämien, akuter oder chronischer myeloischer oder lymphoblastischer Leukämie; Tumormetastasen; bösartigen Tumoren, zum Beispiel Hämangiomen, Akustikusneurinomen, Neurofibromen, Trachomen, und pyogenen Granulomen; Vorläufer bösartiger Tumoren; Astrozytom, Blastom, Chordom, Kraniopharyngeom, Ependymom, Ewing's Tumor, Germinom, Gliom, Glioblastom, Hämangioblastom, Hämangioperzytom, Hodgkin-Lymphom, Medulloblastom, Leukämie, Mesotheliom, Neuroblastom, Non-Hodgkin-Lymphom, Pinealom, Retinoblastom, Sarkom (einschließlich Angiosarkom, Chondrosarkom, Endothelsarkom, Fibrosarkom, Leiomyosarkom, Liposarkom, Lymphangioandotheliosarkom, Lyphangiosarkom, Medulloblastom, Melanom, Meningeom, Myosarkom, Neurinom, Oligodendrogliom, osteogenes Sarkom, Osteosarkom), Seminom, Subependymom, Wilm Tumor, oder aus der Gruppe von Gallengangskarzinom, Blasenkarzinom, Gehirntumor, Brustkarzinom, Bronchialkarzinom, Nierenkarzinom, Zervixkarzinom, Choriokarzinom, Zystadenokarzinom, embryonalem Karzinom, Epithelkarzinom, Speiseröhrenkarzinom, Kolonkarzinom, kolorektales Karzinom, Endometriumkarzinom, Gallenblasenkarzinom, Magenkarzinom, Kopf- und Halskarzinom, Leberkarzinom, Lungenkarzinom, medulläres Karzinom, nichtkleinzelliges bronchiales/ Lungenkarzinom, Eierstockkarzinom, Pankreaskarzinom, papilläres Karzinom, papilläres Adenokarzinom, Prostatakarzinom, Dünndarmkarzinom, Rektumkarzinom, Nierenzellkarzinom, Hautkarzinom, kleinzelliges bronchiales/ Lungenkarzinom, Plattenepithelkarzinom, Talgdrüsenkarzinom, Hodenkarzinom, Uteruskarzinom, und/oder anaplastisches Astrozytom ausgewählt ist.

## Revendications

1. Une composition pharmaceutique comprenant au moins un oligonucléotide anti-sens TGF-bêta2 hybridant avec une région de l'ARN messager (ARNm) et/ou de l'ADN codant pour le TGF-bêta2 choisie dans le groupe consistant en SEQ-IN NO: 28 à 30, et au moins une substance inhibant la prolifération cellulaire et/ou induisant la mort cellulaire, choisi dans le groupe des BCNU, CCNU, gemcitabine, témozolomide et vincristine.

2. La composition pharmaceutique selon la revendication 1, dans laquelle le au moins un antagoniste de TGF-bêta et le au moins une substance inhibant la prolifération cellulaire et/ou induisant la mort cellulaire sont mélangés entre eux ou sont séparés.

3. La composition pharmaceutique selon l'une quelconque des revendications 1 ou 2, dans laquelle au moins un nucléotide de l'oligonucléotide est modifié au niveau du fragment de sucre, de la base et/ou de la liaison internucléotide.

4. La composition pharmaceutique selon la revendication 3, dans laquelle au moins une liaison internucléote modifiée est une liaison phosphorothioate.

5. Une composition comprenant au moins une oligonucléotide TGF-bêta2 hybridant avec une région de l'ARN messager (ARNm) et/ou de l'ADN codant pour le TGF-bêta2 choisi dans le groupe consistant en SEQ-IN NO: 28 à 30, et au moins une substance inhibant la prolifération cellulaire et/ou induisant la mort cellulaire, choisie dans le groupe des BCNU, CCNU, gemcitabine, témozolomide et vincristine, ladite composition étant destinée à être utilisée dans le traitement de néoplasmes.

6. La composition destinée à être utilisée dans le traitement de néoplasmes selon la revendication 5, dans laquelle le néoplasme est l'une des tumeurs du groupe des tumeurs solides, des tumeurs du sang telles que les leucémies, les leucémies lymphoblastiques ou myélotiques aiguës ou chroniques; les métastases tumorales; les tumeurs bénignes, par exemple les hémangiomes, les névromes acoustiques, les neurofibromes, les trachomes et les granulomes pyogéniques; les tumeurs pré-malignes; les astrocytomes, les blastomes, les chordomes, les craniopharyngiome, les épendymome, la tumeur de Ewing, les germinomes, les gliomes, les glioblastomes, les hémangioblastomes, les hémangioperycatiomes, le lymphome de Hodgkin, les médulloblastomes, les leucémies, les mésothéliomes, les neuroblastomes, les lymphomes non-Hodgkiniens, les pinéalomes, les rétinoblastomes, les sarcomes (y compris les angiosarcomes, les chondrosarcomes, les sarcomes endothéliaux, les fibrosarcomes, les léiomyosarcomes, les liposarcomes, les lymphangioandothéliosarcomes, les lyphangiosarcomes, les médulloblastomes, les mélanomes, les méningiomes, les myosarcomes, les neurinomes, les oligodendrogliomes, les sarcomes ostéogéniques, les ostéosarcomes), les séminomes, les subépendymomes, la tumeur de Wilm,
ou est choisi dans le groupe comprenant le carcinome du canal biliaire principale, le carcinome de la vessie, les tumeurs cérébrales, le carcinome du sein, les carcinomes broncho-pulmonaires, le carcinome du rein, le carcinome cervical, le choriocarcinome, le cystadénocarcinome, les carcinomes embryonnaires, le carcinome épithélial, le carcinome oesophagique, le carcinome du col de l'utérus, le carcinome du côlon, le carcinome colorectal, le carcinome du col utérin, le carcinome de la vésicule biliaire, le carcinome gastrique, les carcinomes du cou et de la tête, le carcinome du foie, le carcinome du poumon, le carcinome médullaire, les carcinomes non à petites cellules bronchogéniques/pulmonaires, le carcinome de l'ovaire, le carcinome du pancréas, le carcinome papillaire, l'adénocarcinome papillaire, le carcinome de la prostate, le carcinome de l'intestin grêle, le carcinome rectal, l'hypernéphrone, les carcinomes de la peau, les carcinomes à petites cellules bronchogéniques/pulmonaires, l'épithélioma spinocellulaire, le carcinome de la glande sébacée, le carcinome des testicules, le carcinome de l'utérus et/ou l'astrocytome de type anaplasique.
